Europäisches Patentamt

European Patent Office

Office européen des brevets

(19)

(11) Publication number: **0 201 883**
**A2**

(12) **EUROPEAN PATENT APPLICATION**

(21) Application number: **86106362.6**

(22) Date of filing: **09.05.86**

(51) Int. Cl.⁴: **A 61 B 19/00**

(30) Priority: **14.05.85 US 733762**
**19.02.86 US 831001**

(43) Date of publication of application:
**20.11.86 Bulletin 86/47**

(84) Designated Contracting States:
**AT BE CH DE FR GB IT LI LU NL SE**

(71) Applicant: **ANDRONIC DEVICES Ltd.**
**P.O. Box 58382**
**Vancouver British Columbia V6P 6E4(CA)**

(72) Inventor: **McEwen, James Allen**
**5259 Turquoise Drive**
**Richmond British Columbia V7C 4Z6(CA)**

(72) Inventor: **Auchinleck, Geoffrey Fletcher**
**5-1182 West 7th Avenue**
**Vancouver British Columbia V6H 1B4(CA)**

(72) Inventor: **Osborne, John Calvin**
**209-2920 Ash Street**
**Vancouver British Columbia V5Z 4A6(CA)**

(72) Inventor: **Bohl, Rainer Mattias**
**41 East 40th Avenue**
**Vancouver British Columbia V5W 1L3(CA)**

(72) Inventor: **Jager, Walter Johann**
**45 Helene Crescent**
**Waterloo Ontario N2L 5E6(CA)**

(74) Representative: **Perani, Aurelio et al,**
**c/o JACOBACCI-CASETTA & PERANI S.N.C. Via Visconti di Modrone 7**
**I-20122 Milano(IT)**

(54) **Advanced medical robot.**

(57) An automatic device, such as a robot for moving a medical effector in close proximity to a portion of a living body, such as the limb of a patient. In one form, electronic sensing means senses a physiologic or morphologic condition of a patient to whose limb or body the medical effector is in close proximity and generates signals which are applied to the inputs of a computer or microelectronic processor, which electrically processes and analyses such signals and generates control signals for controlling the operation of the robot to move the medical effector so as to achieve a desired diagnostic or therapeutic effect.

EP 0 201 883 A2

FIG 3

861063626

## FIELD OF THE INVENTION

This invention pertains to medical robots for manipulating an effector relative to a patient in response to changes in a physiological or morphological condition of the patient. In particular, the invention pertains to an advanced medical robot configured as a limb manipulator for assisting in the performance of arthroscopic surgical procedures by manipulating a patient's limb into appropriate configurations.

## BACKGROUND OF THE INVENTION

A "robot" has been defined by the Robot Institute of America as a reprogrammable multifunctional manipulator designed to move materials, tools or specialized devices, through variable programmed motions for the performance of a variety of tasks. An "advanced robot" may be defined as a general-purpose machine system which, like a human, can perform a variety of different tasks under conditions that may not be known a priori. The present invention provides a medical robot in which advanced robotics is applied in a device to assist in surgery, in an effort to control and reduce operating costs while improving the quality of medical treatment and diagnosis.

The costs of continuing to maintain the same quality of diagnosis and treatment in acute-care hospitals, as well as the costs associated with continuing demands for improved health care, appear to be increasing faster than the ability of society of meet them. Approximately 80 percent of the operating budget of many acute-care hospitals is now allocated for labour. At present, much of the labour in hospitals is directed towards repetitive, well-defined tasks which are suited to the application of robotics and related automation. The application of advanced robotics to health care may thus assist, as it has already done in manufacturing and industrial applications, to improve productivity and thus control or reduce costs.

Many medical and surgical procedures are presently designed around inherent limitations of human performance or are constrained by limitations of human performance: Examples may be seen in problems concerning the accurate, safe and consistent manipulation of very small or large structures, or the simultaneous manipulation of multiple structures. Thus, the development and application of advanced robotics to medical and surgical procedures may help to expand or improve the range of treatment and diagnostic procedures available by removing some constraints associated with human performance. Furthermore, in acute-care hospitals, staff and patients may be exposed to a variety of hazardous environments and agents such as radiation, sterilization, harmful gases, and antineoplastic agents, and they may be exposed to a variety of special environments such as sterile, infectious and hyperbaric environments. The application of advanced robotics may help reduce the exposure of personnel to such hazardous or special environments and agents.

It is known in the art to use for the performance of a variety of tasks health-care devices which are capable of reprogrammable manipulation or multifunctional manipulation of parts, tools or specialized objects. It is also known in the art to use for heath-care tasks devices which are capable of manipulating objects through variable or programmed motions. Examples of such devices include: some prosthetic and orthotic aids for functional replacement or augmentation of amputated or disabled limbs; computerized drug-delivery systems which control the amount of drug delivered to patients by manipulating syringes; programmable exercise machines; continuous passive motion devices for moving limbs post-surgically; computerized environmental aids or aids employing industrial robotic elements for use by people who are functionally disabled; pre-programmed stress-testing machines for diagnostic purposes; remotely-operated manipulators for handling hazardous materials or very small objects; operator-controlled, power-assisted wheelchairs; automated patient-transfer devices; and power-assisted operating-room tables and patient-positioning devices. However, none of

4

the health-care devices known in the prior art can be considered to be "medical robots" because they either do not perform a medical function, or they lack at least one of the essential characteristics of a robot, as defined by the Robot Institute of America. A device which performs a medical function may be defined as one which can be employed in the diagnosis or treatment of disease.

Further, none of the devices known in the prior art for the performance of health-care tasks can be considered to be "advanced" medical robots, in accordance with the definition of advanced robots given above: for example, none are general-purpose machine systems which employ transducers to sense a physiologic or morphologic parameter of a patient and then change their diagnostic or therapeutic operation in response to changes in that parameter. Examples of physiologic or morphologic parameters which could be employed in advanced medical robots include: anatomical dimensions and proportions; weights and relative weights; positions and relative locations of various structures or portions of the body; pressures, forces, torques, stresses and strains on various tissues and structures; color and other visual features of tissues, organs and structures; temperature; pH; respiratory waveforms; electrocardiographic waveforms; electroencephalographic waveforms; blood-pressure waveforms; electroretinographic signals; blood-flow signals; and electromyographic signals.

5

One particular opportunity for employing a medical robot is for assisting in the performance of a medical procedure known as an arthroscopy. Arthroscopy allows visual inspection of the interior of a knee or other joint, both for diagnostic purposes and during the performance of corrective surgery, typically via one or two small punctures in the knee or other joint. Using fiberoptic technology to provide light to the interior of the joint, an arthroscope has a sophisticated optical lens system that allows an orthopedic surgeon to view the area, either by direct vision or by using a closed-circuit television camera and monitor. While viewing the injured area through the arthroscope, the surgeon may insert a thin probe, knife or forceps through another puncture, to examine, cut away and remove damaged cartilage, bone, or joint tissue. Other instruments such as an electrically operated rotary cutter may also be inserted through a second puncture, for additional types of surgery. An additional puncture is often used for infusion of a solution to distend the area for better viewing, or to carry away the particles removed during surgery.

Arthroscopy has become well-established for diagnosis and treatment of knee injuries; it is also being used with injuries of the ankle, elbow, hip, and shoulder. The basic techniques are the same for all joints. Diagnostic arthroscopy is quickly replacing its predecessor, arthrography (x-rays using injected contrast

6

material) in many applications. Operative arthroscopy is making possible the repair of many types of joint injuries on an outpatient basis. With more serious injuries that require inpatient care, the patient can become ambulatory much sooner than was possible with the previous surgical treatment. Arthroscopic technology is expanding to the rest of the orthopedic community very rapidly because it allows more cost-effective diagnosis and treatment.

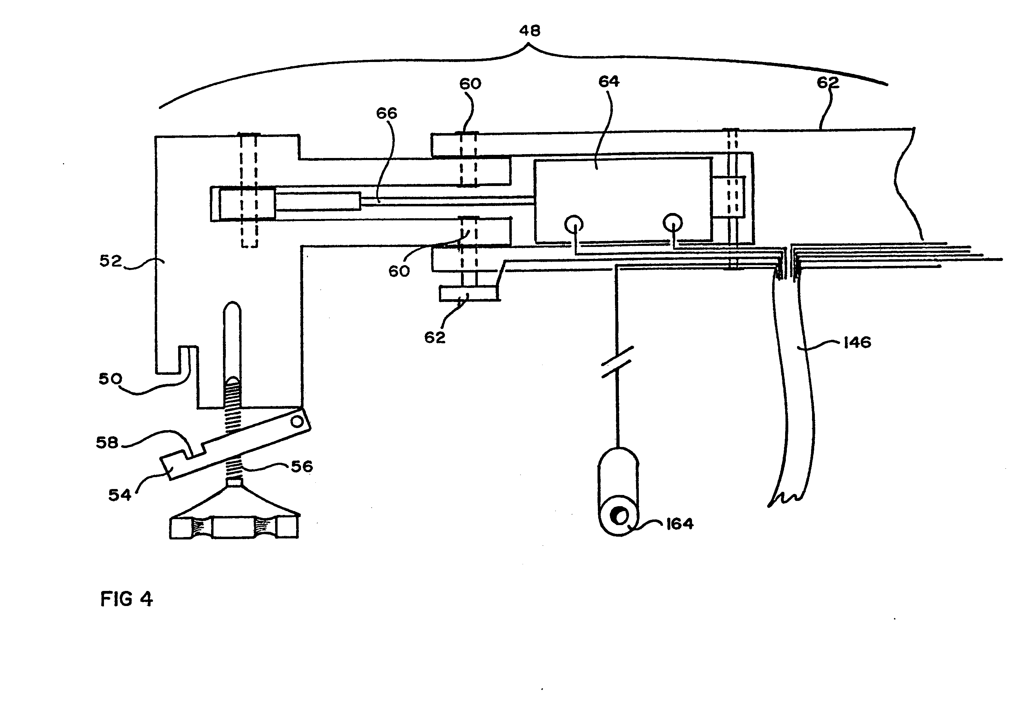

A major problem associated with the performance of arthroscopies at present is the problem of efficient, accurate and safe manipulation of the patient's limb during the surgical procedure. At present, some surgeons attempt to manipulate and hold the patient's limb themselves, while simultaneously attempting to manipulate the arthroscope and various instruments. Other surgeons presently employ a surgical assistant for the purpose of manipulating the patient's limb, holding it in desired positions, and occasionally for assisting in the manipulation of instruments. The present methods of manipulating the patient's limb are labour-intensive and thus costly, and as well are tedious for the surgical assistant. In addition, the quality of the procedure suffers because the surgeon is able to control only indirectly the positioning of the patient's limb and thus his visualization of the various structures within the joint is impaired. Typically for example, a surgeon performing a diagnostic or surgical arthroscopy on a patient's leg may wish to move the leg

7

into a number of positions, including the following: an elevated position, to permit blood to flow out of the limb before a tourniquet is applied; a "full extension" position, in which the patient's leg is moved to a horizontal position; a "full flex" position, in which the lower portion of the patient's leg is at approximately 90 degrees to the upper portion; a "half flex" position, in which the lower portion of the patient's leg is at an angle of approximately 135 degrees to the upper portion of the leg; and a "figure 4" position, in which the patient's leg is rotated and the ankle is brought up to the knee of the adjacent leg, so that the two legs together form a figure 4 in a horizontal plane. In addition, the surgeon may wish to apply a lateral valgus or varus strain to the leg, in order to improve visualization of certain parts of the knee. Also, the surgeon may wish to apply an internal or external rotational torque to the leg for improved visualization. In addition, surgeons may wish to vary the position of the patient's leg from the standard positions. Present devices and manual techniques for surgical arthroscopies and diagnostic arthroscopies have tended to limit the nature of these procedures; the procedures are inherently limited because of the fact that the surgeon has only two arms and hands, and thus must employ instruments and techniques which are suited to such limitations. The availability of a robotic patient limb manipulator to perform the manipulations of the patient's limb directly

8

under the surgeon's control will help to eliminate many of the above-described problems. In addition, such a manipulator should help to reduce the cost of diagnosis and treatment by reducing the labour-intensiveness of the procedure; in many cases the need for a surgical assistant may be eliminated. Also, a suitable robotic limb manipulator would facilitate a method for improving the quality and consistency of such diagnostic and therapeutic procedures. Finally, the invention may help to facilitate the development of new arthroscopic procedures for the knee, and for other joints and areas of the body.

Direct control of a limb manipulator raises a unique problem that now exists in operating rooms: the surgeon must be able to control the manipulator without compromising the sterility of the operating environment. This problem involves more than just the patient manipulator; in many cases, a surgeon would like to be able to directly control a medical device without physically contacting it and thus compromising sterility, and without diverting his eyes or moving his hands from the surgical task at hand. Due to these constraints, control of a manipulator by joystick, hand operated switches, footswitches, lead-through, or power-assisted lead-through, although meeting the requirement of maintaining the surgeon's sterility, are not ideal. As a result, voice control of such a manipulator offers clear advantages over other methods of control, in that it offers both "hands

9

free" and "eyes free" control.

Traditional methods of voice control are not well suited to use in a surgical setting because of limitations on recognition accuracy, particularly in the presence of random background noise, and slow response time to spoken commands. It is recognized that no voice recognition system will accurately recognize a spoken command at all times and under all conditions, thus an operator controller for a device such as a patient limb manipulator must compensate for this by allowing the operator to execute commands at any time, despite failure of the voice recognizer. In addition, it is recognized that no voice recognizer will recognize a spoken command instantly. A voice control system suitable for use in a surgical setting must overcome the above described problems by offering some method for ensuring effective control even when voice recognition fails, and delay-free control when required.

Traditional voice control systems are also unable to solve other problems that arise in a surgical setting. Background noise and multiple simultaneous speakers in an operating room require that a voice collection apparatus which offers high selectivity be used. In addition, any such collection apparatus must not compromise the sterility of the surgical field. Another problem with attempting to employ a conventional microphone in such an application is the presence of the connecting wire between a microphone attached to the user's head or body, and the voice-control circuitry. The presence of a wire would tend to constrain the movement of the surgeon about the operating room, and perhaps could compromise sterility of the surgical site. In addition, surgical masks worn by surgical staff tend to muffle and distort speech sounds, and thus a speech-collection apparatus must compensate for this, without compromising the efficiency of bacterial filtration of the surgical masks. Any alteration to existing surgical masks must take into account differences between facial shapes, mask application techniques, and mask positioning among surgeons.

## SUMMARY OF THE INVENTION

In a broad aspect, the present invention provides apparatus for manipulating an effector relative to a patient in response to changes in a physiological or morphological condition of the patient. The apparatus comprises medical condition sensing means for sensing the patient's physiological or morphological condition of interest and for producing a medical condition output signal representative thereof; position sensing means for sensing the position of the effector relative to the patient and for producing a position output signal representative thereof; signal processing means for receiving the medical condition and position output signals, for comparing the medical condition signal with a signal representative of a desired physiological or morphological condition of the patient, for determining an

11

alternate position of the effector capable of producing the desired physiological or morphological condition in the patient, and for producing a position control signal representative of the alternate position; and, positioning means responsive to the position control signal for positioning the effector in the alternate position. As used herein the term "effector" means apparatus for producing the desired physiological or morphological effect.

In another aspect, the invention provides apparatus for sensing and altering a physiological or morphological condition of a patient. In such case the apparatus includes medical condition sensing means as above, effector means responsive to an effector control signal for producing a desired physiological or morphological condition in the patient; and position sensing means, signal processing means and positioning means as above. The signal processing means may further be adapted to determine the desired physiological or morphological condition of the patient and produce a signal representative thereof. Further, the signal processing means may be adapted to determine an operating condition of the effector means capable of producing the desired physiological or morphological condition in the patient and producing an effector control signal to cause the effector means to assume that operating condition.

Preferably, the signal processing means compares the effector position control signal with a range of signals representative of safe alternate operating positions of the effector means and inhibits transmission of the position control signal to the positioning means if the position control signal is outside the range of safe signals. Similarly, the signal processing means preferably compares the effector control signal with a range of signals representative of safe operating conditions of the effector means and inhibits transmission of the effector control signal to the effector means if the effector control signal is outside the range of safe signals.

Advantageously, the signal processing means is further adapted to determine a range of possible alternate positions of the effector means and a range of possible alternate operating conditions of the effector means, evaluate the physiological or morphological effect on the patient of those possible alternate positions and operating conditions and of combinations thereof, select from the determined ranges an alternate position and operating condition of the effector means most likely to produce the desired physiological or morphological condition in the patient, and produce the position and effector control signals corresponding thereto.

In a particularly preferred embodiment the invention provides a patient limb manipulator comprising selectably positional grasping means for grasping a patient's limb and for moving the limb into a selected position in response to a control signal. The apparatus includes limb position sensing means for sensing the position of the limb while it is grasped by the grasping means and for producing a limb position signal representative of the sensed limb position. The apparatus further includes signal processing means for comparing the limb position signal with a signal representative of the selected limb position, for determining a path for moving the limb from the sensed position to the selected position, and for producing the control signal to cause the grasping means to move the limb from the sensed position along the path to the selected position.

Preferably, the patient limb manipulator compares the control signal with a range of signals representative of safe paths and positions of the limb (i.e. anatomically natural positions) and inhibits transmission of the control signal to the grasping means if the control signal is outside the range of safe signals.

In yet another aspect the invention provides a method of manipulating a portion of a living body for diagnostic or therapeutic purposes. The method comprises the steps of positioning an effector relative to the body portion, monitoring the position of the effector relative to the body portion, monitoring a physiological or morphological condition of the patient, determining an alternate position of the body portion capable of producing a desired change in the patient's physiological or

14

morphological condition, and actuating the effector to position the body portion in the alternate position.

## BRIEF DESCRIPTION OF THE DRAWINGS

FIG. 1 is a block diagram which illustrates the principal operating features of a medical robot according to the invention.

FIG. 2 is a pictorial illustration of a medical robot configured as a patient limb manipulator for assisting in the performance of surgical arthroscopic operations.

FIG. 3 is a side elevation view showing the kinematic structure of the patient limb manipulator of figure 2.

FIG. 4 is an enlarged side elevation view showing the kinematic structure of the shoulder area of the patient limb manipulator of figures 2 and 3.

FIG. 5 is an enlarged top view showing the kinematic structure of the wrist mechanism of the patient limb manipulator of figures 2, 3 and 4.

FIG. 6 is a partially exploded pictorial illustration showing the patient applied part and wrist mechanism of the patient limb manipulator of figures 2, 3, 4 and 5.

FIG. 7 is a block diagram of a typical hydraulic actuator mechanism for use with the patient limb manipulator of figures 2 - 6.

FIG. 8 is a block diagram of a typical pneumatic actuator mechanism for use with the patient limb manipulator of figures 2 - 6.

FIG. 9A is a side elevation view of an operator hand control for use with the patient limb manipulator of figures 2 - 6.

FIG. 9B is a top view of the operator hand control of figure 9A.

FIG. 9C is pictorial illustration of the operator hand control of figures 9A and 9B.

FIG. 10 is a block diagram of a preferred automatic speech input/output sub-system for use with the patient limb manipulator of figures 2 - 6.

FIG. 11 is a pictorial illustration of a speech collection apparatus for use with the speech sub-system of figure 10.

FIG. 12 is a pictorial illustration of the kinematic structure of a braking mechanism for an alternate embodiment of the patient limb manipulator shown in Figure 13.

FIG. 13 is a pictorial illustration of a simplified, low cost patient limb manipulator according to an alternate embodiment of the invention.

FIG. 14 is a schematic diagram which shows the configuration of actuators comprising a simplified operator control interface for use with the alternate embodiment of Figure 13.

16

## DETAILED DESCRIPTION OF THE PREFERRED
## EMBODIMENT

### I. Introduction

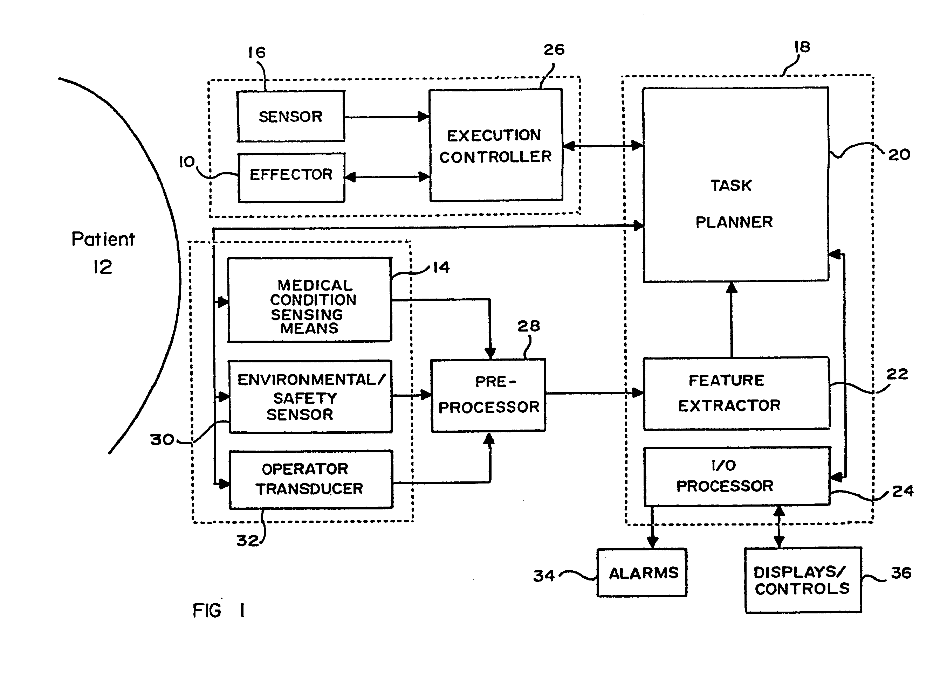

Figure 1 is a block diagram which illustrates the principal operating features of an advanced medical robot according to the invention. The robot constitutes apparatus for manipulating and/or varying the operating condition of an effector 10 (for example, effector 10 may be a tissue retractor, a laser, an electrode, or other diagnostic or theraputic device) relative to a patient 12 in response to changes in a physiological or morphological condition of the patient. The apparatus comprises medical condition sensing means 14 for sensing a desired physiological or morphological condition of the patient and for producing an electronically readable medical condition output signal representative thereof. Medical condition sensing means 14 may for example be an electronic thermometer for sensing the patient's temperature, a blood pressure sensor for sensing the patient's blood pressure, an x-ray machine, a nuclear magnetic imaging system, a positron-emission scanner, or a flouroscopy machine for sensing the position of certain of the patient's body structures, electrodes for sensing the patient's electrocardiographic waveform, electroencephalographic waveform, electromyographic signals, or electroretinographic response, sensors for determining the patient's limb positions, or a pressure sensor for sensing tissue pressure or the patient's respiritory waveform.
The apparatus further comprises position sensing means 16
for sensing the position of effector 10 relative to patient
12 and for producing an electronically readable position
output signal representative thereof. The apparatus
further comprises signal processing means which, in the
preferred embodiment, is a computer 18. Computer 18
receives the medical condition and position output signals
produced by medical condition sensing means 14 and position
sensing means 16; compares the medical condition signal
with a signal representative of a desired physiological or
morphological condition of the patient; determines an
alternate position and/or operating condition of effector
10 capable of producing the desired physiological or
morphological condition in the patient; and produces a
position control signal and/or effector control signal
respresentative, respectively, of that alternate position
and operating condition, thereby causing effector 10 to
assume the alternate position and/or operating condition.
Before transmitting the position and effector control
signals to cause effector 10 to assume the alternate
position and/or operating condition, computer 18 first
compares those signals with ranges of pre-programmed
signals representative of safe alternate positions and
operating conditions of effector 10. If the position or
effector control signals fall outside the safe ranges
aforesaid then they are not transmitted, thereby preventing

18

possible patient injury. Instead, computer 18searches for an acceptable alternate position and/or operating condition of effector 10 which falls within the safe ranges aforesaid and which is capable of producing the desired physiological or morphological condition in the patient or an acceptable approximation thereof.

To accomplish these objectives comptuer 18 is programmed with software (hereinafter described in greater detail) including a task planner 20, a feature extractor 22, an input/output processor 24 and an execution controller 26. More specifically, the medical condition output signals produced by medical condition sensing means 14 are processed by pre-processor 28 to remove noise before the signals are transmitted to feature extractor 22. Signals are also transmitted to pre-processor 28 from environmental/safety sensor 30, which produces electronically readable signals indicative of potential hazards such as obstructions or interferences to the patient manipulator, and from operator transducer 32 which, when activated by the surgeon as hereinafter described, produces electronically readable signals indicative of the surgeon's desired position and/or operating condition for effector 10. Operator transducer 32 may include speech recognition apparatus, a joystick, foot controls or other input devices suitable for use by a surgeon in a sterile field. Feature extractor 22 removes artifact and reduces signal bandwidth by identifying and transmitting only the signal characteristics of interest. Signals passed by feature extractor 22 are interpreted by task planner 20 which generates appropriate control signals for execution by execution controller 26. Input/output processor 24 allows task planner 20 to communciate with alarms 34 and displays/controls 36. Alarms 34 (which may be audible, visual, or a combination of both) are for alerting the operator or surgeon to potentially hazardous operating conditions. Displays/controls 36 may include visual status indicators, audible indicators such as a voice synthesis unit, a video display or other similar devices, for displaying information representative of the position and/or operating condition of effector 10, or for prompting the operator in particular situations. Controls may include voice recognition equipment, footswitches, specialized keypads, joysticks or other input devices, for controlling the operation of effector 10. Execution controller 26 serves, in part, as a "positioning means" which is responsive to the position control signal generated by computer 18 to position effector 10 in whatever alternate position may be determined by computer 18. Execution controller 26 also transmits to effector 10 any control signals produced by computer 18 for causing effector 10 to assume a desired operating condition.

As hereinafter described, the preferred embodiment is a patient limb manipulator for manipulating a patient's limb during a surgical procedure such as an arthroscopy, such manipulation being directed by the operating surgeon. It is to be understood however that the invention is of much broader application and may be adapted to an extremely wide range of health care procedures in which an effector is to be positioned and/or operated relative to a living body or portion thereof, in response to varying physiological or morphological conditions of the body so as to produce a desired physiological or morphological condition in the body. Accordingly, the invention is to be limited only by reference to the appended claims.

The construction and kinematic operation of the preferred patient limb manipulator will first be described, following which a detailed description of the operation of the device by an operating surgeon or operating room assistant will be given. Finally, a description of the software which controls the operation of the preferred embodiment will be presented.

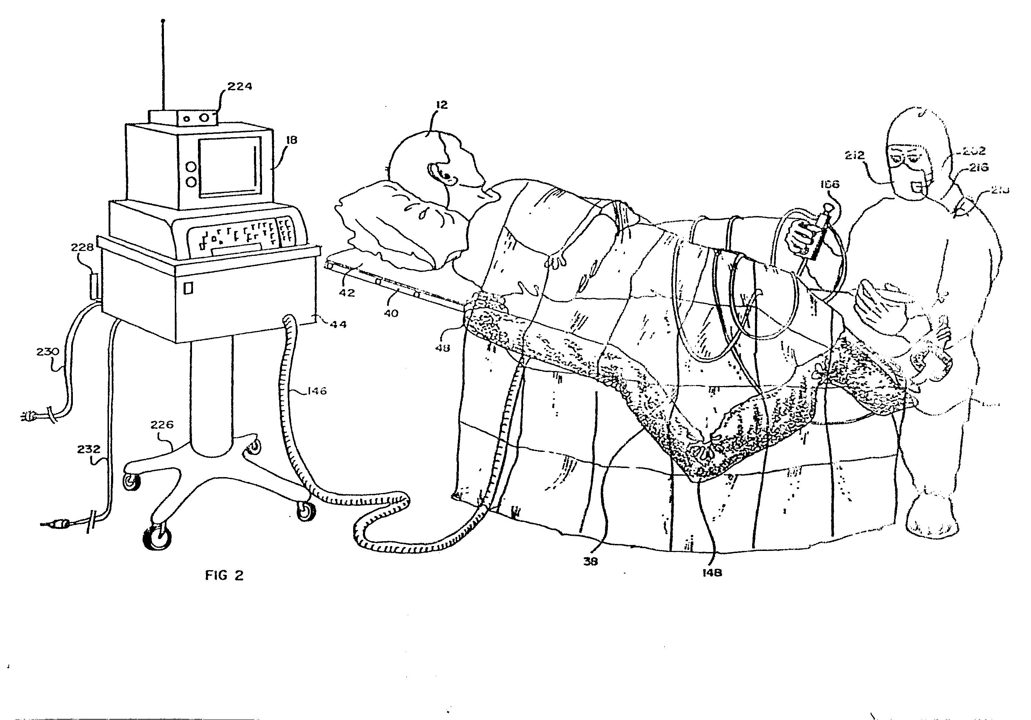

## II. Construction and Kinematic Operation Figures 2 through 6 depict a patient limb manipulator 38 which consists of a kinematic structure having seven degrees of freedom, four of which are hydraulically powered, and three of which are pneumatically powered. Manipulator 38 may be attached to side rail 40 of a standard operating room table 42. The hydraulic cylinders (hereinafter described) which power manipulator 38 are powered by hydraulic pump units located in control box 44. The pneumatic actuators (herinafter described) which power manipulator 38 are controlled by pneumatic servo units 46 included in control box 44 and may be powered by any compressed gas source supplying 40 - 60 PSI, such as a gas bottle or the piped in gas supply usually found in operating rooms. An IBM$^{TM}$ personal computer 18 ("signal processing means") is connected to control box 44 to provide control signals for electromechanical components of control box 44 as hereinafter explained.

One degree of freedom of the kinematic structure of manipulator 38 is provided by shoulder structure 48 (best seen in Figure 4). The apparatus used to attach manipulator 38 to operating table 42 is also located on shoulder structure 48. The attachment mechanism consists of a notch 50 cut into mounting block 52, a clamping plate 54, and a clamping bolt 56. Notch 50 is made to fit over a standard 3/8" by 1 1/8" operating table side rail 40 (Figure 2). Clamping plate 54 is pivotally connected to mounting block 52 and held in place with clamping bolt 56. Notch 58 in clamping plate 54 is made to fit under operating table side rail 40. Turning clamping bolt 56 forces clamping plate 54 against operating table side rail 40 to secure manipulator 38 in place. Mounting block 52 also supports a pair of shoulder rotation pivot axles 60, which are located approximatly 4 inches from the proximal edge of block 52. A first arm section 62 pivots about shoulder rotation pivot axles 60, thereby providing a first degree of freedom of manipulator 38. Pivot axles 60 and the other axles, hereinafter described, about which segments of manipulator 38 may move relative to one another, are hereinafter sometimes called "joints".

First hydraulic actuator 64 is connected between mounting block 52 and shoulder structure 48 in such a way that push rod 66 of hydraulic actuator 64 can pass between shoulder pivot axles 60, allowing first arm section 62 to pivot 180 degrees with respect to mounting block 52. Shoulder pivot axles 60 are rigidly connected to mounting block 52. Shoulder rotation position sensor 68 (which may be a 10K-ohm potentiometer) is connected between one pivot axle 60 and first arm section 62 so that the angle of first arm section 62 can be measured with respect to mounting block 52.

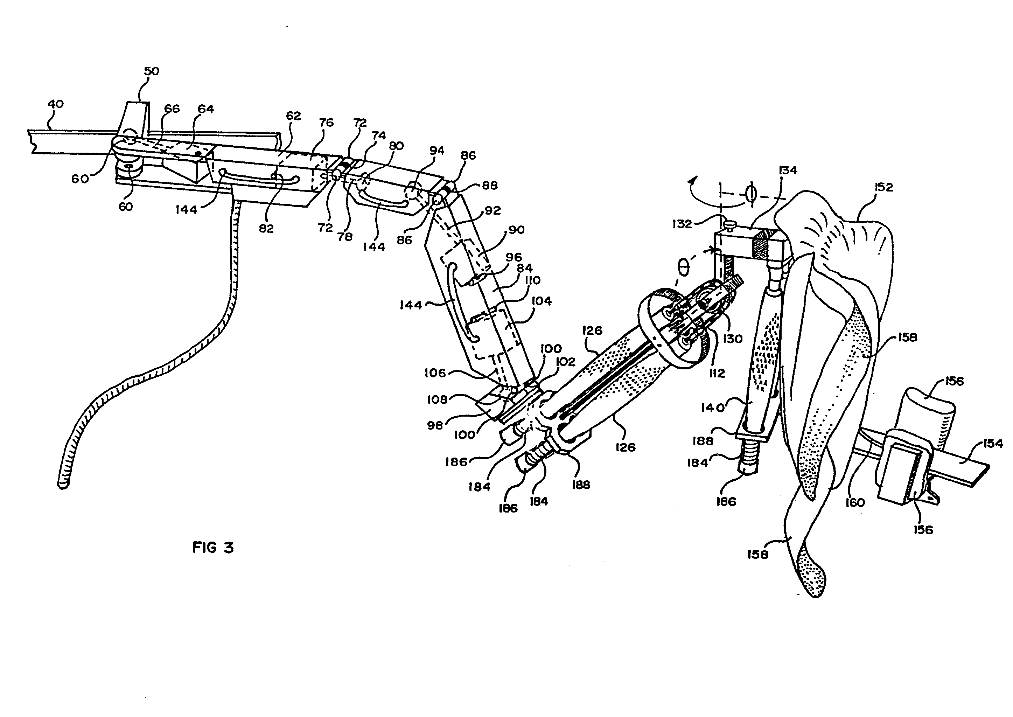

A second arm section 70 (Figure 3) is pivotally connected to the distal end of first arm section 62 by means of a pair of second pivot axles 72, located approximatly 14 inches distal to first pivot axles 60, thereby providing a second degree of freedom of manipulator 38. Second pivot axles 72 are rigidly connected to first arm section 62 and rotate therewith. Second position sensor 74 (also a 10K-ohm potentiometer) is connected between one of pivot axles 72 and second arm section 70 so that the angle of second arm section 70 can be measured with respect to first arm section 62. Second hydraulic actuator 76 is connected between first arm section 62 and second arm section 70, such that its push rod 78 acts on a pivot 80 attached to second arm section 70 at a point 1 inch below and 1 inch distal to pivot axles 72. The rear pivot 82 of hydraulic actuator 76 is located in first arm section 62, and is positioned such that second arm section 70 can rotate through 90 degrees of arc with respect to first arm section 62.

A third arm section 84 is pivotally connected to the distal end of second arm section 70 by means of a pair of third pivot axles 86, located 6 inches distal to second pivot axles 72, thereby providing a third degree of freedom of manipulator 38. Third pivot axles 86 are rigidly connected to third arm section 84 and rotate therewith. Third position sensor 88 (another 10K-ohm potentiometer) is connected between one of pivot axles 86 and second arm section 70 so that the angle of third arm section 84 can be measured with respect to second arm section 70. Third hydraulic actuator 90 is connected between third arm section 84 and second arm section 70 such that the push rod 92 of actuator 90 acts on a pivot 94 located in second arm section 70 one inch below and one inch proximal to third pivot axles 86. The rear pivot 96 of third hydraulic actuator 90 is located in third arm section 84 in such a way that third arm section 84 can rotate through 90 degrees of arc with respect to second arm section 70.

A fourth arm section 98 is pivotally connected to the distal end of third arm section 84 by means of a pair of fourth pivot axles 100, located approximatly 12 inches distal to third pivot axles 86, thereby providing a fourth degree of freedom of manipulator 38. Fourth pivot axles 100 are rigidly connected to third arm section 84 and rotate therewith. Fourth position sensor 102 (also a 10K-ohm potentiometer) is connected between one of pivot axles 100 and third arm section 98 so that the angle of fourth arm section 98 can be measured with respect to third arm section 84. Fourth hydraulic actuator 104 is connected between third arm section 84 and fourth arm section 98, such that the push rod 106 of fourth hydraulic actuator 104 acts on a pivot 108 located in fourth arm section 98 one inch below and one inch distal to fourth pivot axles 100. The rear pivot 110 of fourth hydraulic actuator 104 is located in third arm section 84 such that fourth arm section 98 can rotate through 90 degrees of arc with respect to third arm section 84. Fourth arm section 98 is mounted so that it is normally at 90 degrees to third arm section 84 and can rotate to an angle of 180 degrees, or parallel with third arm section 84.

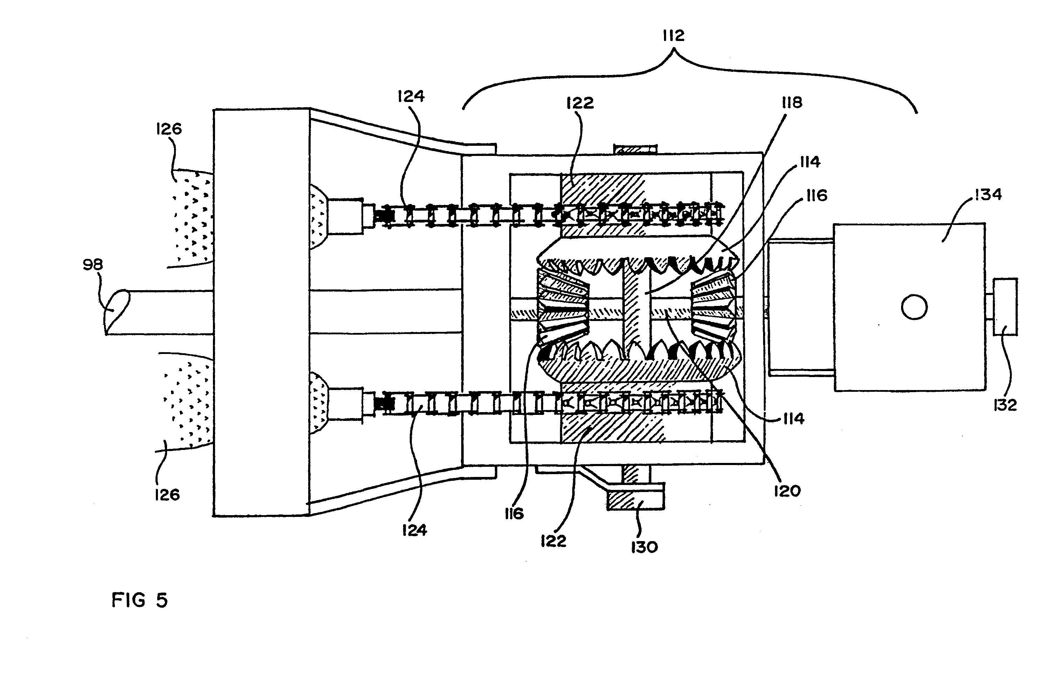

At the distal end of fourth arm section 98 is a differential mechanism 112 (best seen in Figure 5), located approximatly 14 inches distal to fourth pivot axles 100, which provides an additional two degrees of freedom for manipulator 38. Differential mechanism 112 provides a pitching motion about an axis parallel to pivot axles 72, 86 and 100, and a yawing motion about an axis perpendicular to pivot axels 72, 86 and 100. Two pairs of beveled gears 114, 116 are mounted on perpendicular axes formed by a crosspiece comprising axles 118 and 120 to form a differential gear mechanism. Large bevel gears 114 are each rigidly connected to sprockets 122. Large bevel gears 114 and sprockets 122 are mounted on opposed ends of axle 118 to form one half of the differential mechanism. Axle 118 is in turn pivotally attached to fourth arm section 98 such that axle 118 is parallel to pivot axles 72, 86 and 100, to provide a pitch movement. Small beveled gears 116 are rotatably mounted on the opposed ends of axle 120 between large bevel gears 114 to form the other half of the differential mechanism. Around each of sprockets 122 is fitted a short length of chain 124, each end of which is connected to a pneumatic actuator 126. The opposite ends of the four pneumatic actuators are connected to the proximal end of fourth arm section 98 with a tensioning device 128 (see Figure 3). The four pneumatic actuators 126 (only two of which are visible in the drawings) and two chains 124 may be used to alter the angle of the two large bevel gears 114 with respect to fourth arm section 98 and each other, and therefore the position of the small bevel gears 116. Connected between fourth arm section 98 and the axle 118 is pitch position sensor 130 (which may be a 10K-ohm potentiometer), which measures the angle $\theta$ (see Figure 3) of differential mechanism 112 with respect to fourth arm section 98. Connected between one of small bevel gears 116 and axle 120 is yaw position sensor 132

26

(also a 10K-ohm potentiometer), which measures the angle
∅ of small bevel gear 116 with respect to fourth arm
section 98.

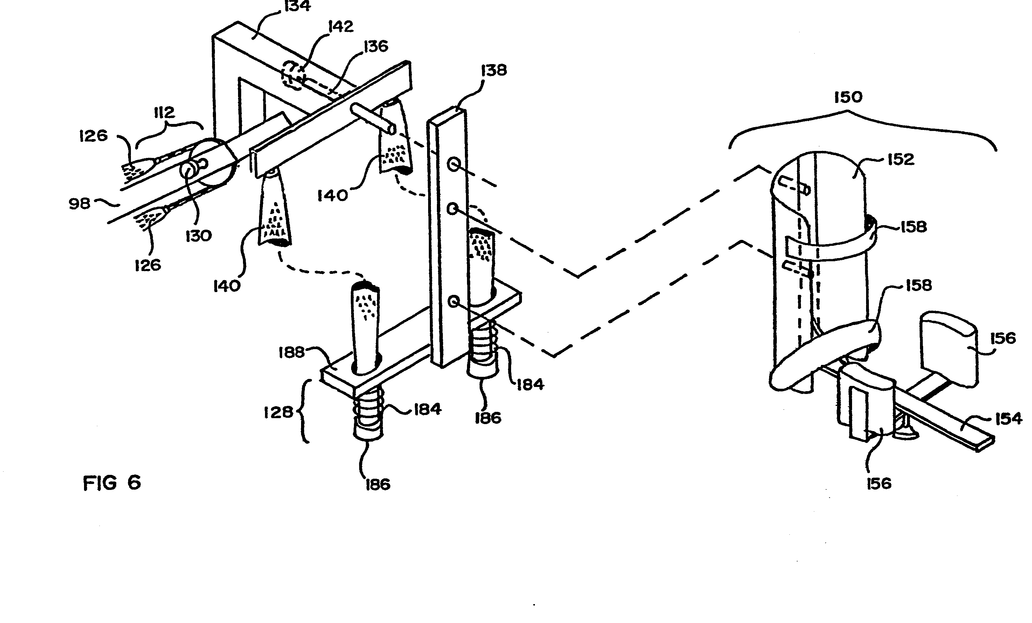

Attached to the small bevel gear 116 closest to
the distal end of manipulator 38 is roll axis block 134.
Within roll axis block 134 is pivotally mounted roll axis
136 (see Figure 6), which provides a seventh degree of
freedom to manipulator 38. Roll axis 136 is rigidly
connected to patient applied part mounting bar 138.
Pneumatic actuators 140 are attached to each side of roll
axis block 134. The other ends of each of pneumatic
actuators 140 are attached to the distal end of patient
applied part mounting bar 138 with tensioning devices 128
(Figure 3). Connected between roll axis block 134 and roll
axis 136 is roll position sensor 142 (also a 10K-ohm
potentiometer) which measures the angle between roll axis
136 and roll axis block 134.

Attached to the sides and bottom of manipulator 38
are environmental saftey sensors 144 (Figure 3). These
sensors consist of lengths of latex rubber tubing which are
sealed at one end, and connected to a pressure switch at
the other. The pressure switch is designed to close an
electrical contact if an input pressure greater than a set
limit is detected. The pressure limit is chosen to be low
enough that any deformation of the latex tubing, such as
would ocurr if the tubing were bumped, or pressed upon,
will cause the electrical contact to close. All of the pressure switches are connected in parallel, such that closure of any switch will be detected. All switches are connected to computer 18 via umbilical cord 146.

During normal operation, the entire structure of manipulator 38 is covered in a vinyl plastic sleeve 148 (Figure 2) which serves to prevent fluids, surgical drapes or other materials from entering moving parts of manipulator 38.

Patient applied part 150 (Figures 3 and 6) consists of a curved plastic piece 152 shaped to fit against the back of a patient's leg. Attached to plastic piece 152 is plastic sole piece 154 shaped to support the sole of a patient's foot. Attached to the underside of sole piece 154 are two adjustable foot pads 156 which can be moved into contact with the sides of a patient's foot and locked into place with a wing nut. These pads serve to maintain correct alignment between the patient's foot and patient applied part 150. Curved plastic part 152 and foot pads 156 are covered with foam rubber padding and vinyl plastic to distribute pressure on the patient's foot and allow patient applied part 150 to be easily cleaned. Two Velcro™ straps 158 are attached to plastic part 152 so that they can be wrapped tightly over the patient's foot and fastened to maintain the position of the patient's foot with respect to patient applied part 150. One of Velcro™ straps 158 is located over the patient's ankle to hold the patient's heel firmly in curved plastic part 152 and against plastic sole piece 154. The other Velcro$^{TM}$ strap 158 is located over the distal part of the patient's tibia to ensure correct alignment of the patient's leg with patient applied part 150.

Located in the heel portion of patient applied part 150 is heel sensor 160. Heel sensor 160 is constructed in a similar fashion to environmental/safety sensors 144. Sensor 160 is attached to patient applied part 150 in two places such that it fits into the heel pocket of patient applied part 150. Heel sensor 160 facilitates detection of proper positioning of the patient's heel in patient applied part 150. If the patient's heel moves out of its proper position during movement of manipulator 38, sensor 160 produces an alarm signal which is detected by computer 18. Upon detection of the alarm signal, computer 18 computes an alternate path for movement of manipulator 38 to maintain proper positioning of the patient's heel within the heel pocket of patient applied part 150, thereby preventing the application of forces to the patient's limb while the limb may not be oriented to accomodate such forces without injury to the patient.

Patient applied part 150 is attached to patient applied part mounting bar 138 with two bolts. Alternatively, patient applied part 150 may be attached to patient applied part mounting bar 138 with a mechanism which would allow patient applied part 150 to be released and detached from patient applied part mounting bar 138. This would allow patient applied part 150 to be attached to and detached from the patient's foot more easily. This alternative attachment mechanism could further be constructed in such a way that any force which exceeds a pre-determined limit would cause the mechanism to be detached from manipulator 38. This would act as a "mechanical fuse", which would prevent excessive forces from being transmitted to the patient's foot. In addition, an electronic sensor could be incorporated in this mechanism so that forces in excess of a pre-determined value could be detected by computer 18 so that appropriate action could be taken to prevent injury to the patient.

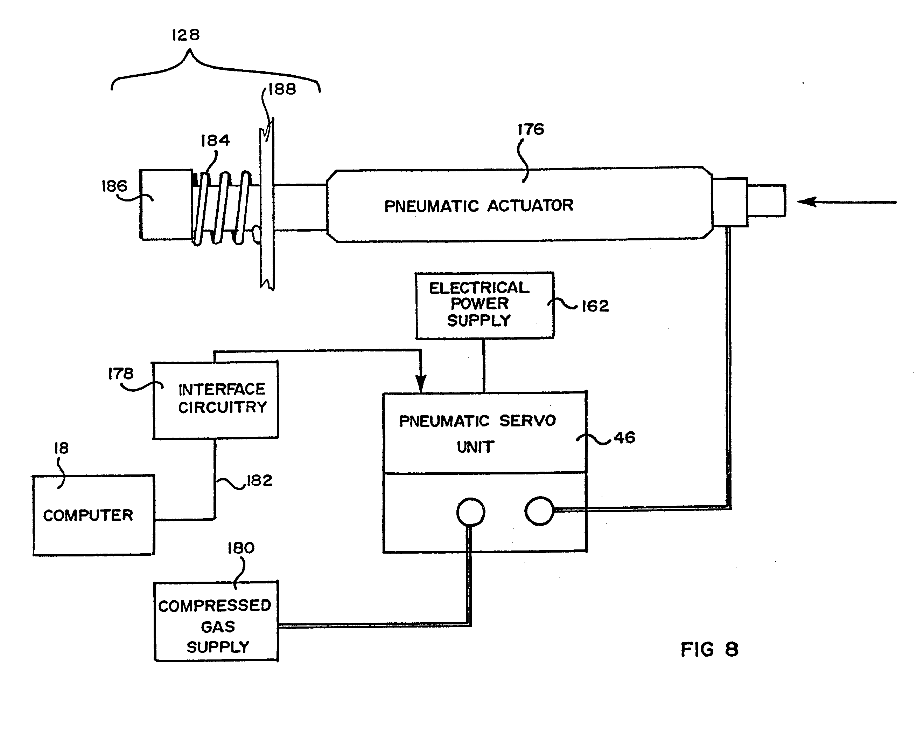

Air lines connected to pneumatic actuators 140 and 126, hydraulic lines connected to hydraulic actuators 64, 76, 90, and 104, and power and signal lines connected to position sensors 68, 74, 88, 102, 130, 132 and 142 are all brought together in first arm section 62 where they are bundled into a single umbilical cable 146 (Figures 2 and 3), which is connected to control box 44. Signal and power supply lines connected to positon sensors 68, 74, 88, 102, 130, 132 and 142 are connected to computer 18 and to power supply 162 (Figure 8) inside control box 44. Also incorporated in umbilical cable 146 is a signal line, one end of which is connected inside control box 44 to computer g18, the other end of which terminates at an electrical connector 164 (Figure 4) located near shoulder structure 48. During normal operation, an operator's hand control 166 may be connected to connector 164.

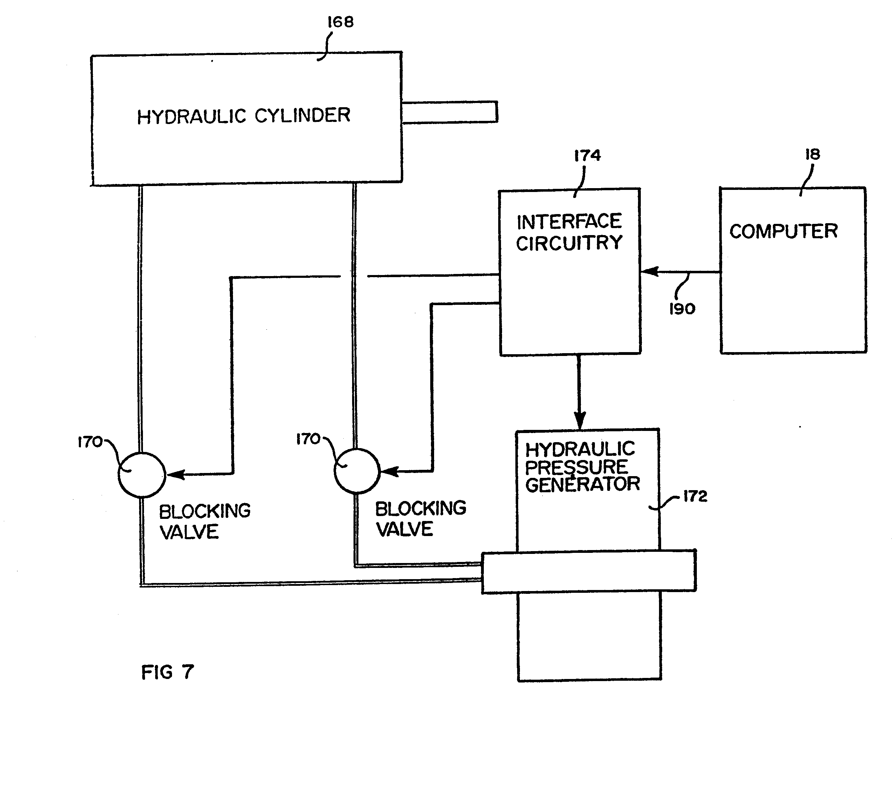

Figure 7 illustrates a single hydraulic actuator system representative of that which comprises each of the four hydraulic actuators 64, 76, 90 and 104. Each hydraulic actuator system consists of a double acting hydraulic cylinder 168, two blocking valves 170 and a hydraulic pressure generator 172, all connected together with 1/8" plastic tubing. Hydraulic pressure generator 172 is a reversible device which can generate pressure at either of two ports while accepting return flow at the other port, thereby returning oil to an internal reservoir. Interface circuitry 174 is connected to hydraulic pressure generator 172, blocking valves 170 and communications lines connected to computer 18. In the preferred embodiment hydraulic cylinders 168 used in the four hydraulic actuators are each Compact Air$^{TM}$ (Compact Air Products Inc., Westminster S.C., USA) model S118X212-RC-HYD-FT hydraulic cylinders, each having a 1 1/8" diameter bore and 2 1/2" stroke. Blocking valves 170 used in the preferred embodiment are Precision Dynamics$^{TM}$ (Precision Dynamics Inc. New Britain CT., USA) model A2011-BB-120/60 120 volt AC valves, and hydraulic pressure generator 172 is an Oildyne$^{TM}$ (Oildyne Inc., Minneapolis, Minnesota, USA) model 108DA19-AL-LVT power unit. All hydraulic components are connected together with Legris$^{TM}$ (Legris Ltd., Rochester, N.Y., USA) 140-53-00 1/8" plastic

31

tubing.

In operation, blocking valves 170 are opened, and hydraulic pressure generator 172 is activated. Pressurized oil is forced out of one port of generator 172 into one side of hydraulic cylinder 168, causing its piston to move and displace oil from the other side of cylinder 168, which is returned to the second port of hydraulic pressure generator 172. When the hydraulic actuator is required to stop moving, hydraulic pressure generator 172 is turned off and blocking valves 170 are closed, preventing oil from moving in to or out of either side of hydraulic cylinder 168.

Also incorporated in interface circuitry 174 is a re-triggerable "watch dog" timer, the trigger of which is connected to computer 18. The output of this timer is connected to a logic controlled voltage regulator, which supplies power to the rest of the circuitry comprising interface circuitry 174. When computer 18 is operating correctly, it generates a trigger pulse at regular intervals, thereby triggering the timer, which in turn enables the logic controlled regulator and interface circuitry 174. The time constant of the "watch dog" timer is slightly longer than that of the pulses expected from computer 18, such that during normal operation of computer 118, the power to circuitry 174 is not interrupted. In the event of a failure of computer 18 or associated circitry, the "watch dog" timer will shut off the power to interface circuitry 174, thereby disabling hydraulic pressure sources 172 and blocking valves 170 to ensure that no unpredictable or uncontrolled motions of hydraulic actuators 168 occur.

Figure 8 illustrates a pneumatic actuator system representative of that which comprises each of actuators 126 and 140 (six pneumatic actuators in all). Each actuator consists of a pneumatic actuator 176, a tensioning device 128, pneumatic servo unit 46 and interface circuitry 178. Pneumatic actuator 176 is, in the preferred embodiment, a Bridgestone 18 mm diameter Rubberturator[TM] (Bridgestone Corporation, Tokyo, Japan). Actuators 126 are each 300mm long, while actuators 140 are 200mm long. Pneumatic servo units 46 used in the preferred embodiment are Bridgestone Corp. ACFAS[TM] model SVO-102-04 air servo units. All pneumatic components are connected together with Legris[TM] 140-53-00 1/8" plastic tubing. Each pneumatic servo unit 46 is connected to a compressed gas supply 180, an electrical power supply 162, and interface circuitry 178. Interface circuitry 178 is also connected to computer 18 via communications lines 182. The pneumatic servo units 46 used in the preferred embodiment are dual units, each of which is able to control two actuators.

In operation, pneumatic servo unit 46 is activated by computer 18 to control the pressure of gas delivered to inflate actuator 176. As the pressure of gas delivered to actuator 176 increases, the inflating actuator increases in diameter and decreases in length, thereby exerting a force

33

between the end points at which it is coupled to the arm sections etc. When not inflated, actuator 176 is pulled straight (in accordance with the manufacturer's recommendations) by tensioning device 128, which consists of tensioner spring 184, cap 186 and retaining plate 188. When actuator 176 is inflated, tensioner spring 1158 is compressed, allowing cap 186 to touch retaining plate 188, and transmit forces from pneumatic actuator 176 thereto, thereby preventing excessive deformation of spring 184.

Pneumatic actuators 176, in conjunction with pneumatic servo units 46, can be controlled by computer g118 to provide variable compliance. In normal operation, an opposed pair of pneumatic actuators 176 are connected to provide opposing motions at a single joint or degree of freedom. Since each pneumatic actuator can apply force in only one direction (i.e. in the direction of its longitudinal axis, as the inflating actuator decreases in length), the two actuators must act in tandem to achieve and maintain a desired position. This may be achieved by setting the pressure in one actuator at a constant level and by adjusting the pressure in the opposed actuator to a level appropriate to achieve the desired position. If the constant set pressure of the first actuator is low, it will offer little resistance to displacement, and hence a high degree of compliance. If, on the other hand, the constant pressure in the first actuator is set to a high level, it will offer a high degree of resistance to displacement and hence a low degree of compliance. In the preferred
embodiment, this compliance is varied in response to the
patient's limb weight to ensure that only the amount of
force required to move a particular patient's limb will be
exerted on that patient.

Control box 44 (Figure 2) contains four hydraulic
pressure generators 172, three pneumatic servo units 46
each capable of driving two pneumatic actuators 176), and
interface circuitry 174 and 178. Also enclosed in control
box 44 is electrical power supply 162, which in the
preferred embodiment is a switching power supply module,
Hammond$^{TM}$ model GFOL 101, (Hammond Electronics Inc.,
Buffalo, N.Y. USA), which provides 5 volts DC at 10amps, 15
Volts DC at 3 amps, -15 volts DC at 3 amps, -5 volts DC at
2 amps, and 24 volts DC at 2 amps. Power supply 162
provides power for interface circuitry 178 and 174, as well
as for pneumatic servo units 46.

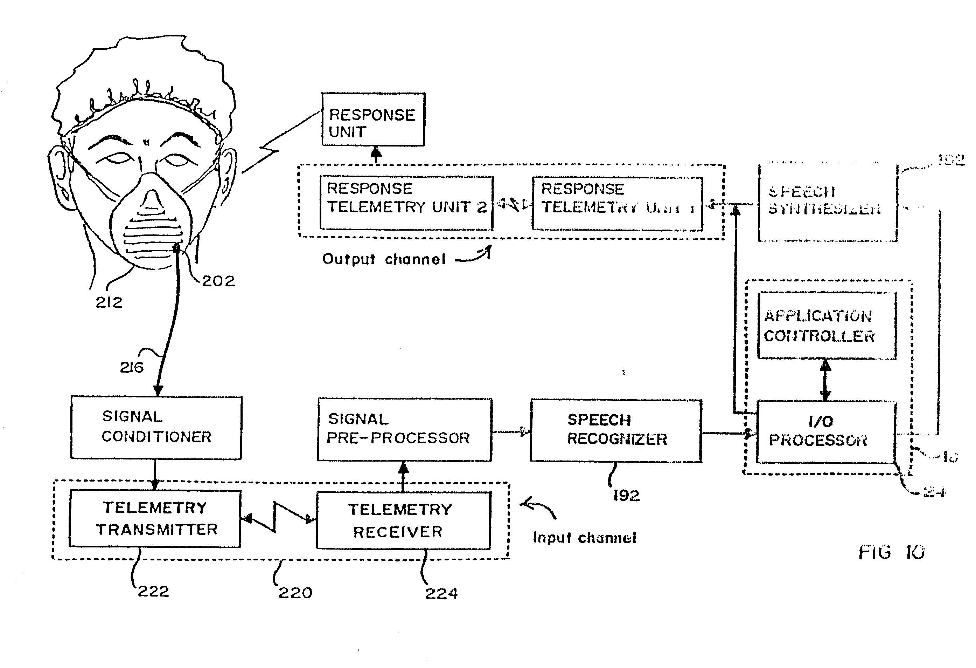

Computer 18 is an IBM$^{TM}$ personal computer
(International Business Machines Corporation, Boca Raton,
Florida, USA) having a monchrome monitor, keyboard,
Intel$^{TM}$ 8087 numerical co-processor, (Intel Corporation,
Santa Clara, California, USA) and dual floppy disk drives.
Input/output processor 24 is a Data Translation$^{TM}$ DT2801
board, (Data Translation Inc., Marlborough, Massachusetts,
USA) within computer 18, which provides sixteen channels of
analog input (of which 7 are used for position sensor
signals, and one is used for operator control signals), and sixteen channels of digital output (of which 4 are used to control blocking valves 170, 8 are used to control hydrualic pressure generators 172, and 2 are used to sense the status of environmental/saftey sensors 144 and heel sensor 160), and a second circuit board within computer 18, a Tecmar Lab Tender$^{TM}$ board, (Tecmar Incorporated, Cleveland, Ohio, USA) which provides 16 analog outputs (of which 6 are used to control pneumatic servo units 46). Communication lines 182 (Figure 8) and 190 (Figure 7) are connected to the two circuit boards just mentioned at the rear of computer 18, and to interface circuitry 174 and 178 located in control box 44. In the preferred embodiment, communications line 190 is a 40 conductor ribbon cable connected to the Data Translation$^{TM}$ DT2801 circuit board, and communications line 182 is a 34 conductor ribbon cable connected to the analog output connector of the Tecmar$^{TM}$ Lab Tender circuit board. An additional circuit board, an NEC$^{TM}$ SAR-10 speech recognition and synthesis circuit 192 (Figure 10), (NEC Corporation, Tokyo, Japan) is also included in computer 18. This circuit board may be connected to a wireless telemetry link to receive voice signals from the surgeon, and to a loudspeaker located in control box 44 through which audible responses may be generated.

The speech recognizer portion of circuit 192 must be "trained" to recognize the surgeon's speech pattern by having the surgeon repeat previously defined command words into a microphone connected to the speech recognition circuit until an acceptable recognition level is attained. This training provides the speech recognition circuit with a pattern against which to match the surgeon's voice commands during normal operation of manipulator 38. Similarly, the speech synthesis portion of circuit 192 must be trained to produce speech patterns before audible responses can be generated. Once these speech recognition and speech synthesis patterns have been developed, they can be stored permanently for future recall on a floppy disk using the disk drive unit included in computer 18.

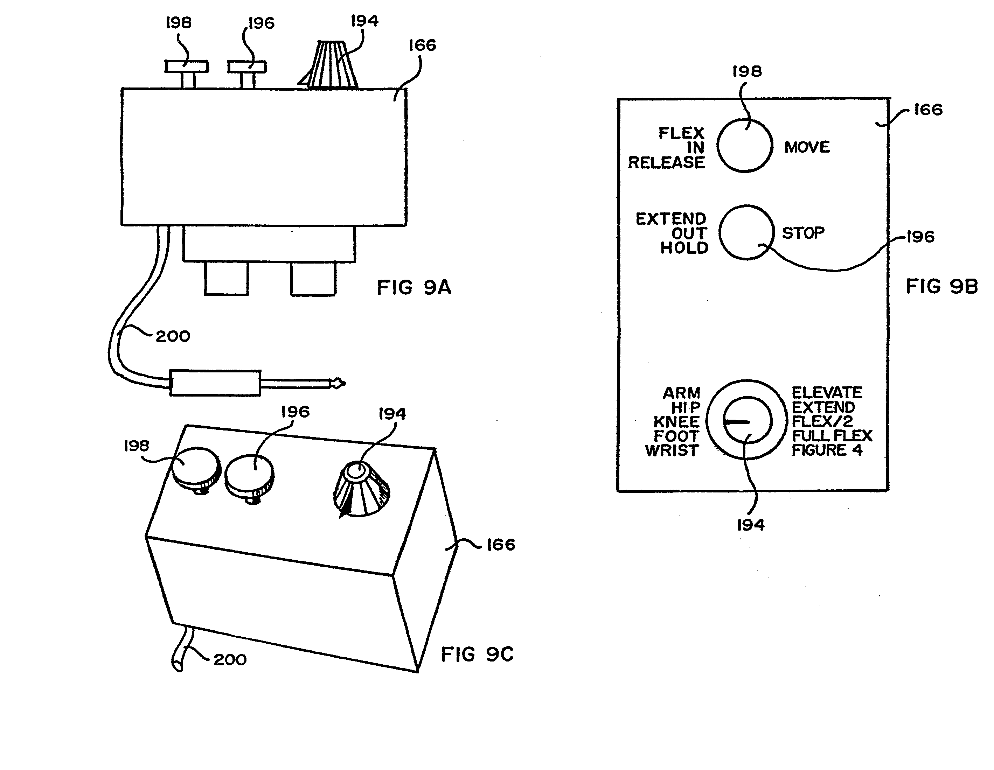

Operator hand control 166 (Figures 2 and 9) consists of selector switch 194, and function switches 196 and 198. Selector switch 194 and function switches 196 and 198 are wired to computer 18 via pendant cable 200. In normal operation, cable 200 is coupled to operator hand control connector 164 (Figure 4). Selector switch 194 is a multi-position selector switch which is wired to generate a distinct voltage for each position of the switch. Function switches 196 and 198 are similarly wired to generate distinct voltages when activated. Selector switch 194, function switches 196 and 198 and associated circuitry are contained in a small metal box. The box and switches are all sealed with latex rubber sheet to prevent fluids from entering the circuitry. Attached to the back of the metal box is a spring clip which may be attached to the arthroscope trocar sleeve usually used during

37

arthroscopic surgery, such that operator hand control 166 remains within the surgeon's reach. The knob of selector switch 194 is inscribed with an indicator (Figure 9B) which points to inscriptions on the surface of the metal box describing the function of each position of switch 194. The functions of each of switches 196 and 198 are also inscribed on the surface of the metal box at appropriate locations. In an alternate embodiment, the edge of the knob of selector switch 194 could be incribed so that it could be read by an operator viewing the hand control from the side. In the preferred embodiment, selector switch 194 is a 10 position Grayhill™ model 2301026 BCD coded switch, and function switches 196 and 198 are Grayhill™ model 39-1 pushbutton momentary contact switches.

In normal use, operator hand control 166 is sterilized using established gas sterilization techniques before it is used in a surgical procedure, so that its use will not compromise the surgeon's sterility.

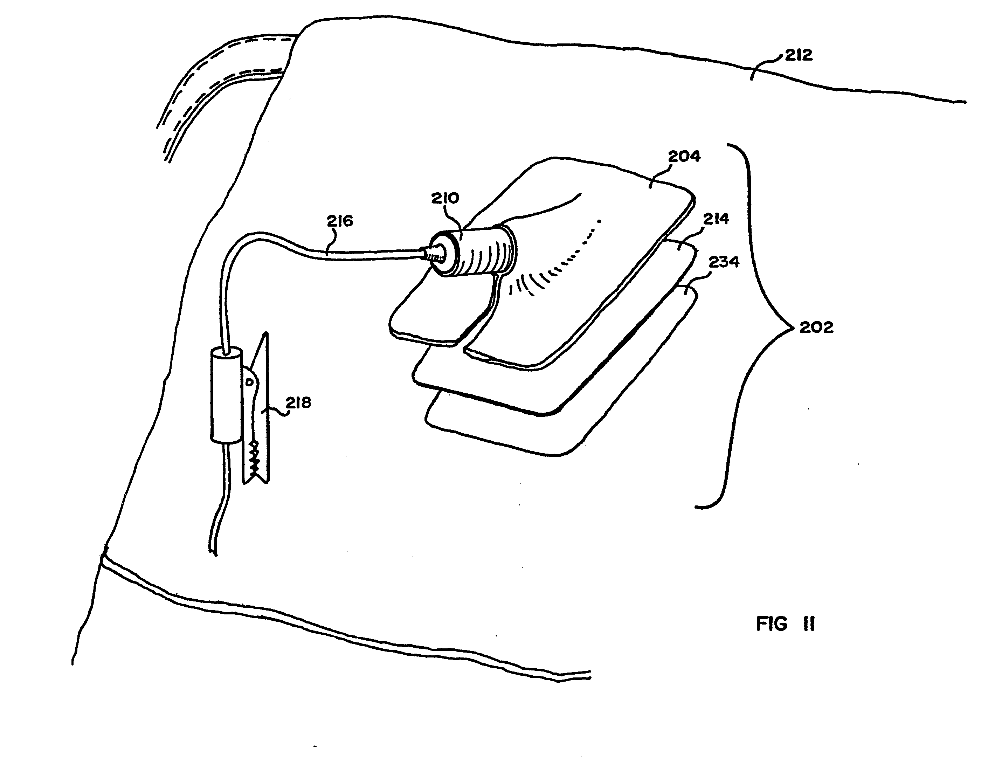

In the preferred embodiment, collection of voice from the surgeon is facilitated by the use of speech collector 202 as shown in Figures 10 and 11. The speech collection apparatus consists of a single piece of plastic polymer material such as polyvinyl-chloride which may be positioned within a surgeon's face mask to maximize the acoustic level of speech which impinges upon an electrical transducer, such as a microphone, within the speech collection apparatus, without impairing the mask's bacterial filtration capability. This plastic shell 204 may be injection moulded or thermoformed in a manner known to one skilled in such arts. Plastic shell 204 has a generally rectangular periphery which is shaped to have rounded corners and smooth edges. Shell 204 also has a sloping ridge 206, which is generally cylindrical in shape, surrounded by a flat area which is the portion of the shell which adheres to the surgeon's surgical mask during normal use (it will be noted that the drawings show various types of surgical masks, to illustrate that this aspect of the invention may be adapted for use with a wide variety of mask configurations). A hole 208 (of diameter slightly less than that of microphone 210), is drilled in plastic shell 204 at one end of sloping ridge 206. A slit is cut in plastic shell 204 from the edge of hole 208 to the edge of the flat part of shell 204 to allow the hole to be spread open by slight deformation of shell 204. In normal use, shell 204 is thus deformed to fit microphone 210 into hole 208. Microphone 210 is then held in place by friction. Plastic shell 204 is attached to surgical mask 212 with a piece of double sided adhesive 214 at a point selected to maximize the acoustic level of speech sounds incident upon microphone 210. Microphone cord 216 of microphone 210 is attached to a small spring clip 218 which may be attached to the surgeon's clothes at a convenient location in order to reduce the strain on microphone cord 216, and reduce the possibility of microphone 210 being

39

dislodged from plastic shell 204. Cord 216 may be directly connected to a signal receiver or it may be coupled to a miniature signal transmitter worn by the surgeon as hereinafter explained.

In the preferred embodiment, microphone 210 is a Sony™ electret condenser microphone model ECM-150T (Sony Corporation, Tokyo, Japan) and adhesive 214 is a piece of 3M™ 950 transfer tape (3M Canada Inc., London Ontario, Canada).

In operation, operator hand control 166 and speech recognition and synthesis circuit 192 are used in conjunction with each other to provide 'eyes free' control of manipulator 38. Every change in the setting of selector switch 194 will cause speech synthesizer 192 to produce an audible response describing the selection presently selected by selector switch 194. If the surgeon so desires, he may initiate the action selected by pressing one of the two function switches 196 or 198. Function switches 196 and 198 are usually programmed to offer opposing functions such as go and stop, more and less foot rotation, or more and less knee flex, although they may be programmed to perform any desired functions. With each activation of function switches 196 and 198, another audible response is generated, describing the function being executed. In addition, the surgeon may choose to make a selection by speaking one of the pre-programmed commands into microphone 210, and then initiate the

40

function with function control switches 196 or 198, or alternatively, make a selection by moving selector switch 194 and initiate the desired action by speaking one of the pre-programmed commands into the microphone.

Input telemetry channel 220 (Figure 10) is implemented with a modified Radio Shack™ FM wireless microphone system (Tandy Electronics, Barrie Ontario, Canada). A low power, pocket sized telemetry transmitter 222 is powered by two AA size batteries and may be conveniently worn in a shirt pocket under the surgical gown. Transmitter 222 is modifed by replacement of the microphone supplied by the manufacturer with an electrical connector matched to that supplied by the manufacturer of microphone 210. Signal conditioning (i.e. bandwith limitation) is performed by telemetry transmitter 222. Telemetry receiver 224 (Figures 2 and 10) is implemented with a Radio Shack™ FM wireless microphone base unit, the microphone output of which is coupled to the microphone input of speech recognizer 192.

### III. Operation by Surgical Staff

As shown in Figure 2, patient 12 is positioned on operating room table 42 in a normal position for arthroscopic surgery and anesthetized in accordance with established medical procedures. The limb which is to be operated upon is prepared by applying a sterile stocking or soft bandage.

Manipulator 38, computer 18, control box 44 and

41

wheeled base 181 are wheeled into a position approximately one meter from operating room table 42, preferably on the side closest to the surgical site. Manipulator 38 is removed from holding brackets 182 located on the back of control unit 44, and mounted on the appropriate side of operating room table 42. This is done by hanging the lip of notch 50 (Figure 4) cut into mounting block 52 over the top edge of operating table side rail 40. Manipulator 38 is positioned correctly by sliding mounting block 52 along side rail 40 until mounting block 52 is located at a position approximately 55 centimeters proximal to the center of the patella of the limb to be operated upon. Manipulator 38 is then secured to side rail 40 by tightening clamping bolt 56.

Control box power cord 183 is plugged into a 110V a.c. power source. The power cord for computer 18 may be plugged into an outlet provided at the rear of control box 44. Ribbon cables comprising communication lines 182 and 190, protruding from control box 44 are plugged into mating receptacles on the back of computer 18. A speaker wire protruding from the back of control box 44 is plugged into a jack labelled "SPK" on back of computer 18. Compressed gas supply line 184 is connected between control box 44 and the operating room compressed gas supply outlet or a compressed gas bottle. The gas supply pressure is then adjusted to 40 - 60 PSI.

If speech control is to be used, microphone 210 is fitted into speech collector 202. Adhesive backing 234 is removed to expose the adhesive back 214 of speech collector 202. Speech collector 202 is then applied to surgical mask 212 to be worn by the surgeon, where it is held in place by adhesive back 214. Speech collector 202 should be located on surgical mask 212 such that it is near either corner of the surgeon's mouth. Microphone cord strain relief 218 is clipped to the surgeon's collar, and microphone cord 216 is plugged into the jack labelled "MIC" on the back of computer 18, using a microphone extension cord if required. The power switch for microphone 210, located at the end of microphone cord 216 is turned on.

If telemetry channel 220 is to be used, the above connections are altered. Microphone cord 216 is plugged into telemetry unit transmitter 222, which may then be placed in the surgeon's pocket. The microphone output of telemetry receiver 224 is connected to the jack labelled "MIC" on the back of computer 18, and the unit is then placed on top of computer 18. Both telemetry transmitter 222 and telemetry receiver 224 are turned on via their respective power switches.

If operator hand control 166 is to be used, it is removed from its sterile packaging as per standard sterile technique, and is connected to hand controller connection 164. If desired by the surgeon, operator hand control 166 may be clipped on to the arthroscope trocar.

A copy of the operating software, stored on a floppy disk, is inserted into the disk drive included in computer 18. Computer 18 and control box 44 are then turned on.

Computer 18 will automatically begin loading all necessary software, and then will display the following message on the video monitor included in computer 18:

strike ALT 1 for Dr. Day's speech patterns

strike ALT 2 for Rob's speech patterns

strike ALT 3 for Geof's speech patterns

strike ALT 4 for Glenn's speech patterns

where "Dr. Day", "Rob", "Geof", and "Glenn" are individuals whose speech patterns speech recognizer/synthesizer 192 has been "trained" to recognize as desribed above.

If speech control is to be used and if the operator's name appears on the monitor, the appropriate keys should be pressed as indicated. This will load both that operator's speech recognition data and the Arthrobot system voice synthesis data into the memory of computer 18. If the operator's name does not appear on the monitor, then the operator must complete a voice training session, which may be accomplished by typing 'vpu' followed by 'enter'. This causes execution of the voice training software provided by NEC$^{TM}$ with speech recognition and synthesis circuit 192. This software may be used in the manner described by NEC^TM to perform the necessary training. If speech control is not to be used, then the operator should stike ALT 1. This will load Dr. Day's speech recognition data (which will not be used) as well as the system voice synthesis data.

Computer 18 will then exectue the control software, which begins by prompting the operator to identify which leg, left or right, of patient 12 is to be operated upon. The operator should enter 'l' or 'r', followed by 'enter' to indicate the left or right leg. When all personnel and equipment are clear of the workspace of manipulator 38, the operator should then press the '2' key, which will cause computer 18 to speak "SETUP" via speech synthesizer 192. The operator should then press the grey '-' key, which will cause computer 18 to say "MOVE", and will cause manipulator 38 to swing such that it is parallel with operating room table 42 and to extend such that patient applied part 150 is brought level with operating room table side rail 40. The operator may be required to gently push manipulator 38 in the direction of the operating room table to help manipulator 38 past the position in which the main structure of manipulator 38 is perpendicular to the operating table side rail 40.

When manipulator 38 has stopped moving, the patient's foot is fitted into patient applied part 150. If required, the two arrow keys marked "<-" and "->" may be

45

used to move the manipulator in motions parallel with table side rail 40 so as to simplify application of patient applied part 150.

The patient's foot is attached to patient applied part 150 by the operator by locating the patient's heel in the heel portion of patient applied part 150. Velcro™ ankle strap 158 is pulled tightly across the patient's ankle and secured. The second Velcro™ strap 158 is pulled tightly over the patient's tibia and secured. Adjustable foot pads 156 are moved together so that they touch on both sides of the patient's foot. If the patient's foot is properly inserted in patient applied part 150, heel sensor 160 will be activated. This is indicated by computer 18 with a message printed on the screen of computer 18. If heel sensor 160 is not activated, the operator should remove the patient's foot from patient applied part 150 and re-locate the patient's heel in the back of patient applied part 150. During normal operation, if the patient's foot is pulled upon, or for some reason dislodged from the patient applied part, heel sensor 160 will detect this change, and cause computer 18 to halt it's present motion and alert the operator.

When the patient's foot is properly attached to patient applied part 150, the operator uses the two arrow keys, "<-" and "->" to move manipulator 38 into a position such that the patient's leg is fully extended, level with operating table 42, after which the operator strikes the "ALT" and "A" keys simultaneously to indicate to computer 18 that the patient's limb is correctly positioned.

Computer 18 will then ask the operator to check that the patient's leg is in the correct position. If it is not, the operator should type 'N', which will allow further adjustments to be made. If the operator types 'Y', computer 18 prompts for the length in millimeters from the center of the patient's patella to the bottom of the patient's heel. This measurement is entered, followed by the 'enter' key. The control software uses the current "full extension" position and the entered data to adapt its operation to the patient. This includes the establishment of safe limits of operation for manipulator 38, calculation of correct positions for manipulator 38 in order to achieve the five pre-programmed positions for surgery, and calculation of paths for anatomically correct motions. Safe limits are imaginary lines drawn within the workspace of manipulator 38 which define the range of anatomically natural motion of each portion (i.e. foot, lower leg and upper leg) of each particular patient's leg. These imaginary lines or safe limits define a subsection of the workspace of manipulator 38, outside of which computer 18 will not allow manipulator 38 to move. The five pre-programmed positions (elevate, full extension, full flex, half flex, figure four) are similarly scaled, such that these positions are anatomically correct for each patient. Anatomical motion is the action of flexing or extending either the patient's knee joint or the patient's hip joint without affecting the angle of the other joint.

Computer 18 prompts the operator via its display for permission to begin calibration of the compliance of actuators 126 and 140 (hereinafter collectively called the "wrist actuators"). If the operator types 'N', this calibration step is skipped, and the compliance is set to a pre-set level. If the operator presses the 'Y' key, manipulator 38 moves the patient's leg into a figure 4 position. When this position is achieved, computer 18 increases the compliance of the pneumatic actuators located in the wrist of manipulator 38, until the patient's leg weight causes the manipulator to shift position. When this motion is detected, the compliance is gradually reduced, until the originally programmed position is regained. The level of compliance needed to regain this position is then recorded and maintained for the duration of the surgical procedure, or until the operator specifically changes it. In this manner, the compilance of the wrist actuators of manipulator 38 is scaled to the patient's leg weight.

When the compliance of manipulator 38 is scaled, the operator will usually press the "F2" key, followed by the grey "-" key, to position the patient's limb in the "elevate" position, in order to facilitate tourniquet application, preparation solution application, and application of a sterile drape.

During surgery, the surgeon may alter the position

48

or operation of manipulator 38 without moving his eyes from the surgical task at hand, using two different interface devices in combination. These are operator hand control 166, and voice recognizer 192. An operator other than the surgeon may intervene or assist the surgeon by pressing the appropriate keys on the keyboard of computer 18. Should a situation arise in which it is necessary to quickly disable manipulator 38 the operator may press the 'space' bar; this will cause manipulator 38 to immediately halt all functions in progress. This same action by be intiated by the surgeon by speaking 'stop' or by pressing the 'stop' button on operator hand control 166.

Computer 18 is programmed to accept commands from speech recognizer 192 and operator hand control 166 interactively, such that both command methods may be used together to construct a single command sequence that will cause manipulator 38 to execute the desired function. A command sequence consists of three consecutive steps: 1) the attention prefix, 2) the selection command, and 3) the action command. The table below illustrates how individual speech commands and operator hand controller commands produce each of the three steps of a command sequence:

| Command | Speech | Hand Control |
|---------|--------|--------------|
| Sequence | Commands | Operation |

49

| Step | User Input | System Output | User Input | System Output |
|---|---|---|---|---|
| Attention Prefix | Surgeon speaks 'ARTHROBOT' | Voice reply 'YES' | Surgeon rotates selector switch | No Reply |
| Selection Command | Surgeon speaks selection word | Voice synthesizer repeats selection word | Surgeon stops rotation of selector switch at desired function indication | Voice synthesizer repeats selection word corresponding to function indicated on selector switch |
| Action Command | Surgeon speaks action word | Voice synthesizer repeats action word | Surgeon presses function switch 196 or 198 | Voice synthesizer repeats action word corresponding to selected action |

Both operator hand control 166 and speech recognizer 192 can be used independantly, since entire command sequences can be entered using either input device alone. The seperate operation of operator hand control 166 and voice recoginzer 192 will now be described, but it should be understood that these two input devices can be used interactivly as descibed above.

As described above, operator hand control 166 is usually clipped to the arthroscope trocar, so that it remains in a position convenient for the surgeon. When the surgeon wishes to use operator hand control 166, he rotates selector switch 194, to indicate to computer 18 that a command sequence is beginning. This performs the function of the "attention prefix" described above. Each time selection switch 194 is rotated to new a position, computer 18 will use voice recognition and synthesis circuit 192 to verbally indicate the new position of selection switch 194. When the surgeon hears the manipulator function he desires, he ceases rotating selector switch 194. Pushbuttons 196 and 198 evoke different manipulator functions, depending upon the position of selection switch 194, according to the table below. Pushing either button 196 or 198 will cause computer 18 to verbally indicate the action to be taken and then move manipulator 38 as required. Visual confirmation of the setting of selector switch 194 and the functions of function switches 196 and

51

198 can be made by reading the label on the face of operator hand control 166.

| Selection Switch Setting | Description | Switch 196 | Switch 198 |
|---|---|---|---|
| Elevate | Elevate Leg | Move | Stop |
| Extend | Full Extension of Leg | Move | Stop |
| Flex/2 | Half Flex of Leg | Move | Stop |
| Full Flex | Full Flex of Leg | Move | Stop |
| Figure Four | Position leg in Figure "4" | Move | Stop |
| Arm | Move Leg Relative to O.R. Table | In | Out |
| Hip | Anatomical Movement of Hip | Flex | Extend |
| Knee | Anatomical Movement of Knee | Flex | Extend |
| Foot | Rotate Foot internally and | In | Out |

52

| Wrist | externally Control of wrist | Release | Hold |
|---|---|---|---|

---

When the surgeon wishes to use voice input, he must speak three command words to alter the position or operation of manipulator 38. This three word command sequence is used as a verification scheme to prevent falsely recognized commands from activating manipulator 38. The attention prefix 'ARTHROBOT' begins the command sequence: it alerts speech recognition and synthesis circuit 192, which causes it to listen for a command word. Computer 18 acknowleges recognition of the attention prefix and the begining of a new command sequence by generating the verbal response 'YES'. The second word spoken by the surgeon may be one of the ten manipulator selection command words listed in the table below, in which case, computer 18 causes speech recognition and synthesis circuit 192 to repeat the selection command word, if it is recognized. If the word spoken is not recognized, computer 18 will emit a short beep after which the surgeon may repeat the selection command word, for another recognition attempt. If the surgeon's spoken manipulator selection command word is not recognized within twenty seconds following acknowledgement

53

of the word 'ARTHROBOT', then speech recognition and sythesis circuit 192 will cease listening until the command sequence is re-initated by the surgeon speaking the attetion prefix 'ARTHROBOT'. If the selection command word recognized by computer 18 is not the one desired by the surgeon, as indicated by the verbal response produced by synthesizer 192, then the surgeon may again speak the selection command word, or say 'NO' to cancel the command sequence. If the command sequence is terminated in this way, computer 18 will verbally respond with the word 'SORRY'. The surgeon then has a twenty second period in which to repeat his desired selection command word. If the surgeon does not succeed in having any selection command word recognized during this period, speech recognition and synthesizer circuit 192 will cease listening until the command sequence is re-initiated with the attention prefix 'ARTHROBOT'.

Once the surgeon's desired selection command word has been successfully recognized, the action of manipulator 38 may be initiated with one of the appropriate action command words, as listed in the table below. When computer 18 recognizes a valid action command, it initiates the corresponding action of manipulator 38 and verbally confirms the action command via speech recognition and synthesis circuit 192. If the spoken manipulator action command is not recognized, computer 18 will emit a short beep, after which the surgeon may attempt to have his

54

action command recognized again. If the surgeon fails to
have his action command recognized within a twenty second
period following acknowledgement of the attention prefix
'ARTHROBOT', then speech recognintion and synthesis circuit
192 will cease listening until the command sequence is
re-initiated by the surgeon speaking the attention prefix
'ARTHROBOT'. If the surgeon has selected one of the
incremental motion commands, (i.e. ARM, HIP, KNEE or FOOT)
he may choose to repeat one or both of the associated
action commands to continue to change the position of
manipulator 38, without having to repeat the entire command
sequence.

| Command Sequence Step | Valid Manipulator Command Word(s) | Description |
|---|---|---|
| Attention Prefix | ARTHROBOT | Initiates command |
| Selection Command | ELEVATE | Elevate leg |
| | FULL EXTENSION | Fully extends leg |
| | HALF FLEX | Half flex the leg |
| | FULL FLEX | Full flex the leg |
| | FIGURE 4 | Position in figure 4 |
| | ARM | Moves leg toward or away from table |

55

| | HIP | | Anatomical motion of hip joint |
|---|---|---|---|
| | KNEE | | Anatomical motion of knee joint |
| | FOOT | | Rotation of foot |
| | WRIST | | Manipulator wrist force setting |
| Action Commands | For ELEVATE: | MOVE | Begins motion |
| | | STOP | Ends motion |
| | For FULL EXTENSION: | MOVE | Begins motion |
| | | STOP | Ends motion |
| | For HALF FLEX: | MOVE | Begins motion |
| | | STOP | Ends motion |
| | For FULL FLEX: | MOVE | Begins motion |
| | | STOP | Ends motion |
| | For FIGURE 4: | MOVE | Begins motion |
| | | STOP | Ends motion |
| | For ARM: | IN | Moves leg toward table |
| | | OUT | Moves leg away from table |

56

| | | | |
|---|---|---|---|
| For HIP: | | FLEX | Flexes hip |
| | | EXTEND | Extends hip |
| For KNEE: | | FLEX | Flexes knee |
| | | EXTEND | Extends knee |
| For FOOT: | | IN | internally rotates foot |
| | | OUT | externally rotates foot |
| For WRIST: | | RELEASE | Release manipulator wrist |
| | | HOLD | Hold manipulator wrist |
| Other Commands | NO | | Verification incorrect |
| | HALT | | Immediately halts manipulator actions |

At any time during the surgical procedure, if an obstruction is detected during motion, such that environmental/saftey sensor 144 is activated, manipulator 38 will immediatly stop moving and will alert the operator. The operator should then clear the obstruction or determine that manipulator 38 is still operating in a safe manner, and then use operator hand control 166 or a voice command to continue moving the manipulator in the

57

desired direction.

At the end of the surgical procedure, manipulator 38 is moved to the elevated position, the surgical draping and tourniquet are removed and the patient is bandaged according to standard medical practice. The manipulator may then be moved to the full extension position to facilitate removal of the patient's foot from patient applied part 150. Manipulator 38 is then moved to the "nested" position by simultaneously pressing the 'ALT' and 'N' keys on the keyboard of computer 18. This causes manipulator 38 to fold into its most compact configuration to facilitate removal from operating table 42. When clamping bolt 56 is loosened, manipulator 38 can be lifted off of operating table side rail 40 and returned to holding brackets 182 on the back of control box 44.

### IV.  Software

A complete source code listing of the computer software used with computer 18 to control manipulator 38 is appended as part VI of this specification. Computer 18 uses IBM$^{TM}$ DOS version 3.0 for an operating system. Two auxillary software programs are used during normal operation of the manipulator: Prokey$^{TM}$ version 2.13 (RoseSoft Co., Seattle, Washington, USA), and the Voice Plus Utilities version 1.01 software package by NEC. Prokey is used to program the keys of the IBM personal computer for special functions, and the Voice Plus Utilities provide several programs to communicate with the NEC SAR-10 speech recognition and synthesis circuit board 192. All custom software for manipulator 38 is written in the "C" language. The "C" compiler used to convert the "C" code to an executable program is the C86$^{TM}$ version 2.3 compiler, (Computer Innovations, Inc., Red Bank, New Jersey, USA) which has been configured as per manufacturer's specifications to support the 8087 numeric co-processor included in computer 18. Also included with the listing are the AUTOEXEC.BAT file used by the DOS 3.0 program to automatically configure computer 18 when started, and the AUTOARTH.PRO file used by Prokey to program the keybord of computer 18.

Although the attached software listing and comments embedded therein are believed to be complete and sufficient to enable one skilled in the art to understand the operation of computer 18 and manipulator 38, a short description is given here to aid in comprehension of the program listing.

When computer 18 is turned on, the DOS 3.0 operating system is read from a disk inserted in the disk drive included in computer 18. After the operating system has been loaded, computer 18 then reads and executes the commands listed in the file AUTOEXEC.BAT. This command list includes the Prokey commands to configure the keyboard of computer 18 for use with manipulator 38, commands to set up and start speech recognition and synthesis circuit 192, and commands to prompt the operator for keyboard input.

59

The last command of the file executes the manipulator control software.

The manipulator control software consists of an initialization section and a control loop. The initialization section sets various operating constants and flags. When the control loop is first entered, the operator is instructed to position the patient appropriately using the keyboard to adjust the position of the manipulator, and to enter the patient's leg length. The software uses the known position of the manipulator determined from the manipulator's position sensors and the patient data to establish the safe range of motion of the manipulator for the particular patient. The pre-programmed patient positions ("elevate", "full extension", "full flex", "half flex", and "figure 4") are then scaled by the software to adapt them to the limb size of the particular patient.

When the position scaling is complete, an additional (optional) automatic variation of operating parameters is performed. At the request of the operator, the patient's limb is moved into the "figure 4" position. The compliance of the wrist actuators is then incrementally increased, until the wrist actuators are no longer able to maintain the desired position of the patient's limb against the gravitational force exerted by the weight of the patient's limb. The wrist actuator's compliance is then incrementally decreased until the wrist actuators are just

60

able to maintain the desired limb position.  This degree of compliance is established as appropriate for the particular patient.  In subsequent use with that patient, the manipulator wrist actuators are never inflated beyond the degree of compliance established as appropriate for the patient, thereby preventing exertion of unnecessarily excessive forces on the patient's limbs.

The control loop consists of three sections which are repeatedly called in sucession.  The first section checks for input from the operator via the keyboard, voice recognizer or hand controller, and if any such input is recognized, calls subroutines to alter the operation of the manipulator appropriately.  The second section reads all sensors associated with the manipulator, including position sensors and settings of hand controls.  The third section of the control loop interprets the sensor readings and the desired mode of operation as indicated by the operator input to alter the operation of the electromechanical components of manipulator 38 appropriately.

Many of the operations that are executed as a result of operator input are contained in subroutines within the control software.  These include such functions as the recall of programmed positions, movement along paths in "world" or "patient" coordinates, and interactive alteration of control parameters, or display of control parameters.  ("World" and "patient" coordinates refer to two coordinate reference systems commonly used in robotic terminology. "World" coordinates are often used to define the spatial position of one robot segment relative to another segment of the same robot; whereas "patient" coordinates equate to the more conventional "tool" coordinates commonly used to define the spatial position of one portion of the patient's body - the "tool" in this application - relative to another segment of the patient's body.) There are also subroutines that are called from various places in the control software. These include routines to test for movement of manipulator 38 beyond positions determined as safe for a particular patient, to stop and maintain a given position of manipulator 38, and to perform other functions.

### V. Alternative Embodiments

Many alterations and adaptations may be made to the preferred embodiment described herein. Accordingly, the invention is to be limited only by reference to the appended claims. For example, although all seven degrees of freedom of the preferred embodiment are either pneumatically or hydraulically powered, another embodiment may have no powering elements, instead offering only braking mechanisms at each joint of the kinematic structure. Such a simplified alternate manipulator may be a desirable low cost version capable of providing some of the basic functions required. This alternate embodiment would use the same geometry as the preferred embodiment, having seven degrees of freedom, four in the arm and three at the wrist, and would be able to manouever within the same workspace. In operation, the surgeon would grasp the arm, use an operator hand controller or voice recognizer to release the brakes, and manually move the arm to the new desired position and orientation. When the new position and orientation is reached, the operator could again use the operator hand control or voice recognizer to cause the brakes to be activated to maintain the set position and orientation.

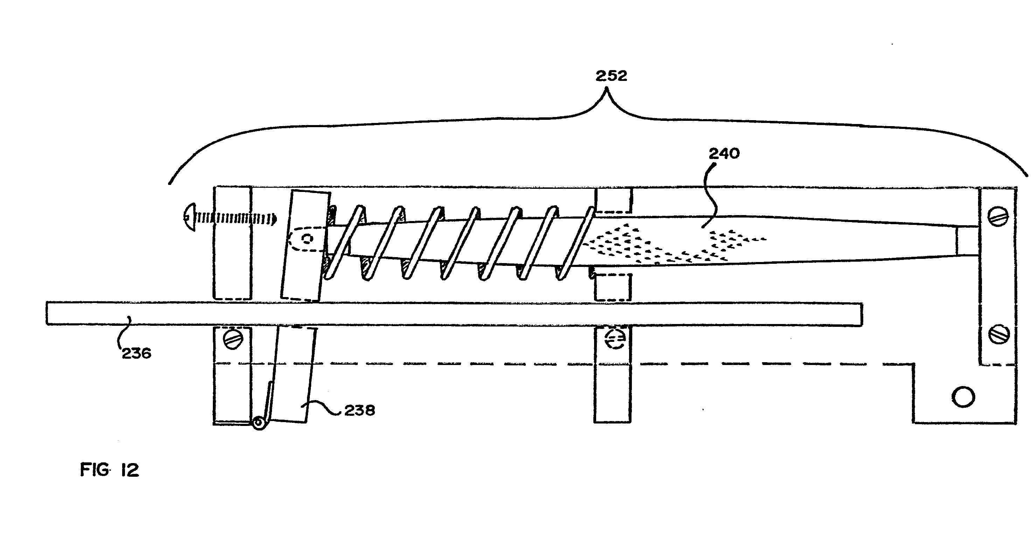

Such an alternate embodiment could be constructed by replacing each of the hydraulic and pneumatic actuators used in the preferred embodiment with a braking mechanism which could consist of a pneumatic or other actuator activating a friction or ratchet mechanism, or a device similar to that shown in Figure 12. This braking mechanism consists of push rod 236 and locking plate 238, which is activated by pneumatic actuator 240, which may be a Bridgestone Rubberturator™. When the mechanism is activated, push rod 236 is firmly locked against longitudinal displacement. The braking mechanism could be realized using servo valves similar to those used in the preferred embodiment. In the alternate embodiment, each hydraulic actuator could be directly replaced with such a braking mechanism, while each pair of actuators in the wrist of the preferred embodiment would be replaced with a braking mechanism and a loop of chain wrapped around an idler sprocket, in similar manner to readily availible

63

"rodless cylinder" actuators.

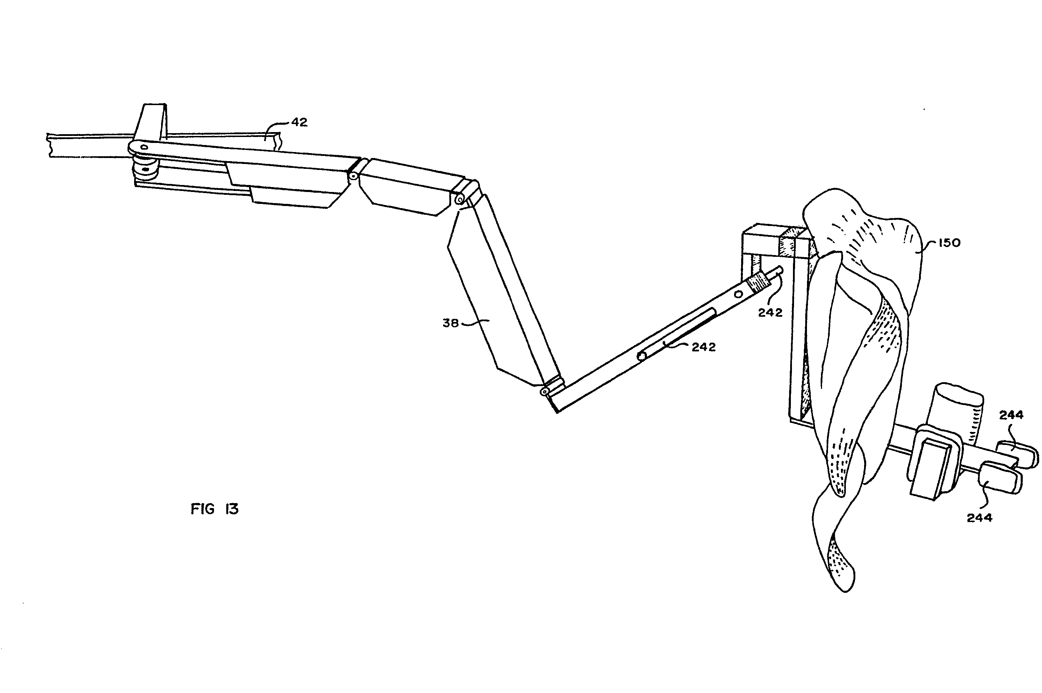

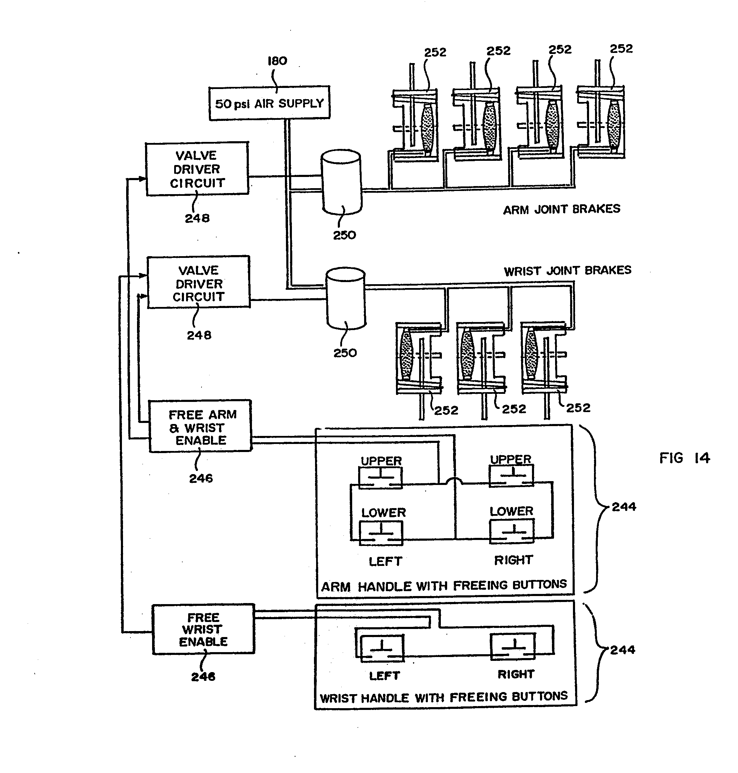

The method by which the operator activates and de-activates the braking mechanism incorporated in an alternate embodiment of the manipulator may be the same as that used with the preferred embodiment, or it may be a simpler method. A suitable alternatative method for controlling the operation of the simplified manipulator shown in Figure 13 could utilize one or more handles attached to the simplified manipulator which, when grasped and squeezed, would release some or all of the manipulator joints while handle(s) 242 are squeezed (each joint corresponding to one of axles 60, 72, 86, 100 or differential 112 described above; the actuators shown in Figure 14 corresponding to those described above with reference to the computer controlled continuously variable movement of the aforementioned axles and differential). Handle(s) 242 would allow the operator to position the manipulator arm and orient the patient applied part while the manipulator joints are unlocked and then release the handle(s) 242 to relock the manipulator joints in the desired position. Selective joint positioning (not shown in detail) would allow separate control of the limb position and orientation. Figure 14 is a schematic diagram which shows the configuration of the actuators comprising a simplified operator interface that may be used with the alternative embodiment of Figure 13 to enable the operator to selectivly lock and unlock the manipulator wrist actuators in order to facilitate movement of the manipulator wrist mechanism, or to enable the operator to selectively lock and unlock the remaining actuators in order to facilitate movement of the rest of the manipulator arm mechanism. The mechanism for activating the pneumatic actutators incorporates solenoid controlled pneumatic valves to either fully inflate of fully deflate the actuators, rather than servo valves which can adjust the actuator pressure to any intermediate state. Switch 244 on handles 242 activate joint freeing circuitry 246 which activate valve drivers 248 which in turn cause air valves 250 to open and release the pressure in the pneumatic actuators 252.

An extension to the alternate embodiment of Figure 13 could include springs to compensate for the weight of the manipulator arm mechanism, and possibly the weight of the patient's limb, to allow easier manipulation by the surgeon. A further alternate embodiment may comprise a lightweight tubular structure with rotating braked joints having a similar kinematic structure to the preferred embodiment hereinbefore described. Pneumatic actuators such as the Bridgestone Rubberturator[TM] could power rotary brakes rather than the linear brake shown in Figure 12, to lock the joints. Each actuator could power two adjacent joints to minimize the manipulator arm weight.

In addition, the method of actuation of the manipulator could be altered by replacing one or more of the hydraulic or pneumatic actuators and associated control circuitry with other actuators and controllers such as the Atchley[TM] pneumatic servo actuators (Atchley Controls Inc., Los Angeles California), or other hydraulic, pneumatic or electric actuators and controllers. The kinematic manipulator structure could also be modified for use in diagnostic and therapeutic arthroscopies on limbs and joints other than legs and knees. The manipulator may be further modified for use in surgical procedures other than arthroscopies, including the repair of fractures and reconstructive surgery.

The manipulator could also be modified to facilitate manipulation and positioning of all parts of the body simultaneously, thus serving as a completely automated, reprogrammable, multifunctional manipulator to replace the conventional operating-room table. The manipulator may also be modified for use as a reprogrammable continuous motion device for repetitivly and optimally manipulating a joint post-surgically to achieve improved joint mobility in certain patients. As a still further alternate embodiment, the manipulator could also be modified for use as a reprogrammable multifunction exerciser or stress-tester which could vary its operation in response to changes in the patient's response to exercise or physical stress.

Many of the advanced medical robotic aspects of the operation of the perferred manipulator described herein could be readily adapted to a wide array of medical robots for (1) improving the quality of diagnosis and treatment, (2) controlling and reducing the costs of such diagnosis and treatment, and (3) reducing the exposure of patients and clinical staff to hazardous environments. For example, a modified version of the manipulator could be used to automatically manipulate a patient's limb so that the limb could be accurately x-rayed immediately following surgery, with the clinical staff remote and protected from exposure to ionizing radiation. The manipulator could also be modified for use as a tissue retractor in surgery, for example, to create the best possible visualization of a surgical site, while minimally damaging the retracted tissue by sensing and controlling the tissue pressure at the retraction site. The same aspect of the invention could be modified to incorporate a laser effector, wherein the control of the actuation of the laser effector and the manipulation of that effector is responsive to changes in a physiologic or morphologic parameter of the patient; for example, the color, temperature or surface topology of the tissue being subjected to laser irradiation might change the power, duration, and wavelength of the laser effector and might change the direction and speed of manipulation of the laser effector.

If a patient parameter such as an electrocardiographic signal, an electromyographic signal, or an electroencephalographic signal were sensed, then a

.modified form of the instant invention could be used to automatically position a diagnostic or therapeutic electrode near structures associated with the heart, nerve, muscle, or brain, with great precision and with minimal damage to adjacent structures. Another potentially useful modification of the invention involves morphological parameters of the patient derived from an imaging system, such as an x-ray machine, a nuclear magnetic resonance scanner, a positron-emission scanner, or a fluoroscopy machine. By sensing and analyzing such parameters an advanced medical robot could automatically insert an effector such as radioactive source or the tip of a fiber-optic instrument into the body very precisely and with a low risk of unnecessary injury to the patient. Other modified forms of the invention could be used as novel micro-manipulators to manipulate small anatomical structures, or small instruments accurately in relation to tissue, organs or other structures, in a sensor-driven mode where changes in the patient's parameters would normally direct the operation of the manipulator; such that the operator would intervene only in abnormal circumstances which could not be adequately or safely handled by the manipulator.

Other aspects of the instant invention could be modified for significant medical benefit. For example, the "eyes free" control system employed in one mode of operation of the manipulator could be used separately in operating rooms and other medical situations for hands and eyes free control of many medical devices; for example, the system could be used to replace or alternately actuate the switches and controls on many medical devices. In the operating room, this would permit such devices to be efficiently and accurately controlled by the surgeon without the distraction and interruption now often associated with actuation of such controls and switches by hand or foot. Also, the voice recognintion system herein described could be used by anesthesiologists, nurses and others who might routinely wear masks similar to the mask described above to reliably record data related to the patient and a diagnostic or medical procedure in a hands-free mode in such environments. Widespread use of such a speech-recognition system might thus help to reduce the labor-intensivness and improve the quality of many therapeutic and diagnostic procedures.

## VI. Software Source Code Listing

```
:

:

:AUTOEXEC.BAT

:

:

echo off
assign b=a
prokey  autoarth.pro/r
cls
echo              Copyright  1986  Andronic Devices Ltd.
echo      ARTHROBOT  and  ANDRONIC  are trade marks of Andronic Devices Ltd.
echo
echo            Welcome to ARTHROBOT II,  the Surgical Robot
echo      To load the Voice Patterns and run the Arthrobot Control Program
echo
echo          Strike  Alt 1  for Dr. Day's speech patterns
echo          Strike  Alt 2  for Rob's    speech patterns
echo          Strike  Alt 3  for Glenn's  speech patterns
echo          Strike  Alt 4  for Geof's   speech patterns
echo          Strike  Alt 5  for Others   speech patterns
echo

:

:   AUTOARTH.PRO

:


<alt-><alt1>
copy  a:brian.spb  a:arthroII.spb<enter>
vpl   a:arthroII  40 40 40 0 30 1 0 0 8 1<enter>
arthroII<enter>
<alt->
*


<alt-><alt2>
```

```
copy   a:rob.spb   a:arthroII.spb<enter>
vpl    a:arthroII   40 40 40 0 30 1 0 0 8 1<enter>
arthroII<enter>
<alt->
*


<alt=><alt3>
copy   a:glenn.spb   a:arthroII.spb<enter>
vpl    a:arthroII   40 40 40 0 30 1 0 0 8 1<enter>
arthroII<enter>
<alt->
*


<alt=><alt4>
copy   a:geof.spb   a:arthroII.spb<enter>
vpl    a:arthroII   40 40 40 0 30 1 0 0 8 1<enter>
arthroII<enter>
<alt->
*


<alt=><alt5>
copy   a:other.spb   a:arthroII.spb<enter>
vpl    a:arthroII   40 40 40 0 30 1 0 0 8 1<enter>
arthroII<enter>
<alt->
*
:
:  CONFIG.SYS
:
break=on
files=20
buffers=10
fcbs=32,32
device=ansi.sys
device=vpu.sys
/******************
   DEFINES.H                        860202
  ******************/
```

```
/*

        All the constants used by the ARTHROII program
*/



#define     abs(x)          (((x) < 0)  ?  -(x)  :  (x))
#define     max(x,y)        (((x) < (y))  ?  (y)  :  (x))
#define     min(x,y)        (((x) < (y))  ?  (x)  :  (y))


#define     TRUE            1
#define     FALSE           0


#define     YES             1
#define     NO              0



#define     RAD_PER_POT0     0.001150000
#define     RAD_PER_POT1     0.001237822
#define     RAD_PER_POT2     0.001165279
#define     RAD_PER_POT3     0.001066393
#define     RAD_PER_POT4     0.001150000
#define     RAD_PER_POT5    -0.000994175
#define     RAD_PER_POT6     0.001150000
#define     SHOULDER         2088      /* was 2066 */
#define     HYDRO            0
#define     BRIDGES          1
#define     LOCK            'e'
#define     HOLD            'l'
#define     MOVE            'm'
#define     FREE            'f'
#define     MUSH            'z'
#define     TRACK           'k'
#define     FORWARD         0x0492
#define     REVERSE         0x0249
#define     OFF              0
#define     OPEN            0xFFFF
#define     SHUT             0
#define     GRIP_DEPTH       165.0
#define     WIDTH            220.0
```

0201883

```
#define    DEPTH           165.0
#define    PI              3.141592654
#define    ANG_STEP        2.0


#define    WDON            0x1000
#define    TIMERS          4
#define    POTERR          1
#define    WDOFF           0xEFFF


#define    USER_SUPPLIED_CLOCK_ROUTINE_VECTOR      0x1c


/*
    The following is a list of the characters that will be placed in the
    keyboard buffer for the appropriate command.  Remember that the source
    of these characters can be the keyboard, voice, or the hand control
unit.
    NOTE:  The lower byte contains the ASCII character of the key
           and the upper byte contains the scan code for the key.
*/



#define    F1                  59 * 256
#define    F2                  60 * 256
#define    F3                  61 * 256
#define    F4                  62 * 256
#define    F5                  63 * 256
#define    F6                  64 * 256
#define    F7                  65 * 256
#define    F8                  66 * 256
#define    F9                  67 * 256
#define    F10                 68 * 256


#define    ALT_A               30 * 256
#define    ALT_B               48 * 256
#define    ALT_C               46 * 256
#define    ALT_D               32 * 256
#define    ALT_E               18 * 256
```

```
#define    ALT_F              33 * 256
#define    ALT_G              34 * 256
#define    ALT_H              35 * 256
#define    ALT_I              23 * 256
#define    ALT_J              36 * 256
#define    ALT_K              37 * 256
#define    ALT_L              38 * 256
#define    ALT_M              50 * 256
#define    ALT_N              49 * 256
#define    ALT_O              24 * 256
#define    ALT_P              25 * 256
#define    ALT_Q              16 * 256
#define    ALT_R              19 * 256
#define    ALT_S              31 * 256
#define    ALT_T              20 * 256
#define    ALT_U              22 * 256
#define    ALT_V              47 * 256
#define    ALT_W              17 * 256
#define    ALT_X              45 * 256
#define    ALT_Y              21 * 256
#define    ALT_Z              44 * 256


#define    CURSOR_UP          72 * 256
#define    CURSOR_DOWN        80 * 256
#define    CURSOR_LEFT        75 * 256
#define    CURSOR_RIGHT       77 * 256




#define    VOICE              'v'
#define    HAND_CONTROL       'p'
```

```
#define    ARTHROBOT_COMMAND           'a'
#define    OKAY_COMMAND                'k'
#define    NO_COMMAND                  'n'
#define    QUIET_COMMAND               'q'
#define    EMERGENCY_STOP_COMMAND      '*'           /*   Will also be ' '   */
#define    RESERVED_6_COMMAND


#define    ELEVATE_COMMAND             F2
#define    FULL_EXTENSION_COMMAND      F4
#define    HALF_FLEX_COMMAND           F6
#define    FULL_FLEX_COMMAND           F8
#define    FIGURE_4_COMMAND            F10
#define    SETUP_COMMAND               '2'
#define    RESERVED_13_COMMAND
#define    RESERVED_14_COMMAND


#define    ARM_COMMAND                 F1
#define    HIP_COMMAND                 F3
#define    KNEE_COMMAND                F5
#define    FOOT_COMMAND                F7
#define    WRIST_COMMAND               F9
#define    COMPLIANCE_COMMAND          '1'
#define    RESERVED_21_COMMAND
#define    RESERVED_22_COMMAND


#define    MOVE_COMMAND                'g'
#define    STOP_COMMAND                's'
#define    IN_COMMAND                  'i'
#define    OUT_COMMAND                 'o'
#define    FLEX_COMMAND                'f'
#define    EXTEND_COMMAND              'e'
#define    RELEASE_COMMAND             'r'
#define    HOLD_COMMAND                'h'
#define    LESS_COMMAND                'l'
#define    MORE_COMMAND                'm'
```

```
#define    RESERVED_33_COMMAND
#define    RESERVED_34_COMMAND
#define    RESERVED_35_COMMAND
#define    RESERVED_36_COMMAND


#define    MINUS_COMMAND              '-'
#define    PLUS_COMMAND               '+'


/*
        The voice output word numbers for each command
*/


#define    ARTHROBOT_RESPONSE          1
#define    OKAY_RESPONSE               2
#define    NO_RESPONSE                 3
#define    QUIET_RESPONSE              4
#define    EMERGENCY_STOP_RESPONSE     5
#define    RESERVED_6_RESPONSE         6


#define    ELEVATE_RESPONSE            7
#define    FULL_EXTENSION_RESPONSE     8
#define    HALF_FLEX_RESPONSE          9
#define    FULL_FLEX_RESPONSE         10
#define    FIGURE_4_RESPONSE         11
#define    SETUP_RESPONSE            12
#define    RESERVED_13_RESPONSE     13
#define    RESERVED_14_RESPONSE     14

#define    ARM_RESPONSE             15
#define    HIP_RESPONSE             16
#define    KNEE_RESPONSE            17
#define    FOOT_RESPONSE            18
#define    WRIST_RESPONSE           19
#define    COMPLIANCE_RESPONSE      20
```

```
#define    RESERVED_21_RESPONSE     21
#define    RESERVED_22_RESPONSE     22


#define    MOVE_RESPONSE            23
#define    STOP_RESPONSE            24
#define    IN_RESPONSE              25
#define    OUT_RESPONSE             26
#define    FLEX_RESPONSE            27
#define    EXTEND_RESPONSE          28
#define    RELEASE_RESPONSE         29
#define    HOLD_RESPONSE            30
#define    LESS_RESPONSE            31
#define    MORE_RESPONSE            32
#define    RESERVED_33_RESPONSE     33
#define    RESERVED_34_RESPONSE     34
#define    RESERVED_35_RESPONSE     35
#define    RESERVED_36_RESPONSE     36



#define    LIMIT                    37



#define    MINUS_RESPONSE           ?         /* Depends on the selection */
#define    PLUS_RESPONSE            ?         /*    "      "   "   "    "    */
/*******************
   EXTRNVAR.H                    860202
*******************/


/*
    Declarations of all the external variables used by the ARTHROII program.
*/



extern   int     mode;
extern   int     are_we_in_continuous_motion;
extern   int     cont;
extern   int     PERIOD;
extern   int     side;
```

```
extern  int     shoulder;                                       0201883
extern  int     pots[ 7 ];
extern  int     joint;
extern  int     memory[ 6 ][ 7 ];
extern  int     attention;
extern  int     autocomp;
extern  int     pend_on;
extern  int     outbits;
extern  int     ref_dac;
extern  int     penpressed;
extern  int     pendsample;
extern  int     timer[ 4 ];
extern  int     fault[ 7 ];
extern  int     dac[ 6 ];
extern  int     debug;
extern  int     debugger[ 64 ];
extern  int     realpos[ 7 ];
extern  int     pathon ;
extern  int     prepropos, onpath;
extern  int     recalling;
extern  int     safety;
extern  int     slowmode;
extern  int     trained;
extern  int     cycler, cycle_position, ctime;
extern  int     external_rotation;
extern  int     wrparms[ 20 ];              /* wrist params, for wrist ctrl */
extern  int     wrcompmap[ 9 ];             /* wrist compliance map */

extern  double  leg,      shiftx,   shifty,   z0:        /* patient scaling
data */
extern  double  hx,       hy,       kx,       ky,  minq; /* hip and knee
coordinates */
extern  double  offset1, offset2, offset3, offset5 ;
extern  double  elevate, flex_ang ;                      /* preprogrammed
positions */
extern  double  elev,     flex ;                         /* anatomical
motion */
extern  double  yaw_center;
extern  double  pitch_center;
```

```
extern  double   roll_center;



extern  struct  sset     set[ 7 ];

extern  struct  sstate   state[ 7 ];

extern  struct  sparms   parms[ 7 ];

     .

extern  struct  spump    pump[ 4 ];




/* definition of math function returns

*/

extern  double   sqrt(), sin(), cos(), tan(), asin(), acos(), atan();
/*******************

   GLOBVARS.H                             860202

 ******************/


/*

     All the global variables used by the ARTHROII program

*/



int     cont;                        /* program on switch */

int     PERIOD    = 5;

int     side      = 1;               /* polarity factor accounting for side of

table */

int     shoulder  = 1;               /* polarity factor accouting for actual

shoulder pos*/

int     pots[7];                     /* current pot readings */

int     joint;                       /* used as a joint counter global variabl

*/

int     memory[6][7]={{9999}, {9999}, {9999}, {9999}, {9999}, {9999}};  /*

pos. memory */

int     attention;

int     autocomp  = FALSE;              /* automatic wrist compliance

scaling */

int     pend_on   = TRUE;                     /* pendant enable flag */
```

```
int     outbits    = 0;
int     ref_dac    = FALSE;                /* Flag to enable background
dac refresh */
int     penpressed = FALSE;    .           /* Pendant knob & buttons used
indicator */
int     pendsample;                        /* ADC reading from pendant */
int     timer[4]   = {0,0,0,0};            /* Set of duty cycle timers */
int     fault[7]   = {0,0,0,0,0,0,0};      /* Error flags for each joint
*/
/*      dac[6]     = {128,128,128,128,128,128};   refresh values for BRST
actuators */
int     dac[6]     = {0,0,0,0,0,0};     /*  refresh values for BRST actuators
*/
int     debug;                             /* on the fly debugger flag */
int     debugger[64];                      /* array of variables for
debugger*/
int     realpos[7];                        /* temporary position storage
*/
int     pathon = 0 ;                       /* toggle flag for path
routine */
int     prepropos, onpath = 0;             /* for path routines */
int     recalling  = 0;
int     safety     = FALSE ;
int     slowmode   = 0 ;
int     trained    = 0;                     /* flag to show that positions
are trained*/
int     cycler, cycle_position, ctime;     /* variables for automatic
cycling */
int     external_rotation;                 /* flag to show external
rotation called*/
int     wrparms[20];
int     wrcompmap[9] = {8,96,64,48,32,24,16,12,8}; /* wrist compliance map */


double  leg,     shiftx,   shifty,    z0;         /* patient scaling data */
double  hx,      hy,       kx,        ky,   minq; /* hip and knee coordinates
*/
double  offset1, offset2, offset3, offset5 ;
double  elevate, flex_ang ;                       /* preprogrammed positions
*/
```

80

```
double    elev,      flex ;                          /* anatomical m020188

double    yaw_center   = 1906 ;

double    pitch_center = 1876 ;

double    roll_center  = 2083 ;              .


struct    sset     set[ 7 ];

struct    sstate   state[ 7 ];

struct    sparms   parms[ 7 ];


struct    spump    pump[ 4 ] = {    {  0,   0x0003,   OFF,   1.0   },

                                    {  0,   0x0018,   OFF,   1.0   },

                                    {  0,   0x00C0,   OFF,   1.0   },

                                    {  0,   0x0600,   OFF,   1.0   }

                                };


/* definition of math function returns

*/

double    sqrt(), sin(), cos(), tan(), asin(), acos(), atan();


int       mode       =  0;

int       are_we_in_continuous_motion  =  NO;

/*        standard i/o header file for c86

*/


#ifndef      _stdioh

#ifdef _C86_BIG

#define NULL (0L)

#else

#define NULL 0

#endif


#define EOF (-1)   /* standard end of file */

#define EOS '\0'   /* standard end of string */

#define AREAD 0            /* ascii read */

#define AWRITE 1   /* ascii write */
```

81

```
#define AUPDATE 2 /* ascii update (take care with this one) */   0201883
#define BREAD 4          /* binary update */
#define BWRITE 5  /* binary write */
#define BUPDATE 6 /* binary update */


#define SEEK_CUR 1        /* seek rel to current */
#define SEEK_END 2        /* seek rel to end */
#define SEEK_SET 0        /* seek absolute */


/*    This is the max no. of files that the run-time allows
      you to have open at one time. The operating system
      also imposes additional constraints.
*/
#define SYS_OPEN  32


typedef char FILE;


#define getchar() fgetc(stdin)
#define getc(x) fgetc(x)
#define putchar(x) fputc(x,stdout)
#define putc(x,y) fputc(x,y)
#define ungetch(c) ungetc(c,stdin)


extern FILE *stdin, *stdout, *stderr;


extern FILE *fopen(), *freopen();
extern long ltell(), lseek(), ftell(), fseek();
extern char *gets(), *fgets();


#endif


#define _stdioh


/*    end of standard header file
*/
/******************
   STRUCTS.H                      860123
 *****************/
```

```
/*
        All the structure templates used by the ARTHROII program
*/



struct    sset
  {
    int mode;              /* Joint mode (lock, hold, move, free) */
    int pos;               /* Desired pot reading */
    int duty;              /* Duty cycle for speed control */
  };




struct    sparms
  {
    int    type;           /* Type of actuator (HYDRO, BRIDGES) */
    int    f_hys;          /* Forward hysteresis (used only for hydro) */
    int    r_hys;          /* Reverse hysteresis (used only for hydro) */
    int    clock;          /* Pointer to duty cycle timer */
    int    outadr;         /* Pump number for hydro, DAC address for atchleys.
*/
    int    vmask;          /* Valve mask */
    int    reset;          /* Mask to reset motor and valve to off and closed */
    int    xgain;          /* Position error gain */
    int    vgain;          /* Velocity error gain (used only for atchleys) */
    int    dead;           /* Length of time to wait after attaining position */
    int    inc;            /* Increment factor for each joint */
    int    msh;            /* mush mode constant, wrist only  */
  };




struct    spump
  {
    int    serve;          /* Bits determine which motors are being served */
    int    mask;           /* Bit mask for pump motor */
```

```
    int      motion;         /* Current state of the motor (FORWARD, REVERSE,
OFF) */
    double   flow;           /* Flow rate compensation factor */
  };




struct   sstate
  {
    int   pot;               /* Actual pot reading */
    int   deltax;            /* Differential pot reading w.r.t. time */
    int   motor;             /* Motor state */
                             /* For hydro, valid states are FORWARD, REVERSE, OFF
*/
                             /* For atchleys, motor contains DAC output */
    int   valve;             /* Valve state (OPEN, SHUT) */
                             /* For hydro, the valves lock the joint when closed.
*/
                             /* For atchleys, the valves supply air when open. */
    int   wait;              /* Timer to rest joint after reaching position */
  };
/*******************

   ACTONCOM.C                                     860206

   ******************/


/*

     This procedure reads the keyboard buffer, speaks the word, and
     commands the joints accordingly.
     Note that characters in the keyboard buffer may come from any one of
     three sources:  the keyboard, the SAR-10's Transparent Handler, or
     the operator's hand control.

*/




#include  "stdio.h"


#include  "defines.h"
```

```c
#include  "structs.h"                                       )201883
#include  "extrnvar.h"



act_on_commands_from_the_keyboard_buffer()
  {

    static  int  the_determined_selection,  the_determined_action,
               have_the_selection_and_action_been_verified;


    switch(  mode  )
      {

        case  0 :

/*
    Mode 0 is the normal operating mode for the Arthrobot.
    Commands entered via the keyboard or the SAR-10 speech recognizer are
    deposited into the keyboard buffer by interrupt driven procedures in DOS
    and the SAR-10 Transparent Handler software, respectively.
    The hand control is not interrupt driven so we'll have to read the
    hand control & determine if it has any commands to dump into the
    keyboard buffer.
*/

        if(  pend_on  )
          check_to_see_if_the_hand_control_has_any_commands_for_us(
the_determined_selection,

the_determined_action       );

/*
    Now read the keyboard buffer, speak the command word, and set the
    determined selection & the determined action appropriately.
*/

          make_a_decision_on_the_determined_selection_and_action(
```

```
                        &the_determined_selection,

                        &the_determined_action,

                        &have_the_selection_and_action_been_verified

);



/*

   ·Finally, initiate the selection and action

*/


        initiate_the_determined_selection_and_action(

                        the_determined_selection,

                        the_determined_action,

                        &have_the_selection_and_action_been_verified

);

        break;



     case  1 :


/*

   The Arthrobot runs in Mode 1 when doing incremental joint control.

*/


        if(  key_scan() ==  (-1)   )

           return( -1 );


        increment(  key(),   &mode  );
        break;



     case  2 :


/*

   The Arthrobot runs in Mode 2 when storing individual preprogrammed
positions.

*/
```

```
        if( key_scan() ==  (-1)   )
          return( -1 );

        store( key(),     smode );
        break;


    }


  }
/*******************
   ADJCOMP.C                              860124
******************/

/*
    The wrist compliance or firmness can be set to 8 levels, 1 being the
    softest and 8 being the hardest. This routine accepts integer values
    1 to 8 and sets the compliance accordingly. It also accepts the char-
    acters '-' and '+' to decrement or increment the compliance by 1 step.
*/



#include  "stdio.h"

#include  "defines.h"
#include  "structs.h"
#include  "extrnvar.h"

adjust_the_wrist_compliance( i )
int i;
{

if ( i == '-' ) wrcompmap[ 0 ]--;
  else if ( i == '+' ) wrcompmap[ 0 ]++;
    else if ( i > 0 && i < 9 ) wrcompmap[ 0 ] = i;

if ( wrcompmap[0] < 1 ) wrcompmap[0] = 1;    /* trim compliance scale 1 - 8
*/
```

```
else if ( wrcompmap[0] > 3 ) wrcompmap[0] = 3;


pokeb( 308, 0xb000, '0' + wrcompmap[0] );


wrparms[13] = wrcompmap[ wrcompmap[0] ];
wrparms[14] = 4095 / wrparms[13];



}
/*******************
     ADJUST.C                    360123
******************/


/*
     This routine allows the user to adjust the joint parameters.
*/



#include   "stdio.h"

#include   "defines.h"
#include   "structs.h"
#include   "extrnvar.h"



adjust()
  {


    int i, reply;
    double num1, num2;


        lockup(TRUE);
        clearscreen (10,24);
        movecursor (12,0);
        printf ("Alter which joint? (0-6) ");
        scanf ("%d\n", &i);
```

```
        if (  i >= 0      &&       )

          {


              printf ("\n\nF_hys (wrist: window), now %d : ", parms[i].f_hys);
              scanf ("%d\n", &parms[i].f_hys);
```

/*  .    Since yaw and pitch require same constants, updating yaw constant */
/*       will automatically update corresponding pitch constant           */

```
              if ( i == 4 ) parms[5].f_hys = parms[4].f_hys;


              printf ("R_hys (wrist: delay) , now %d : ", parms[i].r_hys);
              scanf ("%d\n", &parms[i].r_hys);
              if ( i == 4 ) parms[5].r_hys = parms[4].r_hys;


              printf ("Xgain, now %d : ", parms[i].xgain);
              scanf ("%d\n", &parms[i].xgain);
              if ( i == 4 ) parms[5].xgain = parms[4].xgain;


              printf ("Vgain, now %d : ", parms[i].vgain);
              scanf ("%d\n", &parms[i].vgain);
              if ( i == 4 ) parms[5].vgain = parms[4].vgain;


              printf ("Wait time, now %d : ", parms[i].dead);
              scanf ("%d\n", &parms[i].dead);
              if ( i == 4 ) parms[5].dead = parms[4].dead;


              printf ("Mush mode constant, now %d : ", parms[i].msh);
              scanf ("%d\n", &parms[i].msh);
          }


        clearscreen (10,24);
        lockup(FALSE);

    }


adjustwr()        /* adjust wrist parameters  */


    {
```

```
        lockup(TRUE);

        clearscreen (10,24);

        movecursor (12,0);

        printf ("Adjust wrist parameters\n\n");
printf("Wrist mode: 1 = normal  2 = lock YAW & PITCH  3 = pitch
tracking\n\n");


        printf ("Wrist mode, now %d : ", wrparms[0]);

        scanf ("%d\n", &wrparms[0]);


/*      printf ("V offset, now %d : ", wrparms[1]);

        scanf ("%d\n", &wrparms[1]);


        printf ("Pitch Window, now %d : ", wrparms[2]);

        scanf ("%d\n", &wrparms[2]);


        printf ("Overshoot constant, now %d : ", wrparms[3]);

        scanf ("%d\n", &wrparms[3]);


        printf ("Pitch velocity scaling factor, now %d : ", wrparms[4]);

        scanf ("%d\n", &wrparms[4]);


        printf ("Count down timer, now %d : ", wrparms[5]);

        scanf ("%d\n", &wrparms[5]);

*/






        /*  ELEVATE OFFSET PARAMETERS */
/*      printf ("Yaw offset const for elevate, now %d : ", wrparms[10]);

        scanf ("%d\n", &wrparms[10]);


        printf ("Pitch elevate offset, now %d : ", wrparms[11]);

        scanf ("%d\n", &wrparms[11]);
```

J201883

```
        printf ("Roll extend/elevate offset. now d :    rparms[12]);
        scanf ("%d\n", &wrparms[12]);
*/

        /*  COMPLIANCE SCALING FACTORS    */

        printf ("COMPLIANCE SCALING (3:hard - 14:soft), now %d : ",
wrparms[13]);
    .   scanf ("%d\n", &wrparms[13]);
        wrparms[14] = 4095 / wrparms[13];
        printf("WRIST COMPLIMENTARY CONSTANT = %d\n", wrparms[14] );


        /* parameters for pitch tracking B      */

        printf ("Pitch tracking offset constant, now %d : ", wrparms[15]);
        scanf ("%d\n", &wrparms[15]);


        printf ("Roll tracking offset constant, now %d : ", wrparms[16]);
        scanf ("%d\n", &wrparms[16]);


        clearscreen (10,24);
        lockup(FALSE);


    }
/********************

  ANATOM.C                                       851203
 *****************/


/*
        This routine does the anatomical leg motion calculation coordination.
        It calls routines REVERSE.C and FORWARD.C and accepts as parameters an
        incremental hip ang and flex ang.
*/


#include    "stdio.h"

#include    "defines.h"
#include    "structs.h"
#include    "extrnvar.h"
```

```
anatom(de,df)
  double de,df ;


  {


    double ang0, ang1, ang2, ang3, ang4, ang5, ang6 ;
    double elv,flx ;



  if ( trained  )
    {
    if ((set[1].mode != MOVE) && (set[2].mode != MOVE) && (set[3].mode !=
MOVE))
      {
    lockup(TRUE);
    ang1 = pots[1] * RAD_PER_POT1 + offset1;   /* calc arm coordinates */
    ang2 = pots[2] * RAD_PER_POT2 + offset2;   /* calc arm coordinates */
    ang3 = pots[3] * RAD_PER_POT3 + offset3;
    ang5 = pots[5] * RAD_PER_POT5 + offset5;   /* calc pitch attitude  */
    ang0 = ang4 = ang6 = 0.0 ;
    for_leg(&elv,&flx,ang0,ang1,ang2,ang3,ang4,ang5,ang6);
    elev= (elv / PI) * 180.0 ;
    flex = (flx / PI) * 180.0 ;
    elev += de ;
    flex += df ;
    elv = (elev/ 180.0) * PI ;
    flx = (flex / 180.0) * PI ;
    rev_leg(&ang0,&ang1,&ang2,&ang3,&ang4,&ang5,&ang6,elv,flx);
    set[1].pos = (ang1 - offset1) / RAD_PER_POT1;
    set[2].pos = (ang2 - offset2) / RAD_PER_POT2;
    set[3].pos = (ang3 - offset3) / RAD_PER_POT3;
    set[1].mode = MOVE ;
    set[2].mode = MOVE ;
    set[3].mode = MOVE ;
    /* pitch pot sets itself after move */
    lockup(FALSE);
```

```c
          }
     }
 else
    {
      clearscreen(22,24);
      movecursor(22,0);
      printf(" Anatomical motion is not available until patient is scaled.\n");
      printf("  \n");
      movecursor(24,0) ;
    }


}
/*******************
  AROUT.C                                        360122
 ******************/


/*
     A set of routines for communicating directly with the AR-10 for doing
     voice verification for other functions.  Main routine arout(x) will
cause
     speech synthesizer to say word x form its trained vocbaulary.
*/

#include "stdio.h"
#include "defines.h"
#include "structs.h"
#include "extrnvar.h"



arout( x  )
  int x;
  {

#define  SAR10_DATA_PORT     0x0350
#define  SAR10_STATUS_PORT   0x0351

     unsigned char  output_string[ 6 ];
     int     i;
```

```
        if (debug)
          printf ("arout:  word %d\n", x);


        if(    x < 1   ||  x > 33  )
          return( 0 );


      .
        if( inportb( SAR10_STATUS_PORT ) & 0x0004  )  /* Check if ready to
*/
          return( 0 );                               /* receive audio
output*/
                                                     /* command
*/


        strcpy( output_string,  "OA \r" );


        if( x > 9 )
            output_string[ 2 ] = (unsigned char) ( x / 10 + '0' );
        output_string[ 3 ]    = (unsigned char) ( x % 10 + '0' );


        for( i = 0; output_string[ i ] != 0; i++ )
          {
            while(      ( inportb( SAR10_STATUS_PORT ) & 0x0002 ) == 0x0002
                  && ( inportb( SAR10_STATUS_PORT ) & 0x0004 ) == 0x0004
);
            outportb( SAR10_DATA_PORT, output_string[ i ] );
          }


        return( x );


      }
/******************
   ARTHROII.C                     860202
 *****************/
                         /****************************/
                         /*                          */
                         /*  A R T H R O B O T  II  */
                         /*                          */
```

```
/*                  with            /
/*                                  /
/*          BRIDGESTONE             /
/*          .                       /
/********************************/
```

```
/* Arthrobot II Control Program */


#include  "stdio.h"

#include  "defines.h"
#include  "structs.h"
#include  "globvars.h"




main ()
  {
   int  i, j;


      clearscreen(  0,  24  );
      movecursor(  0,  32  );
      setup();                              /* Initialize system */


      mainmenu();



      cont = TRUE;
      while (cont)                          /* Main Loop */
        {
        do_ctrl_loop();
        if ( autocomp )
           automatic_wrist_compliance_scaling();
        }
```

```
      dtreset();

      clearscreen(  0,  24  );
      movecursor(   0,  0   );
      printf ( "Goodbye from ARTHROBOT II \n");


      unlink_from_interrupt(   USER_SUPPLIED_CLOCK_ROUTINE_VECTOR   );



}



do_ctrl_loop()


  {
  snapshot();
  process();                                /* Fake distributed processing
*/
  act_on_commands_from_the_keyboard_buffer();  /* Get control input and
adjust joints */
  outbits  &=  WDOFF;
  dwrport (  8, outbits  );
  getstatus();                              /* Read pots and get error
conditions */
  }
/********************
   AUTOCOMP.C                               860130
 *****************/


/*
    This routine will move the patients leg to a figure 4 position and
    determines the mimimum wrist stiffness to maintain a correct wrist
    position.
*/
```

```c
#include   "stdio.h"

#include   "defines.h"
#include   "structs.h"
#include   "extrnvar.h"


automatic_wrist_compliance_scaling()


{
     /* move to figure 4 position */
recall( '5' );          /* figure 4 is position 5 */
delay( 10000 );         /* wait for 10 seconds, till fig 4 is reached  */


while ( wrist_at_set_position() && wrcompmap[0] > 1 )
  {
  adjust_the_wrist_compliance( '-' );  /* decrement compliance by one step */
  delay( 1500 );
  }



printf(" ");  /* signify that wrist has reached too weak point */

while ( !wrist_at_set_position() && wrcompmap[0] < 9 )
  {
  adjust_the_wrist_compliance( '+' );  /* increment compliance by one step */
  delay( 1500 );
  }

autocomp = FALSE;

}    /* end of automatic_wrist_compliance_scaling()   */



/* this routine checks if the actual wrist position is the same as the
   desired wrist set position and returns a boolean response          */
```

```
wrist_at_set_position()


{
int  flag, joint;


flag = TRUE;


for, ( joint = 4; joint < 7; ++joint )
   if ( abs( set[ joint ].pos - state[ joint ].pot ) > 30 )
      {
      flag = FALSE;
      break;
      }
return( flag );
}




/* This routine delays by the passed number of milliseconds and continues
   to execute the main arthrobot control loop */


delay( msec )
unsigned int msec;


{
unsigned long   get_the_time_of_day();
unsigned long   base, max_ticks;


/* max_ticks = msec * 18.2 ticks/sec / 1000 msec/sec = ticks */
max_ticks = ( unsigned long) msec * 182L / 10000L;


base = get_the_time_of_day();


while ( get_the_time_of_day() - base < max_ticks )
   do_ctrl_loop();   /* run normal control loop */
}
```

0201883

```
/* scale_the_wrist is called immediately after scale and prompts the operator
   to verify wrist compliance scaling                                    */


scale_the_wrist()


{
int    ch;


lockup(TRUE);
clearscreen (10,24);
movecursor (12,0);
printf(" ");  /* beep for operator attention */
printf ("The arthrobot is about to scale the wrist compliance by \n");
printf ("moving the leg into the 'figure 4' position. \n\n");
printf ("If compliance scaling is desired and arthrobot path is clear, \n");
printf ("enter 'y': ");


while(  (  ch = key()  ) == (-1)   ) ;
clearscreen (10,24);
movecursor (12,0);
if ( ch == 'y' || ch == 'Y' )
  {
  hold_the_wrist();     /* lock wrist if not already locked */
  autocomp  =  TRUE;    /* set flag to do auto compliance scaling */
  printf("The compliance of the wrist is shown in the top right corner \n");
  printf("of the screen. Compliance ranges from 1 (very soft) to 8
(firm)\n\n");
  }
else
  {
  printf("Compliance not scaled.\n");
  autocomp = FALSE;
  }


lockup(FALSE);
}    /* scale_the_wrist */
/******************
```

    BRIDGES.C                              860130

```
***************/

/*

     This routine controls the Bridgestone rubber actuators
*/



#include   "stdio.h"

#define YAW    4
#define PITCH  5
#define ROLL   6


#include   "defines.h"
#include   "structs.h"
#include   "extrnvar.h"




bridges (joint)
int joint;


{
static int modeflag[7];  /* init to 'free' on first pass at startup */
double temp;



   switch (set[joint].mode)
     {
       case HOLD:
       case MOVE:
/* set central starting point first time move/hold is entered after free */
         if ( modeflag[joint] == FREE )   /* at last pass setmode was FREE */
           {
           modeflag[joint] = MOVE;
           state[joint].motor = 2048;  /* set starting point to middle  */
           }
         if ( modeflag[joint] == TRACK )   /* at last pass setmode was TRACK
*/
```

```
          if ( wrparms[18] == TRUE )   /* set to true if pos = 'I' */
          {
          modeflag[joint] = MOVE;
          wrparms[18] = FALSE; .
          set[PITCH].pos = wrparms[11];
          }


      bhold(joint);
      break;


   case TRACK:
     modeflag[joint] = TRACK;
     bhold(joint);
     set[joint].pos = state[joint].pot;  /* to reset pitchset during track
*/

     break;


   case FREE:
     modeflag[joint] = FREE;
     state[joint].motor = 0;
     set[joint].pos = state[joint].pot;  /* to reset wrist during free */
     break;


   case MUSH:
     modeflag[joint] = FREE;     /* uses same resetting as free mode */
     state[joint].motor = parms[joint].msh; /* assign mush parameter */
     set[joint].pos = state[joint].pot;  /* to reset wrist during mush */
     break;


   case LOCK:
     /* if previous mode was FREE keep wrist FREE in emerg. lock  */
     if ( modeflag[joint] == FREE )   /* at last pass setmode was FREE */
        set[joint].mode = FREE;
     break;
   }
 bdriver(joint);
}
```

```
/* BHOLD: Similar to HHOLD, but for Bridgestone. This routine compensates for
forces on the actuators by varying the position voltage. It uses proportional
control and velocity feedback. */


bhold (i)
int i;


{


  int offset;
  int xerror, verror, motor;


  xerror = set[i].pos - state[i].pot;
   /* limit speed by limiting the max absolute xerror   */
  if ( xerror > parms[i].r_hys )  xerror = parms[i].r_hys;
  else if ( xerror < -parms[i].r_hys )  xerror = -parms[i].r_hys;


  verror = state[i].deltax * parms[i].vgain;              /* vel. feedback */


  if ( xerror < -parms[i].f_hys ||  xerror > parms[i].f_hys )
    offset = ( ( xerror * parms[i].xgain ) / 32 ) - verror;
  else offset = 0;


  switch ( i )   /* the pitch and yaw actuators are coupled and must be   */
     {           /* handled together                                      */


    case YAW:
     if ( set[PITCH].mode == TRACK )  /* pitch tracking  */
        ;       /* do nothing with yaw during pitch tracking    */
     else        /* normal wrist hold mode */
       {
       state[4].motor -= offset/2; /* factor of 2 because of 2:1 gear   */
       state[5].motor += offset/2; /* ratio of yaw joint            */
          /* trim the complementary values    */
       if ( state[5].motor > 4095 ) state[5].motor = 4095;
       else if ( state[5].motor < 0 ) state[5].motor = 0;
       }
```

```
            break;


        case PITCH:
            if ( set[i].mode == TRACK )
                {
                state[i].motor = wrparms[15] + ( state[i].pot - pitch_center ) * 2
;
                state[YAW].motor = state[PITCH].motor;
                }
            else
                {
                state[4].motor += offset;
                state[5].motor += offset;
                }
            /* trim the complementary values   */
            if ( state[4].motor > 4095 ) state[4].motor = 4095;
            else if ( state[4].motor < 0 ) state[4].motor = 0;
            break;


        case ROLL:
            if ( set[ROLL].mode == TRACK )   /* roll tracking  */
                state[i].motor = wrparms[16] + ( state[i].pot - roll_center ) * 2.6 ;

            else
                state[i].motor += offset;
            break;
        }  /* end of switch */


if ( state[i].motor > 4095 ) state[i].motor = 4095; /* trim to 0 - 5 v range
*/
else if ( state[i].motor < 0 ) state[i].motor = 0;


/*  for testing roll only, pitch and yaw are set to const vals in parms */
/*                  2048 gives approx centering on yaw, pitch          */
/*  allows playing with ctrl parameters for 1 d/f, ie roll joint       */
if ( wrparms[0] == 2 )  /* 2 = code to center pitch and yaw */
    {
    state[4].motor = parms[4].dead;  /* spare variable initialized to 2048 */
    state[5].motor = parms[5].dead;
```

```
    }

} /* end of bhold  */

/* BDRIVER: Similar to HDRIVER, but for Bridgestone actuators */

bdriver (i)
int i;

{
  int dacout, comp_dacout;

  dacout = state[i].motor / wrparms[13];   /* scale 4095 to D/A range  */

  if ( set[i].mode == FREE || set[i].mode == MUSH )
     comp_dacout = dacout;     /* set complementary valve to primary value */
  else
     /* complementary value, so P1 + P2 = const; */
     comp_dacout = wrparms[14] - dacout;


  if (dacout > 255)          /* trims (clips) dacout to maximum pressure  */
     dacout = 255;

  if (comp_dacout > 255)    /* trims (clips) comp_dacout to maximum pressure
*/
     comp_dacout = 255;


  dac[parms[i].outadr] = dacout;            /* send posn. to DAC array */
  dac[parms[i].outadr + 3] = comp_dacout;  /* send complementary posn. to DAC
*/

  ltdac (parms[i].outadr, dacout);             /* output to dac  */
  ltdac (parms[i].outadr + 13, comp_dacout); /* output compl. to dac */

}
/********************
  CEN_WRST.C                              851213
```

```
*****************/

/*
      Routine for reading wrist pot values when wrist is centered
*/


#include   "stdio.h"

#include   "defines.h"
#include   "structs.h"
#include   "extrnvar.h"


cen_wrst()
{

 char dummy[15] ;

 clearscreen(0,24) ;
 movecursor(0,0) ;
 printf("Center wrist then press 'c' then press ENTER \n") ;
 scanf("%s\n",&dummy[0]) ;
 yaw_center = pots[4] ;
 pitch_center = pots[5] ;
 roll_center = pots[6] ;
 printf("centered values : YAW = %f, PITCH = %f, ROLL =
%f\n",yaw_center,pitch_center,roll_center) ;


}
/*******************
   CHANGEV.C                                     860206
 ******************/

/*
      This routine changes some of the voice recognition parameters
      The alterable parameters are:

      rth    =  Reject Threshold
```

```
th0    -  Word begining detection threshold

th1    -  Word boundary detection threshold

thu    -  Update score threshold

lf     -  Self Learning Flag

thl    -  Self Learning score threshold


vcf    =  Voice control flag

rjbzf  =  Reject buzzer flag

echbf  =  Echo back flag


*/



#include   "stdio.h"

#include   "defines.h"
#include   "structs.h"
#include   "extrnvar.h"



change_the_voice_recognition_parameters()
  {
    unsigned char  send_string[ 128 ],  receive_string[ 128 ];
    static unsigned int  rth = 40,  th0 = 40,  th1 = 40,  thu = 40;
    static unsigned int  lf = 0,  thl = 30,  vcf = 1,  rjbzf= 0,  echbf =
0;



    lockup( TRUE );
    movecursor(  0, 0  );
    clearscreen(  0, 24  );

    printf( "SAR-10 Voice Recognition Parameter Alteration Routine \n" );
    printf( "      Enter new values or <CR> for no change \n\n" );
```

```
flush_the_keyboard_buffer();


send_command_to_the_SAR10(  " ",   receive_string  );   /* Stop recog */



/*
    Get the current values of the variables
*/


send_command_to_the_SAR10(  "R\r",  receive_string  );
sscanf(  receive_string,  "%d ",  &rth  );


send_command_to_the_SAR10(  "A\r",  receive_string  );
sscanf(  receive_string,  "%d %d %d %d %d ",  &th0,  &th1,  &thu,
                                                &lf,   &thl          );


send_command_to_the_SAR10(  "B\r",  receive_string  );
sscanf(  receive_string,  "%d %d %d ",  &vcf,  &rjbzf,  &echbf       );



/*
    Ask the operator for the new values
*/


    ask_operator_for_input(  "Reject Threshold                   rth
(%3d): ",  &rth  );


    ask_operator_for_input(  "Word Begin Detection Threshold     th0
(%3d): ",  &th0  );
    ask_operator_for_input(  "Word Boundary Detection Threshold  th1
(%3d): ",  &th1  );
    ask_operator_for_input(  "Update Score Threshold             thu
(%3d): ",  &thu  );
    ask_operator_for_input(  "Self Learning Flag                 lf
(%3d): ",  &lf  );
    ask_operator_for_input(  "Self Learning Score Threshold      thl
(%3d): ",  &thl  );
```

```
    ask_operator_for_input(  "Which Control Flag                         vcf
    (%d): ",  &vcf  );
    ask_operator_for_input(  "Reject Buzzer Flag                         rjbzf
    (%d): ",  &rjbzf );
    ask_operator_for_input(  "Echo Back Flag                             ecnbf
    (%d): ",  &ecnbf );




    Write back the changed parameters
*/


    sprintf(  send_string,  "E,%d\r",  rth  );
    send_command_to_the_SAR10(  send_string,  receive_string  );



    sprintf(  send_string,  "A %d,%d,%d,%d,%d\r",  th0,  th1,  thu,  lf,
 th1  );
    send_command_to_the_SAR10(  send_string,  receive_string  );



    sprintf(  send_string,  "B %d,%d,%d\r",  vcf,  rjbzf,  ecnbf  );
    send_command_to_the_SAR10(  send_string,  receive_string  );



    send_command_to_the_SAR10(  "M\r",  receive_string  );  /* Start recog
*/



    movecursor(  0,  0  );
    clearscreen(  0, 24  );
    mainmenu();
    lockup(  FALSE  );


    }
```

```
ask_operator_for_input( pointer_to_query_string, pointer_to_variable )
  unsigned char  *pointer_to_query_string;
  int            *pointer_to_variable;

  {

      printf( pointer_to_query_string,  *pointer_to_variable  );
      while( key_scan()  ==  (-1)  );
      if(   ( key_scan() & 0x00ff )  ==  0x000d   )
          {

          key();
          printf( "\n" );

          }
        else
          {

          scanf( "%d ", pointer_to_variable );
          *pointer_to_variable  &=  0x00ff;

          }


      }



send_command_to_the_SAR10( send, receive )
  unsigned char  *send, *receive;
  {


#define  SAR10_DATA_PORT    0x0350
#define  SAR10_STATUS_PORT  0x0351

      int  i;



      if( ( inportb( SAR10_STATUS_PORT ) & 0x0040 )  ==  0x0040   )
          {
```

```c
        printf( "  SAR-10 Hardware error ! \n"  );
        return( 0 );
    }


    while( ( inportb( SAR10_STATUS_PORT ) & 0x0002 ) == 0x0002 );
    outportb( SAR10_STATUS_PORT, 0x00 );        /* Disable interrupt
*/



    for( i = 0; send[ i ] != 0; i++ )
      {
        while( ( inportb( SAR10_STATUS_PORT ) & 0x0002 ) == 0x0002
);

        outportb( SAR10_DATA_PORT, send[ i ] );
      }



/*
    When the transparent handler is on,  output is sent to the keyboard
*/

    i = 0;

    do
      {
        while( ( inportb( SAR10_STATUS_PORT ) & 0x0001 ) == 0x0001 );
        receive[ i ] = inportb( SAR10_DATA_PORT ) & 0x00ff;
        if(                 receive[ i ]      != '\r'
            && isdigit( receive[ i ] ) == 0      )
          receive[ i ] = ' ';
      }
    while( receive[ i++ ] != '\r' );

    receive[ i ] = '\0';
```

)201883

Enable the SAR10 board interrupt


```
    while(  (  inportb( SAR10_STATUS_PORT )  &  0x0002  )  == 0x0002  );
    outportb( SAR10_STATUS_PORT,  0x01  );          /*  Enable interrupt  */
```


```
/***************

    DAC_RFS.C                                      060117

****************/


/*
    This routine refreshes the DAC outputs for the Bridgestone
    actuators.  It is activated by the timer interrupt, so it runs
    in the background.
*/



#include  "stdio.h"

#include  "defines.h"
#include  "structs.h"
#include  "extrnvar.h"



dac_refresh()


{
  if (ref_dac == TRUE)
    {
    ltdac(0, dac[0]);
    ltdac(1, dac[1]);
    ltdac(2, dac[2]);
    ltdac(13, dac[3]);
    ltdac(14, dac[4]);
    ltdac(15, dac[5]);
    }
```

}

```
/*******************

  DESELACT.C                        360205

 *****************/
```

```
/*

     This procedure reads the keyboard buffer, determines which selection

     or action has been requested, speaks the command word, and then

     returns the determined selction and determined action.


     Note that this routine determines the source of the command by the

     one character prefix to the command character.


          Source of Command              Command Character Prefix

            Keyboard                        None

            SAR-10 Speech Recognizer        'v'       -

            Hand Control Unit (Pendant)     'p'


*/
```

```
#include  "stdio.h"


#include  "defines.h"

#include  "structs.h"

#include  "extrnvar.h"




make_a_decision_on_the_determined_selection_and_action(
p2_determined_selection,  p2_determined_action,

p2_has_the_selection_and_action_been_verified    )
   int  *p2_determined_selection,  *p2_determined_action,
*p2_has_the_selection_and_action_been_verified;
   {
```

```
static   unsigned long     when_the_SAR10_microphone_got_turned_on;
         unsigned long     get_the_time_of_day();
static   unsigned int      was_the_most_recent_response_word_spoken,
                           the_most_recent_response_word;
static   unsigned int      command, previous_character,  command_source;
static   unsigned int      special_command;
         unsigned int      key();


/*

     If it has been more than 20 seconds since the ARTHROBOT command word
     was given, attention is turned off.  All voice commands are ignored
     until the word ARTHROBOT is heard.
 */


     if(  (  get_the_time_of_day() - when_the_SAR10_microphone_got_turned_on
) >  0x016CL  )
        {
          if(  attention -- YES  )
            {
              set_the_reject_buzzer_flag(  FALSE  );
              attention       -  NO;
            }
        }


/*
     Try to speak the last command word if it was not spoken
*/


     if(  was_the_most_recent_response_word_spoken  --  NO  )
        was_the_most_recent_response_word_spoken  -  speak(
the_most_recent_response_word  );



/*
     If there are no characters in the keyboard buffer return (-1)
*/
```

```
        if(     key_scan()    ==    (-1)        )
          return( -1 );



/*
        At this point there is at least one key in the buffer.  Read it.
*/ .


        previous_character   =   command;
        command              =   key();


        special_command   =   FALSE;


        switch(   command  )
           (


/*
        Deal with the special cases first
*/


            case  EMERGENCY_STOP_COMMAND :
            case  ' ' :
                        the_most_recent_response_word            =
EMERGENCY_STOP_RESPONSE;
                        was_the_most_recent_response_word_spoken = speak(
the_most_recent_response_word  );
                        *p2_determined_selection  = EMERGENCY_STOP_COMMAND;
                        *p2_determined_action     = 0;
                        *p2_has_the_selection_and_action_been_verified = YES;
                        special_command = TRUE;
                        break;


          case  VOICE :
                        command_source = VOICE;
                        special_command = TRUE;
                        break;


          case  HAND_CONTROL :
```

114                                          0201883

```
                    command_source  =  HAND_CONTROL;
                    special_command  =  TRUE;
                    break;


          case  ARTHROBOT_COMMAND :
                    set_the_reject_buzzer_flag( TRUE );
                    the_most_recent_response_word          =
ARTHROBOT_RESPONSE;
                    was_the_most_recent_response_word_spoken  =  speak(
the_most_recent_response_word );
                    when_the_SAR10_microphone_got_turned_on  =
get_the_time_of_day();
                    attention                              = YES;
                    *p2_determined_selection                = 0;
                    *p2_determined_action                   = 0;
                    *p2_has_the_selection_and_action_been_verified = NO;
                    special_command  =  TRUE;
                    break;


          case  OKAY_COMMAND :
                    the_most_recent_response_word          =
OKAY_RESPONSE;
                    was_the_most_recent_response_word_spoken  =  speak(
the_most_recent_response_word );
                    *p2_has_the_selection_and_action_been_verified = YES;
                    special_command  =  TRUE;
                    break;



          case  NO_COMMAND :
                    set_the_reject_buzzer_flag( FALSE );
                    the_most_recent_response_word          =
NO_RESPONSE;
                    was_the_most_recent_response_word_spoken  =  speak(
the_most_recent_response_word );
                    attention                              = NO;
                    *p2_determined_selection                = 0;
                    *p2_determined_action                   = 0;
                    *p2_has_the_selection_and_action_been_verified = NO;
```

```
                              special_command  =  TRUE;
                              break;

              default :
                              break;


          ;



/*
      Now deal with the selection commands
*/


      if( special_command ==  TRUE  )
        return( -1 );



      if(      previous_character  ==  VOICE
          &&  attention           ==  NO        )
            return( -1 );
        else
          {
      switch(  command  )
        {

          case  ELEVATE_COMMAND :
                      the_most_recent_response_word             =
ELEVATE_RESPONSE;
                      was_the_most_recent_response_word_spoken = speak(
the_most_recent_response_word );
                      *p2_determined_selection  = ELEVATE_COMMAND;
                      *p2_determined_action     = 0;
                      break;

          case  FULL_EXTENSION_COMMAND :
                      the_most_recent_response_word             =
FULL_EXTENSION_RESPONSE;
```

```
                was_the_most_recent_response_word_spoken = speak(
the_most_recent_response_word  );
                *p2_determined_selection  = FULL_EXTENSION_COMMAND;
                *p2_determined_action      = 0;
                break;


        case  HALF_FLEX_COMMAND :
                the_most_recent_response_word               =
HALF_FLEX_RESPONSE;
                was_the_most_recent_response_word_spoken = speak(
the_most_recent_response_word  );
                *p2_determined_selection  = HALF_FLEX_COMMAND;
                *p2_determined_action      = 0;
                break;


        case  FULL_FLEX_COMMAND :
                the_most_recent_response_word               =
FULL_FLEX_RESPONSE;
                was_the_most_recent_response_word_spoken = speak(
the_most_recent_response_word  );
                *p2_determined_selection  = FULL_FLEX_COMMAND;
                *p2_determined_action      = 0;
                break;


        case  FIGURE_4_COMMAND :
                the_most_recent_response_word               =
FIGURE_4_RESPONSE;
                was_the_most_recent_response_word_spoken = speak(
the_most_recent_response_word  );
                *p2_determined_selection  = FIGURE_4_COMMAND;
                *p2_determined_action      = 0;
                break;


        case  WRIST_COMMAND :
                the_most_recent_response_word               =
WRIST_RESPONSE;
                was_the_most_recent_response_word_spoken = speak(
the_most_recent_response_word  );
                *p2_determined_selection  = WRIST_COMMAND;
```

```
                    *p2_determined_action        = 0;
                    break;


        case  FOOT_COMMAND :
                    the_most_recent_response_word              =
FOOT_RESPONSE;
                    was_the_most_recent_response_word_spoken  = speak(
the_most_recent_response_word  );
                    *p2_determined_selection  = FOOT_COMMAND;
                    *p2_determined_action     = 0;
                    break;


        case  KNEE_COMMAND :
                    the_most_recent_response_word              =
KNEE_RESPONSE;
                    was_the_most_recent_response_word_spoken  = speak(
the_most_recent_response_word  );
                    *p2_determined_selection  = KNEE_COMMAND;
                    *p2_determined_action     = 0;
                    break;


        case  HIP_COMMAND :
                    the_most_recent_response_word              =
HIP_RESPONSE;
                    was_the_most_recent_response_word_spoken  = speak(
the_most_recent_response_word  );
                    *p2_determined_selection  = HIP_COMMAND;
                    *p2_determined_action     = 0;
                    break;


        case  ARM_COMMAND :
                    the_most_recent_response_word              =
ARM_RESPONSE;
                    was_the_most_recent_response_word_spoken  = speak(
the_most_recent_response_word  );
                    *p2_determined_selection  = ARM_COMMAND;
                    *p2_determined_action     = 0;
                    break;
```

I am including two additional selection commands that at present
can only be activated from the keyboard or by voice.  However, their
actions can be initiated from the hand control unit.

```
        case  COMPLIANCE_COMMAND :
                the_most_recent_response_word              =
COMPLIANCE_RESPONSE;
                was_the_most_recent_response_word_spoken  =  speak(
the_most_recent_response_word  );
                *p2_determined_selection  =  COMPLIANCE_COMMAND;
                *p2_determined_action     =  0;
                break;


        case  SETUP_COMMAND :
                the_most_recent_response_word              =
SETUP_RESPONSE;
                was_the_most_recent_response_word_spoken  =  speak(
the_most_recent_response_word  );
                *p2_determined_selection  =  SETUP_COMMAND;
                *p2_determined_action     =  0;
                break;




/*
        And with the action commands
*/



/*
        The '+' and '-' characters are specially treated keystokes.
        They will be assigned different actions depending on what the selection
        is.  This function has been put in so that the keyboard can truely
        mimic the hand control unit.
*/
        case  MINUS_COMMAND :
```

```
                        case  ELEVATE_COMMAND :
                        case  FULL_EXTENSION_COMMAND :
                        case  HALF_FLEX_COMMAND :
                        case  FULL_FLEX_COMMAND :
                        case  FIGURE_4_COMMAND :
                        case  SETUP_COMMAND :
                          the_most_recent_response_word            =
MOVE_RESPONSE;

                          was_the_most_recent_response_word_spoken =
speak( the_most_recent_response_word );
                          *p2_determined_action                   =
MOVE_COMMAND;

                          *p2_has_the_selection_and_action_been_verified =
YES;

                          break;


                        case  ARM_COMMAND :
                          the_most_recent_response_word            =
IN_RESPONSE;

                          was_the_most_recent_response_word_spoken =
speak( the_most_recent_response_word );
                          *p2_determined_action                   =
IN_COMMAND;

                          *p2_has_the_selection_and_action_been_verified =
YES;

                          break;


                        case  HIP_COMMAND :
                          the_most_recent_response_word            =
FLEX_RESPONSE;

                          was_the_most_recent_response_word_spoken =
speak( the_most_recent_response_word );
                          *p2_determined_action                   =
FLEX_COMMAND;

                          *p2_has_the_selection_and_action_been_verified =
YES;
```

```
                               break;

               case  FREE_COMMAND :
               the_most_recent_response_word            =
FLEX_RESPONSE;
               was_the_most_recent_response_word_spoken =
speak(  the_most_recent_response_word  );
               *p2_determined_action                    =
FLEX_COMMAND;
               *p2_has_the_selection_and_action_been_verified =
YES;
               break;
               case  FOOT_COMMAND :
               the_most_recent_response_word            =
IN_RESPONSE;
               was_the_most_recent_response_word_spoken =
speak(  the_most_recent_response_word  );
               *p2_determined_action                    =
IN_COMMAND;
               *p2_has_the_selection_and_action_been_verified =
YES;
               break;

               case  WRIST_COMMAND :
               the_most_recent_response_word            =
RELEASE_RESPONSE;
               was_the_most_recent_response_word_spoken =
speak(  the_most_recent_response_word  );
               *p2_determined_action                    =
RELEASE_COMMAND;
               *p2_has_the_selection_and_action_been_verified =
YES;
               break;

               case  COMPLIANCE_COMMAND :
               the_most_recent_response_word            =
LESS_RESPONSE;
               was_the_most_recent_response_word_spoken =
speak(  the_most_recent_response_word  );
```

```
                              *p2_determined_action
LESS_COMMAND;

                              *p2_has_the_selection_and_action_been_verified  =
YES;

                              break;


                           default:
                              break;


                        ;


                 break;



        case   PLUS_COMMAND :
                    switch(  *p2_determined_selection  )
                       {


                          case   ELEVATE_COMMAND :
                          case   FULL_EXTENSION_COMMAND :
                          case   HALF_FLEX_COMMAND :
                          case   FULL_FLEX_COMMAND :
                          case   FIGURE_4_COMMAND :
                          case   SETUP_COMMAND :
                             the_most_recent_response_word          =
STOP_RESPONSE;

                             was_the_most_recent_response_word_spoken =
. speak(  the_most_recent_response_word  );
                             *p2_determined_action                   =
STOP_COMMAND;

                             *p2_has_the_selection_and_action_been_verified  =
YES;

                             break;


                          case  ARM_COMMAND :
                             the_most_recent_response_word          =
OUT_RESPONSE;

                             was_the_most_recent_response_word_spoken =
speak(  the_most_recent_response_word  );
```

```
                            *p2_determined_action                =
OUT_COMMAND;
                            *p2_has_the_selection_and_action_been_verified  =
NO;

                            break;

                    case  HIP_COMMAND :
                            the_most_recent_response_word           =
EXTEND_RESPONSE;
                            was_the_most_recent_response_word_spoken =
speak(  the_most_recent_response_word  );
                            *p2_determined_action                =
EXTEND_COMMAND;
                            *p2_has_the_selection_and_action_been_verified  =
NO;

                            break;

                    case  KNEE_COMMAND :
                            the_most_recent_response_word           =
EXTEND_RESPONSE;
                            was_the_most_recent_response_word_spoken =
speak(  the_most_recent_response_word  );
                            *p2_determined_action                =
EXTEND_COMMAND;
                            *p2_has_the_selection_and_action_been_verified  =
YES;

                            break;
                    case  FOOT_COMMAND :
                            the_most_recent_response_word           =
OUT_RESPONSE;
                            was_the_most_recent_response_word_spoken =
speak(  the_most_recent_response_word  );
                            *p2_determined_action                =
OUT_COMMAND;
                            *p2_has_the_selection_and_action_been_verified  =
YES;

                            break;

                    case  WRIST_COMMAND :
```

```
                    the_most_recent_response_word         =
HOLD_RESPONSE;
                    was_the_most_recent_response_word_spoken =
speak(  the_most_recent_response_word  );
                    *p2_determined_action                   =
HOLD_COMMAND;
                    *p2_has_the_selection_and_action_been_verified  =
YES;
                    break;


                case  COMPLIANCE_COMMAND :
                    the_most_recent_response_word         =
MORE_RESPONSE;
                    was_the_most_recent_response_word_spoken =
speak(  the_most_recent_response_word  );
                    *p2_determined_action                   =
MORE_COMMAND;
                    *p2_has_the_selection_and_action_been_verified  =
YES;
                    break;


                default:
                    break;


                }


                break;



/*
        Back to regular commands
*/


        case  MOVE_COMMAND :
                    the_most_recent_response_word         =
MOVE_RESPONSE;
                    was_the_most_recent_response_word_spoken  =  speak(
the_most_recent_response_word  );
                    *p2_determined_action  =  MOVE_COMMAND;
```

```
                *p2_has_the_selection_and_action_been_verified =
YES;

                break;


        case    STOP_COMMAND :
                the_most_recent_response_word               =
STOP_RESPONSE;

                was_the_most_recent_response_word_spoken  =  speak(
the_most_recent_response_word );
                *p2_has_the_selection_and_action_been_verified  = YES;
                *p2_determined_action   =   STOP_COMMAND;
                break;


        case    RELEASE_COMMAND :
                the_most_recent_response_word               =
RELEASE_RESPONSE;

                was_the_most_recent_response_word_spoken  =  speak(
the_most_recent_response_word );
                *p2_determined_action   =   RELEASE_COMMAND;
                  *p2_has_the_selection_and_action_been_verified  =
YES;

                break;


        case    HOLD_COMMAND :
                the_most_recent_response_word               =
HOLD_RESPONSE;

                was_the_most_recent_response_word_spoken  =  speak(
the_most_recent_response_word );
                *p2_determined_action   =   HOLD_COMMAND;
                  *p2_has_the_selection_and_action_been_verified  =
YES;

                break;


        case    IN_COMMAND :
                the_most_recent_response_word               =
IN_RESPONSE;

                was_the_most_recent_response_word_spoken  =  speak(
the_most_recent_response_word );
```

```
                *p2_determined_action = CUT_COMMAND;
                *p2_has_the_selection_and_action_been_verified =
YES;

                break;


        case  CUT_COMMAND :
                the_most_recent_response_word            =
CUT_RESPONSE;
                was_the_most_recent_response_word_spoken  =  speak(
the_most_recent_response_word );
                *p2_determined_action  =  CUT_COMMAND;
                *p2_has_the_selection_and_action_been_verified  =
YES;

                break;


        case  FLEX_COMMAND :
                the_most_recent_response_word
FLEX_RESPONSE;
                was_the_most_recent_response_word_spoken  =  speak(
the_most_recent_response_word );
                *p2_determined_action  =  FLEX_COMMAND;
                *p2_has_the_selection_and_action_been_verified  =
YES;

                break;


        case  EXTEND_COMMAND :
                the_most_recent_response_word            =
EXTEND_RESPONSE; .
                was_the_most_recent_response_word_spoken  =  speak(
the_most_recent_response_word );
                *p2_determined_action  =  EXTEND_COMMAND;
                *p2_has_the_selection_and_action_been_verified  =
YES;

                break;


    /*
        Additional actions for the COMPLIANCE command
    */
```

```
        case  LESS_COMMAND :

                        the_most_recent_response_word                =
LESS_RESPONSE;

                        was_the_most_recent_response_word_spoken  =  speak(
the_most_recent_response_word  );

                        *p2_determined_action  =  LESS_COMMAND;

                          *p2_has_the_selection_and_action_been_verified  =
YES;

                        break;


        case  MORE_COMMAND :

                        the_most_recent_response_word                =
MORE_RESPONSE;

                        was_the_most_recent_response_word_spoken  =  speak(
the_most_recent_response_word  );

                        *p2_determined_action  =  MORE_COMMAND;

                          *p2_has_the_selection_and_action_been_verified  =
YES;

                        break;


/*
    The rest are keyboard only commands. They are used strictly for
    debugging purposes.
*/

        default :

                        *p2_determined_selection  =  command;
                        *p2_determined_action      =  0;
                        *p2_has_the_selection_and_action_been_verified  = YES;
                        break;




        }


    return( command );
```

```
        )


  )
/*****************

  .DISPERR.C                          860129

 ****************/


/*

    If this routine is invoked with "rstflag" false, then it alerts the

    user to an error condition and locks the arm.  The user is then queried

    whether or not to ignore the error when operation is resumed.  If
"rstflag"

    is true, all subsequent errors are not ignored.

*/



#include   "stdio.h"


#include   "defines.h"

#include   "structs.h"

#include   "extrnvar.h"



disperr(err, joint, rstflag)

int err, joint, rstflag;


{

  int i, reply;

  static int ignore;


  if (rstflag == TRUE)

    ignore = FALSE;

  if (rstflag == FALSE)

    ignore = TRUE;

  if (ignore == FALSE)

    {
```

```
    if (err != FALSE)
        {
        lockup(TRUE);
        clearscreen (10,24);
        printf ("\07");
        movecursor (24,0);
        for (i=0; i<7; ++i)
            set[i].mode = LOCK;
        }


    switch (err)
        {
        case POTERR:
            printf ("Malfunction in pot %d, pot = %d", joint, pots[joint]);
            break;
        }


    if (err != FALSE)
        {
        movecursor (15,0);
        printf ("Ignore errors from now on? (y/n) ");
        while(  ( reply = key() ) == (-1)   );
        if (reply == 'y')
            ignore = TRUE;
        lockup(FALSE);
        }
    }
/******************

    DISPLAY.C                       851213

 ****************/


/*
    This routine displays the status of all joints.
*/



#include  "stdio.h"
```

0201883

```c
include "defines.h"
include "structs.h"
include "extrnvar.h"


display()


{

  int i, bad;
  double a1, a2, a3, a5, wx, wy;


  wx = wy = 0.0;
  lockup(TRUE);
  clearscreen (10,24);
  movecursor (10,0);
  printf ("Joint\tMode\tSet\tPot\n");
  for (i=0; i<7; ++i)
    printf ("%d\t%c\t%d\t%d\n", i, set[i].mode, set[i].pos, pots[i]);
  a1 = pots[1] * RAD_PER_POT1 + offset1;
  a2 = pots[2] * RAD_PER_POT2 + offset2;
  a3 = pots[3] * RAD_PER_POT3 + offset3;
  bad = arm2world (&wx, &wy, a1, a2, a3);
  for_leg(&elev,&flex,0.0,a1,a2,a3,0.0,0.0,0.0);
  a1 *= 57.29577951;
  a2 *= 57.29577951;
  a3 *= 57.29577951;
  elev *= 57.29577951;
  flex *= 57.29577951;
  if (bad == -1)
    printf ("Bad data in arm2world\n");
  printf ("\nArm coordinates (%.0f,%.0f,%.0f)\n",a1,a2,a3);
  printf ("World coordinates (%.0fmm,%.0fmm)\n",wx,wy);
  printf(" elevate = %3.2f    flex = %3.2f\n",elev,flex);


  if (pend_on == TRUE)
    printf ("Pendant is on.  sample = %d\n", pendsample);
  else
    printf ("Pendant is off\n");
```

```c
    setup(FALSE);
}

/*********************
   DIS_SCAL.C                                      130129
  *****************/

/*
      This routine displays the preprogrammed positions as stored
      in memory.
*/


#include   "stdio.h"
#include   "defines.h"
#include   "structs.h"
#include   "extrnvar.h"


dis_scale()

{
double x,y,angle1,angle2,angle3 ;
int i,j ;

  lockup(TRUE) ;
  clearscreen(15,24) ;
  movecursor(15,0) ;
  printf("position  x          y      pot1      pot2      pot3\n") ;
  for (i=0 ; i<5 ; i++ )
    {
    angle1 = memory[i][1]   * RAD_PER_POT1 + offset1 ;
    angle2 = memory[i][2]   * RAD_PER_POT2 + offset2 ;
    angle3 = memory[i][3]   * RAD_PER_POT3 + offset3 ;
    j = 1 + i ;
    arm2world( &x, &y, angle1, angle2, angle3 ) ;
    printf("   %2.0d   %6.2f   %6.2f   %6.0d   %6.0d
%6.0d\n",j,x,y,memory[i][1],memory[i][2],memory[i][3] );
    }
if (side == 1)
  printf("left leg\n");
```

```c
if (side == -1)
  printf(" right leg\n");
printf("leg = %6.2f   shiftx = %6.2f   shifty = %6.2f \n",leg,shiftx,shifty);
printf("elevate =%6.2f             , flex_ang = %6.2f \n",elevate,flex_ang) ;
lockup(FALSE) ;
}
/*******************
    FORWARD.C                              851213, 851031, 850828
 ****************/

/*
      This routine finds the (x,y) position of the end of the arm
      from the joint angles (a1,a2,a3).
      Units are millimeters for (x,y) and radians for (a1,a2,a3).
      Returns '0' if data good ; returns '-1' if data bad
*/



#include  "stdio.h"
#include  "defines.h"
#include  "structs.h"
#include  "extrnvar.h"



arm2world(x,y,a1,a2,a3)
double *x,*y,a1,a2,a3 ;


{
extern double sin(),cos();
int okay ;
double len0,len1,len2,len3,temp_a,xcoord,ycoord,pi  ;


len0 =  370.00 ;
len1 =  152.40 ;
len2 =  304.80 ;
len3 =  374.00 ;
pi   =  3.141592654 ;
okay = 1 ;
```

```
if ( okay == 1 )
{
temp_a = a1 +a2 ;
xcoord =   len0 ;
xcoord += ( len1 * cos(a1) ) ;
xcoord += ( len2 * cos(a2) * cos(a1) ) ;
xcoord -= ( len2 * sin(a2) * sin(a1) ) ;
xcoord += ( len3 * cos(a3) * cos(temp_a) ) ;
xcoord -= ( len3 * sin(a3) * sin(temp_a) ) ;
ycoord = ( len1 * sin(a1) ) ;
ycoord += ( len2 * cos(a2) * sin(a1) ) ;
ycoord += ( len2 * sin(a2) * cos(a1) ) ;
ycoord += ( len3 * cos(a3) * sin(temp_a) ) ;
ycoord += ( len3 * sin(a3) * cos(temp_a) ) ;
*x = xcoord ;
*y = ycoord ;
return(0);
}
else return(-1);
}
/******************
   FOR_LEG.C                                       851203, 851115
 ****************/


/*
    This routine calculates the leg hip elevation and knee flex angles
    from the arm joint angles.
*/



#include  "stdio.h"

#include  "defines.h"
#include  "structs.h"
#include  "extrnvar.h"



for_leg(elevt,flexx,a0,a1,a2,a3,a4,a5,a6)
```

```c
double *elevt, *flexx,a0,a1,a2,a3,a4,a5,a6 ;

{
extern double sin(),cos(),acos(),asin(),sqrt(),atan() ;
double elv,flx,u,v,x,y,ul,ll,att,hhx,hhy,kkx,kky,pi ;
double flx_offset_ang,temp_ang,r,u2,v2,ul2,r2,A,B,C,NA,NB,NC ;

                        /* a0        shoulder swing
                           a1        joint #1
                           a2        joint #2
                           a3        joint #3
                           a4        yaw
                           a5        pitch
                           a6        roll


                           WIDTH     PAP WIDTH
                           DEPTH·    PAP DEPTH
                           ul        upper leg length
                           ll        lower leg length
                           leg       total leg length
                           shiftx    horizontal shoulder to arm
                           shifty    vertical shoulder to arm
                           x,y       arm coordinates of pitch axis
                           u,v       leg coordinates of pitch axis
                        */
                        /* calculate upper and lower leg lengths */
pi = 3.141592654 ;
ul = leg / 2.0 ;
ll = ul ;
                        /* calculate x,y */
arm2world(&x,&y,a1,a2,a3) ;
                        /* calculate u,v */
u = x - shiftx ;
v = y - shifty ;


                        /* calculate knee x */
r = sqrt ( (WIDTH*WIDTH) + ((ll-DEPTH)*(ll-DEPTH)) ) ;
u2 = u * u ;
ul2 = ul * ul ;
```

0201883

```
u2 = r * r ;
v2 = v * v ;
A =  4 * (u2 + v2) ;
B =  4 * v * (r2 - ul2 - u2 - v2) ;
C =  ((r2-ul2)*(r2-ul2)) + ((u2-v2)*(u2-v2)) ;
C+= 2*r2*(u2-v2) + 2*ul2*(v2-u2) + 4*u2*(v2-r2) ;
NC = C/A ;
NB = B/A ;
kky = (-NB + sqrt( (NB*NB) - 4 * NC ))/2 ;
kkx = sqrt( ul2 - (kky*kky) ) ;



                          /* calculate elevate and flexx angles */
if ( (kkx/ul) > 0.5 )
    elv = asin(kky/ul) ;
    else elv = acos(kkx/ul) ;
if ( elv < 0.0 )
   elv = 0.0 ;


flx_offset_ang = atan( WIDTH / (ll-DEPTH) ) ;
if ( ((v-kky) > 0.0)  && ((u-kkx) > 0.0) )
    temp_ang = atan( (v-kky) / (u-kkx) ) ;
else if ( ((v-kky) < 0.0)  && ((u-kkx) > 0.0) )
       (
        if ( ((u-kkx)/r) > 0.5 )
           temp_ang = asin( (v-kky) / r ) ;
         else temp_ang = -acos( (u-kkx) / r ) ;
        )
else if ( ((v-kky) < 0.0)  && ((u-kkx) < 0.0) )
       (
         if ( ((u-kkx)/r) > 0.5 )
           temp_ang = (-pi/2.0) + asin( (v-kky) / r ) ;
         else temp_ang =  - acos( (u-kkx) / r ) ;
       )
att = temp_ang + flx_offset_ang ;
flx = att - elv ;


if ( flx > 0.0 )
   flx = 0.0 ;
```

```
                               /* return elevate and flex angle values */
     *elevt = elv ;
     *flexx = flx ;
     :

     /***************
        GETSTATS.C                          860129
     ***************/


     /*

          This routine retrieves the position and error status from each joint
     */



       include   "stdio.h"


     #include   "defines.h"
     #include   "structs.h"
     #include   "extrnvar.h"




     getstatus()


     {

       int i, error;

       for (i=0; i<7; ++i)
         {
         pots[i] = state[i].pot;              /* get latest pot reading */
         error = fault[i];                    /* check for faults */
         if (error != 0)
           {
           disperr(error, i, -1);
           fault[i] = 0;
           }
         }
     }
     /*****************
        HANDCONT.C                          860206
```

```
*****************/

                                                      0201883

/*

        This file contains 4 routines that are associated with

        sensing and acting on values read from the new hand control units

*/




#include   "stdio.h"

#include   "defines.h"
#include   "structs.h"
#include   "extrnvar.h"




check_to_see_if_the_hand_control_has_any_commands_for_us(
current_desired_selection,  current_desired_action  )
  int  current_desired_selection,  current_desired_action;
  {
        static   int     present_p_selection,  previous_p_selection,
                         present_p_action,     previous_p_action,
                         average_pendant_sample;



        average_pendant_sample  =  examine_the_pendant_input(  pendsample  );

        if(   average_pendant_sample  >=  0    )
            {

/*

        You have a stable pendant reading.  Decide whether it is a
        selection or an action.
*/

                if(   average_pendant_sample  >   800   )
                    {
```

```
/*

     It is a selection.  Categorize it.
*/


              present_p_selection  =  categorize_selection(
average_pendant_sample  );


              if( present_p_action != 0 )
                flush_the_keyboard_buffer();



              if( present_p_action != 0   &&   trained == YES  )
                switch( current_desired_selection )
                    {

                                                 /* Stop the arm
and hold the wrist    */
                      case ARM_COMMAND :         /* when an action
button is released */
                      case HIP_COMMAND :         /* after doing an
incremental command */
                      case KNEE_COMMAND :
                      case FOOT_COMMAND :


stop_and_maintain_the_present_position_of_the_arm();
                              break;


                      default :
                          break;


                    }



              present_p_action  =  0;
              previous_p_action =  0;
              are_we_in_continuous_motion  =  NO;


              if(    present_p_selection >= 0
```

```
                    if  present_p_selection != previous_p_selection
                    {

                    stuff_selection_or_action_into_the_keyboard_buffer(
present_p_selection,  present_p_action );
                    previous_p_selection = present_p_selection;
                    }


                }



            else
                {


    /*

        Its an action.  Categorize it.

    */


                present_p_action = categorize_action(
average_pendant_sample );


                if(     present_p_action  != previous_p_action
                    &&  present_p_action  >= 0                    )
                    {

                    stuff_selection_or_action_into_the_keyboard_buffer(
present_p_selection,  present_p_action );
                    previous_p_action = present_p_action;
                    }


                if(     is_the_arm_moving()  == YES
                    &&  present_p_action      >= 0               )
                    {

                    stuff_selection_or_action_into_the_keyboard_buffer(
present_p_selection,  present_p_action );
                    previous_p_action = present_p_action;
                    are_we_in_continuous_motion = YES;
                    }
```

```
                }



                }

/*
    .   Thats it for this routine
*/



    }




examine_the_pendant_input( digital_value )
   int  digital_value;
   {



#define     SAMPLES_TO_LOOK_AT        3
#define     ARRAY_SIZE                SAMPLES_TO_LOOK_AT + 1
#define     VWINDOW                   24



     static   int   previous_pendant_values[ ARRAY_SIZE ];
     int            i, average, stable_readings;


      previous_pendant_values[ SAMPLES_TO_LOOK_AT ] = digital_value;

      for( i = 0;  i < SAMPLES_TO_LOOK_AT;  i++ )
        previous_pendant_values[ i ] = previous_pendant_values[ i + 1 ];
```

```
    for(  average = 0,   i = 0;   i < SAMPLES_TO_LOOK_AT;   i++  )
      average += previous_pendant_values[ i ];


    average /= SAMPLES_TO_LOOK_AT;



    for(  stable_readings = 1,  i = 0;   i < SAMPLES_TO_LOOK_AT;   i++  )
       if(   iabs( previous_pendant_values[ i ]  -  digital_value  )  >
VWINDOW   )

             stable_readings  =  0;



    if(   stable_readings   )
       {
        pokeb(  314,  0xb000,  ' '  );
        return(  average  );
       }
     else
       {
        pokeb(  314,  0xb000,  '?'  );
        return(  -1  );
       }



  }




categorize_selection( average_value )
  int  average_value;
  {


#define  NUMBER_OF_SELECTIONS  10
#define  SWINDOW               49
```

141

```
static    int      the_selection_ranges_1[ NUMBER_OF_SELECTIONS  ]
               = ( 4095, 2763, 3308, 2379, 3662,
                   2557, 3016, 2225, 3868, 2655  );


int               i, rotary_switch_position;



    .    for( rotary_switch_position = (-1), i = 0;  i <
NUMBER_OF_SELECTIONS;  i++ )
        if( iabs( average_value - the_selection_ranges_1[ i ] )  <
SWINDOW  )
                rotary_switch_position = i;


    if( rotary_switch_position <  0 )
            pokeb( 316, 0xb000, '?' );
      else pokeb( 316, 0xb000, '0' + rotary_switch_position );


    return( rotary_switch_position );



    }




categorize_action( average_value )
  int average_value;
  {


#define NUMBER_OF_ACTIONS    4
#define AWINDOW             31

    static    int      the_action_ranges_1[ NUMBER_OF_ACTIONS  ]
               = ( 4095, 433, 206, 150 );
```

```
        int                    i, push_button_setting;


        for(   push_button_setting = (-1),  i = 0;   i < NUMBER_OF_ACTIONS;
i++  )
            if(   iabs( average_value - the_action_ranges_1[ i ]  )   <
AWINDOW  )
                    push_button_setting  =  i;


        if( push_button_setting  <  0  )
                pokeb( 318,  0xb000,  '?'  );
            else  pokeb( 318,  0xb000,  '0' + push_button_setting  );


        return( push_button_setting  );



    }
```

```
/*****************

    HOME.C                                         851213, 850828

 ****************/


/*
    This routine uses absolute values for potentiometer nested
    arm positions  to move the arm to a nested or 'home' position
    when a 'N' is pressed in lock mode.
*/



#include  "stdio.h"

#include  "defines.h"
#include  "structs.h"
#include  "extrnvar.h"


  home()

  {
```

```
double angle1,angle2,angle3,pi,nstpot1,nstpot2,nstpot3 ;


  pi = 3.141592654 ;


  /* experimentally determined pot readings at the desired nest position*/
  nstpot1 = 1700 ;
  nstpot2 = 1363 ;
  nstpot3 = 2815 ;


                    /* set positions for arm nest position */
  set[1].pos = nstpot1 ;
  set[2].pos = nstpot2 ;
  set[3].pos = nstpot3 ;


  set[0].duty = PERIOD;
  set[1].duty = PERIOD;
  set[2].duty = PERIOD;
  set[3].duty = PERIOD;


  }
/******************
   HYDRO.C                          851203
  ***************/


/*
     Each type of actuator has its own control subroutines.  This routine
     controls the hydraulic actuators.  Hydraulic actuators have lock, hold,
     and move modes, but no free mode.
*/



#include  "stdio.h"

#include  "defines.h"
#include  "structs.h"
#include  "extrnvar.h"
```

```
hydro (joint)
int joint;


   {
   switch (set[joint].mode)
     {
       case LOCK:
         state[joint].motor - OFF;
         state[joint].valve - SHUT;
         set[joint].pos - state[joint].pot;
         break;
       case HOLD:
       case MOVE:
       case FREE:
         hhold (joint);
         break;
     }
   hdriver (joint);
   }
```

/* HHOLD: This is the guts of the HYDRO routine.  It uses pulse width
modulation to move the actuator at varying speeds and it holds positions
constant. */

```
hhold (i)
int i;

{
   int error, clock, deadtm;

   clock - parms[i].clock;
   deadtm - state[i].wait;
   error - (set[i].pos - state[i].pot) * parms[i].xgain;
   if ((parms[i].r_hys < error  &&  parms[i].f_hys > error)
       || deadtm > 1)
```

```
  {
    if (set[i].mode == MOVE)
      set[i].mode = HOLD;
    state[i].motor = OFF;
    state[i].valve = SHUT;
    if (deadtm == 0)                    /* in position for first time? */
      deadtm = parms[i].dead;           /* if so, set wait timer */
  . else
      if (deadtm > 1)                   /* if still counting then */
        --deadtm;                       /* count down */
  }
  else
    {
    deadtm = 0;                         /* set flag to show joint is moving
*/
    if (timer[clock] > set[i].duty)
      state[i].motor = OFF;
    else
      {
      state[i].valve = OPEN;
      if (error <= parms[i].r_hys)
        state[i].motor = REVERSE;
      else
        state[i].motor = FORWARD;
      }
    }
  state[i].wait = deadtm;
}


/* HDRIVER  The guts of the hydro routine, actually diddles the bits
   to turn off and on pumps, motors and things.                      */


hdriver(i)
int i;


{
int j;


j = parms[i].outadr;
```

```
outbits &= parms[i].reset;

outbits |= pump[j].mask & state[i].motor;

outbits |= parms[i].vmask & state[i].valve;

}
/*******************

   INCREM.C                        851203

 *****************/


/*

     This routine allows the joints to be incremented and decremented

     through the keyboard.  The user first types an "i", then the joint

number.

     Using the + and - keys, the joints can then be moved one step at a time.

     To exit this mode, the "i" key is pressed again.
*/



#include  "stdio.h"


#include  "defines.h"
#include  "structs.h"
#include  "extrnvar.h"



increment (code, mode)
int code, *mode;


{
   static int jnt=0;
   static int adj;

   if (*mode == 0)

     {
     lockup(TRUE);
     clearscreen (2,24);
     movecursor (2,0);
     printf (" J O I N T   I N C R E M E N T\n\n");
     printf ("      0-6  Joint number\n");
     printf ("      +    Extend joint\n        -    Contract joint\n");
```

```
                              147                          0201883
      printf ("     c    Coarse adjust      f    Fine adjust\n");
      printf ("     i    Exit increment mode\n");
      for (jnt=0; jnt<4; ++jnt)
        {
        set[jnt].mode = HOLD;          /*put each joint into hold mode */
        set[jnt].duty = PERIOD;        /*run at full duty cycle */
        }
    adj = 10;
      *mode = 1;
      lockup(FALSE);
      }
  else
      {
      if (code > 47  &&  code < 55)
        jnt = code - 48;
      else if (code == '+')
        set[jnt].pos += parms[jnt].inc * adj;
      else if (code == '-')
        set[jnt].pos -= parms[jnt].inc * adj;
      else if (code == 'c')
        adj = 10;
      else if (code == 'f')
        adj = 1;
      else if (code == 's')
        {
        set[jnt].duty -= 1;
        if (set[jnt].duty == 0) set[jnt].duty = 1;
        }
      else if (code == 'a')
        {
        set[jnt].duty += 1;
        if (set[jnt].duty >PERIOD) set[jnt].duty = PERIOD;
        }
      else
        {
        if (code != 'i')
          for (jnt=0; jnt<7; ++jnt)
            set[jnt].mode = LOCK;
        *mode = 0;
```

```
    mainmenu();

      }

    }

}

/******************

    INC_XY.C                              851213, 850731

 *****************/


/*        `

    This routine takes the current position of the arm, adds an offset,
    then sets a new position for the arm.  It also sets the speeds of the
    joints so that they will reach their final positions roughly at the
    same time.

*/



#include   "stdio.h"
#include   "defines.h"
#include   "structs.h"
#include   "extrnvar.h"



inc_xy (dx, dy)
   int dx, dy;


{

   int bad1, bad2, jnt,emergency ;
   double x, y;
   double angle1, angle2, angle3;

   emergency = 0 ;
   for (jnt=0; jnt<4; jnt++)
      if (set[jnt].mode == LOCK)
         emergency = 1 ;
   if ( !emergency )
     {
       bad1 = bad2 = 0;
       angle1 = pots[1] * RAD_PER_POT1 + offset1;   /* calc arm coordinates */
       angle2 = pots[2] * RAD_PER_POT2 + offset2;   /* calc arm coordinates */
```

```
      angle3 = pots[3] * RAD_PER_POT3 + offset3;
      if (debug)
       {
       printf("**********\n");          .
       printf("calling arm2world with a1=%f a2=%f
a3=%f\n",angle1,angle2,angle3);
       }
     bad1 = arm2world( &x, &y, angle1, angle2, angle3 ); /*calc x,y */
      if (debug)
       {
       printf("returns bad=%d x=%f y=%f\n",bad1,x,y);
       }


      x += dx;
      y += dy;
      if (debug)
       printf("after incrementing call world2arm with x=%f y=%f\n",x,y);


      bad2 = world2arm (&angle1, &angle2, &angle3,x,y);
      if (debug)
       printf("returns bad=%d a1=%f a2=%f a3=%f\n",bad2,angle1,angle2,angle3);


      if ((bad1 < 0) || (bad2 < 0))
        {
        x -= dx;
        y -= dy;
        }
      else
        {
        set[1].pos = (angle1 - offset1) / RAD_PER_POT1;   /* set new desired
pos */
        set[2].pos = (angle2 - offset2) / RAD_PER_POT2;   /* set new desired
pos */
        set[3].pos = (angle3 - offset3) / RAD_PER_POT3;
        for (jnt=0; jnt<4; jnt++)
          set[jnt].mode = MOVE;
        }
     setspeed(); /* set speed of each joint so they finish simultaneously */
     }
```

```
)
/******************
  INSELACT.C                                    860206
 ******************/


/*
     This procedure initiates the determined selection and action.
   . Also, several other housekeeping and status related functions
     can be chosen.
*/




#include  "stdio.h"

#include  "defines.h"
#include  "structs.h"
#include  "extrnvar.h"




initiate_the_determined_selection_and_action(  the_determined_selection,
                                               the_determined_action,
                                               p2_command_verified        )
   int  the_determined_selection,  the_determined_action,
        *p2_command_verified;
   {

#define  RESET    0

     if(  *p2_command_verified  ==  NO  )
       return(  -1  );


     switch(  the_determined_selection  )
       {
```

```
                           15        0201883

    case   EMERGENCY_STOP_COMMAND :
    case   ' ' :
                  put_the_arm_into_emergency_stop();
                  *p2_command_verified  -  RESET;
                  onpath - FALSE ;
                  return;
                  break;



    case   ELEVATE_COMMAND :

                  switch( the_determined_action  )
                    {
                    case  MOVE_COMMAND:
                                   elevate_the_arm();
                                   *p2_command_verified  -  RESET;
                                   break;
                    case  STOP_COMMAND:

stop_and_maintain_the_present_position_of_the_arm();
                                   *p2_command_verified  -  RESET;
                                   break;
                    default:
                                   break;
                    }
                  break;



    case   FULL_EXTENSION_COMMAND:

                  switch( the_determined_action  )
                    {
                    case  MOVE_COMMAND:
                                   full_extension_the_arm();
                                   *p2_command_verified  -  RESET;
                                   break;
                    case  STOP_COMMAND:

stop_and_maintain_the_present_position_of_the_arm();
```

```
                                        *p2_command_verified  =  RESET;

                                        break;

                    default:

                                        break;

                }

            break;



        case  HALF_FLEX_COMMAND:

                switch(  the_determined_action  )
                    {
                    case  MOVE_COMMAND:

                                        half_flex_the_arm();

                                        *p2_command_verified  =  RESET;

                                        break;

                    case  STOP_COMMAND:


stop_and_maintain_the_present_position_of_the_arm();

                                        *p2_command_verified  =  RESET;

                                        break;

                    default:

                                        break;

                    }

                break;



        case  FULL_FLEX_COMMAND:

                switch(  the_determined_action  )
                    {
                    case  MOVE_COMMAND:

                                        full_flex_the_arm();

                                        *p2_command_verified  =  RESET;

                                        break;

                    case  STOP_COMMAND:


stop_and_maintain_the_present_position_of_the_arm();

                                        *p2_command_verified  =  RESET;
```

```
                              break;
            default:
                              break;
        )             .
      break;



    case   FIGURE_4_COMMAND:

            switch(  the_determined_action  )
              (
              case  MOVE_COMMAND:
                              figure_4_the_arm();
                              *p2_command_verified  -  RESET;
                              break;
              case  STOP_COMMAND:

stop_and_maintain_the_present_position_of_the_arm();
                              *p2_command_verified  -  RESET;
                              break;
              default:
                              break;
              )
            break;



    case   SETUP_COMMAND:

            switch(  the_determined_action  )
              (
              case  MOVE_COMMAND:
                              setup_the_arm();
                              *p2_command_verified  -  RESET;
                              break;
              case  STOP_COMMAND:

stop_and_maintain_the_present_position_of_the_arm();
                              *p2_command_verified  -  RESET;
                              break;
```

```
                    default:

                                break;

                }

            break;



    case    ARM_COMMAND:

                switch(  the_determined_action  )
                {

                    case   IN_COMMAND:
                                move_arm_in();
                                *p2_command_verified  =  RESET;
                                break;
                    case   OUT_COMMAND:
                                move_arm_out();
                                *p2_command_verified  =  RESET;
                                break;
                    case   STOP_COMMAND:

stop_and_maintain_the_present_position_of_the_arm();
                                *p2_command_verified  =  RESET;
                                break;
                    default:

                                break;


                }

            break;



    case   HIP_COMMAND:

                switch(  the_determined_action  )
                {

                    case   FLEX_COMMAND:
                                flex_the_hip();
```

```
                                        *p2_command_verified  =  RESET;

                                        break;

                    case  EXTEND_COMMAND:

                         .          extend_the_hip();

                                        *p2_command_verified  =  RESET;

                                        break;

                    case  STOP_COMMAND:


stop_and_maintain_the_present_position_of_the_arm();

                                        *p2_command_verified  =  RESET;

                                        break;

                    default:

                                        break;



                    }


                break;



        case   KNEE_COMMAND:


                switch(  the_determined_action   )
                    (

                        case  FLEX_COMMAND:

                                        flex_the_knee();

                                        *p2_command_verified  =  RESET;

                                        break;

                        case  EXTEND_COMMAND:

                                        extend_the_knee();

                                        *p2_command_verified  =  RESET;

                                        break;

                        case  STOP_COMMAND:


stop_and_maintain_the_present_position_of_the_arm();

                                        *p2_command_verified  =  RESET;

                                        break;

                        default:
```

```
                                    break;                          0201883

                )


                break;



        case   FOOT_COMMAND:


                switch(  the_determined_action  )
                 {


                    case   IN_COMMAND:
                                rotate_foot_in();
                                *p2_command_verified  -  RESET;
                                break;
                    case   OUT_COMMAND:
                                rotate_foot_out();
                                *p2_command_verified  -  RESET;
                                break;
                    case  STOP_COMMAND:

stop_and_maintain_the_present_position_of_the_arm();
                                *p2_command_verified  -  RESET;
                                break;
                    default:
                                break;


                 }


                break;



        case   WRIST_COMMAND:


                switch(  the_determined_action  )
                 {


                    case   RELEASE_COMMAND:
```

157

```
                                  release_the_wrist();        0201883
                                  *p2_command_verified  =  RESET;
                                  break;
                    case  HOLD_COMMAND:
                                  hold_the_wrist();
                                  *p2_command_verified  =  RESET;
                                  break;
                    case  STOP_COMMAND:

stop_and_maintain_the_present_position_of_the_arm();
                                  *p2_command_verified  =  RESET;
                                  break;
                    default:
                                  break;


                    )


             break;



        case  COMPLIANCE_COMMAND:

                  switch(  the_determined_action  )
                    (

                        case  LESS_COMMAND:
                                  adjust_the_wrist_compliance(  '+'  );
                                  *p2_command_verified  =  RESET;
                                  break;
                        case  MORE_COMMAND:
                                  adjust_the_wrist_compliance(  '-'  );
                                  *p2_command_verified  =  RESET;
                                  break;
                        case  STOP_COMMAND:

stop_and_maintain_the_present_position_of_the_arm();
                                  *p2_command_verified  =  RESET;
                                  break;
                        default:
```

```
                                     break;                    0201883

                  }


               break;



       case    ALT_N :                        /* absolute nesting to home
position */
          for (joint=0; joint<4; joint++)
            set[joint].mode = MOVE;
          home();
          break;


       case    CURSOR_UP :
          inc_xy (0,25);                      /* go up */
          break;


       case    CURSOR_DOWN :
          inc_xy (0,-25);                     /* go down */
          break;


       case    CURSOR_LEFT :
          inc_xy (25*side,0);                 /* go left */
          break;


       case    CURSOR_RIGHT :
          inc_xy (-25*side,0);                /* go right */
          break;



       case    ALT_I :                        /* increment mode */
          increment( 'i', &mode);
          break;


       case    ALT_J :
          adjust();                           /* adjust joint parameters */
```

```
      break;


case    ALT_W :
  adjustwr();                          /* adjust wrist parameters */
  break;


case    ALT_M :                        /* wrist mush mode */
  for (joint=4; joint<7; ++joint)
    set[joint].mode = MUSH;
  external_rotation = 0;
  break;


case    ALT_T :                        /* wrist tracking mode
*/
  set[5].mode = TRACK;                 /* set pitch to tracking mode
*/
  set[6].mode = TRACK;                 /* set roll to tracking mode
*/
  external_rotation = 0;
  break;


case    ALT_C :
  cen_wrst() ;                         /* Centre the wrist   */
  break ;


case    ALT_B :
  scale_the_wrist();                   /* Setup for auto compliance
*/
  break ;


case    ALT_S :                        /* train current position */
  store( 's',    &mode);
  break;


case    ALT_A :
  scale();                             /* Autoscaling of workspace */
  scale_the_wrist();
  break ;
```

```
        case    'Q':                        /* perform nest command */
            nest();
            break ;

        case    ALT_V :                      /* Change some of the
voice   */
            change_the_voice_recognition_parameters(); /* recognition
parameters    */
            break;

        case    'D':                         /* display status */
            display();
            break;

        case    'A' :
            dis_scale();                     /* Display scaled parameters
*/
            break ;

        case    ALT_O :                      /* toggle safe leg limits
in/out */
            safety ^= TRUE ;
            break ;

        case    ALT_H :                      /* toggle path on/off */
            pathon ^= TRUE ;
            break ;

        case    ALT_P :
            pend_on ^= TRUE;                 /* toggle pendant on/off */
            break;

        case    ALT_D :                      /*  Toggle debug  */
            debug  ^= TRUE;
            break;

        case    ALT_Z :                      /*  Toggle position cycler  */
            cycler ^= TRUE;
```

```
            cycle_position = 0;
            break;

        case    '%' :                            /*  Temporarily return to DOS
*/
            lockup(  TRUE  );                    /*  Temporarily hold the arm
*/
            movecursor( 0, 0 );
            clearscreen( 0, 24 );
            system( "command.com"  );
            movecursor( 0, 0 );
            clearscreen( 0, 24 );
            mainmenu();
            lockup(  FALSE );                    /*  Return to proper control
*/
            break;


        case    ALT_X :                          /*  Signify program exit
*/
            cont = FALSE;
            break;


        default  :
            break;




        }



    *p2_command_verified = RESET;




    }
/*****************
```

```
/**********************/

/* key uses the functions "key_scan" and "key_getc" from the
   C86 V2.3 library.  These are the most direct ways to get
   characters from the keyboard.
*/


key()
  {

    int   pressed;

    if( key_scan() == (-1)  )
      return(  -1  );

    pressed =  key_getc();
    if(  ( pressed & 0x00ff )  ==  0   )
          return(  pressed  );
      else  return(  pressed & 0x00ff  );


  }
/******************
   LOCKUP.C                           851203
 ******************/


/*
    This routine locks the arm during times when it cannot
    be continuously controlled.
*/



#include   "stdio.h"

#include   "defines.h"
#include   "structs.h"
#include   "extrnvar.h"
```

```
lockup (lockflag)
int lockflag;


(

  if (lockflag == TRUE)
     (
     dwrport (8, outbits & 0xE000);
     ref_dac = TRUE;
     )
  else
     (
     wtchdog();          /*writes out valve settings and trips timer */
     ref_dac = FALSE;
     )
)
/*******************

   MAINMENU.C                     860129

 *****************/


/*

    This routine prints the list of main menu choices.
*/



#include  "stdio.h"

#include  "defines.h"
#include  "structs.h"
#include  "extrnvar.h"



mainmenu()

(

  lockup(TRUE);
  clearscreen(  0,  24  );
  movecursor(  0,  23  );
  printf (           "  ARTHROBOT II    Control Program\n\n\n"  );
```

```
quick_puta( 0, 23, "              "    );    /* high intens0201883

clearscreen(2,23);
movecursor(2,0);


printf( "f1 - Arm     f2 - Elevate         Grey - : Move, In, Flex,
Release, Less\n" );
printf( "f3 - Hip     f4 - Full Extension   Grey + : Stop, Out, Extend,
Hold, More\n" );
printf( "f5 - Knee    f6 - Half Flex        SPACE  : Emergency Halt
\n" );
printf( "f7 - Foot    f8 - Full Flex
\n" );
printf( "f9 - Wrist  f10 - Figure 4          A : Display scaled values
\n" );
printf( " 1 - Compl    2 - Setup             D : Display joint status
\n" );
printf( "                          <- + ->   Alt A : Scale the workspace
\n" );
printf( "             Moving the arm        Alt B : Setup for auto
compliance    \n" );
printf( "                                   Alt C : Centre the wrist
\n" );
printf( "Alt Q : Reserved                   Alt D : Set debug mode
\n" );
printf( "Alt R : Reserved                   Alt E : Reserved
\n" );
printf( "Alt S : Store current position     Alt F : Reserved
\n" );
printf( "Alt T : Set wrist tracking mode    Alt G : Reserved
\n" );
printf( "Alt U : Reserved                   Alt H : Toggle path on or off
\n" );
printf( "Alt V : Change recog parameters    Alt I : Incremental joint
control    \n" );
printf( "Alt W : Adjust wrist parameters    Alt J : Adjust joint
parameters        \n" );
printf( "Alt X : Program exit               Alt K : Reserved
\n" );
```

```
  printf(  "Alt Y : Reserved                        Alt L : Toggle leg safe
limits         \n"  );
  printf(  "Alt Z : Toggle position cycler        Alt M : Set wrist mush mode
\n"  );
  printf(  "     @ : Perform nest calculations  Alt N : Absolute nesting to
home      \n"  );
  printf(  "     %% : Temporarily go to DOS        Alt O : Reserved
\n"  );
  printf(  "                                       Alt P : Toggle pendant on or
off      \n"  );



  /* compliance level is displayed in top right corner of monitor */
  pokeb( 308, 0xb000, '0' + wrcompmap[0] );


  lockup(FALSE);


}



quick_puts(  r,  c,  string_pointer  )              /* Quick put string
*/
  unsigned short  r, c;
  unsigned char   *string_pointer;
  {
    unsigned short  video_segment;
    unsigned short  peek();
                                                    /* Check equip flags
*/

      if(  (  peek(  0x0410,  0x0000  )  &  0x0030  )  ==      0x0030     )
            video_segment  =  0xb000;                /*  Monochrome board
*/
        else  video_segment  =  0xb800;             /*  Colour board
*/
```

```
        for (  r =  r * 160  +  c * 2;
               pokeb(  r,  video_segment,  *(string_pointer++)  )  != 0;
               r += 2           .
);


   }
```

```
quick_puta(  r,  c,  attribute_pointer  )      /* Quick put attribute
*/
  unsigned short  r, c;
  unsigned char   *attribute_pointer;
  {
    unsigned short  video_segment;
    unsigned short  peek();
                                                  /* Check equip flags
*/

      if(  (  peek(  0x0410,  0x0000  )  &  0x0030  )  ==  0x0030  )
             video_segment = 0xb000;             /* Monochrome board
*/
        else  video_segment = 0xb800;           /* Colour board
*/

      for (  r =  r * 160  +  c * 2  +  1;
             pokeb(  r,  video_segment,  *(attribute_pointer++)  )  != 0;
             r += 2
);


   }
/******************
   NEST.C                        851203
  *****************/
```

```
/*
    This routine interprets the current position of the arm as the nested
    position.  It sets the variables "offset1" ,"offset2", and "offset3" to
    establish an absolute angular. reference.
*/



#include  "stdio.h"

#include  "defines.h"
#include  "structs.h"
#include  "extrnvar.h"



nest ()


{


double nstang1,nstang2,nstang3 ;

        /* desired arm nest angles in radians */
  nstang1 = -1.570796327 ;
  nstang2 = -1.570796327 ;
  nstang3 =  2.972392682 ;


        /* Now assume arm is properly nested ; get pot readings */
        /* Calculate the new calibrated offsets */
  offset1 = nstang1 - pots[1] * RAD_PER_POT1;
  offset2 = nstang2 - pots[2] * RAD_PER_POT2;
  offset3 = nstang3 - pots[3] * RAD_PER_POT3;
  printf ("Nest offset values: o1=%f, o2=%f, o3=%f\n", offset1, offset2,
          offset3);
  printf ("Pot values for offset: p1= %d, p2= %d, p3= %d\n", pots[1],
pots[2],
        pots[3]);
}
/*******************
```

PATH.C                                              851213, 850919

```
******************/

/*
    This routine determines intermediate points along a straight line
    path from the present position to any preprogrammed position
*/


#include "stdio.h"
#include "defines.h"
#include "structs.h"
#include "extrnvar.h"



path()
{
/* golbals used :

                  prepropos
                  onpath
                  pots[]
                  MOVE
                  RAD_PER_POT1,2,3
                  offset1,2,3
                  set[].pos
                  set[].mode        */


double angle1,angle2,angle3,x1,y1,x2,y2,x,y,slope,intercept,step ;

char dummy ;

int bad,moving,i ;
x=y=x1=y1=x2=y2=0.0 ;
step = 100 ;
moving = 0 ;
for ( i = 1 ; i < 4 ; ++i )
   {
   if ( set[i].mode == 'm'  )
       moving = 1 ;
   }
```

```
if ( moving != 1 )

  {

  /*

                                IF NOT MOVING AND PATH=1

                                     THEN HAVE REACHED TEMPORARY POINT

                                OR

                                IF NOT MOVING AND PATH!=1

                                     THEN FIRST CALL


                                EITHER WAY CALCULATE INTERMEDIATE POINT

  */

  lockup(TRUE);

  onpath = 1 ;      /* indicate arm is on a path */

                    /* get present position of arm */

  angle1 = pots[1] * RAD_PER_POT1 + offset1 ;

  angle2 = pots[2] * RAD_PER_POT2 + offset2 ;

  angle3 = pots[3] * RAD_PER_POT3 + offset3 ;

                    /* get present (x,y) position */

  bad = arm2world( &x1, &y1, angle1, angle2, angle3 ) ;

                    /* get (x,y) of desired programmed position */

  angle1 = memory[prepropos][1] * RAD_PER_POT1 + offset1 ;

  angle2 = memory[prepropos][2] * RAD_PER_POT2 + offset2 ;

  angle3 = memory[prepropos][3] * RAD_PER_POT3 + offset3 ;

  bad = arm2world( &x2, &y2, angle1, angle2, angle3 ) ;


                    /* calculate straight line path */

  slope = (y2 - y1) / (x2 - x1) ;

  intercept = y2 - (slope * x2) ;

                    /* determine whether to step x or y */

  if ( (slope > -1) && (slope < 1) )

      {

      if ( x2 > x1 )

        {

          x = x1 + step ;

          if ( x >= x2 )

            {

              x = x2 ;

              onpath = 0 ;

            }
```

```c
                y = (slope * x) + intercept ;
              }
          else if ( x1 >= x2 )
                {
                  x = x1 - step ;
                  if ( x <= x2 )
                    {
                      x = x2 ;
                      onpath = 0 ;
                    }
                  y = (slope * x) + intercept ;
                }
          }
      else if ( (slope < -1) || (slope > 1) )
          {
           if ( y2 > y1 )
             {
               y = y1 + step ;
               if ( y >= y2 )
                 {
                   y = y2 ;
                   onpath = 0 ;
                 }
               x = (y-intercept) / slope ;
             }
          else if ( y1 >= y2 )
                {
                  y = y1 - step ;
                  if ( y <= y2 )
                    {
                      y = y2 ;
                      onpath = 0 ;
                    }
                  x = (y-intercept) / slope ;
                }
          }

                /* find angles required */
bad = world2arm( &angle1, &angle2, &angle3, x, y ) ;
lockup(FALSE);
```

```
    if ( bad != -1 )
```

```
      (
       for (i=1; i<4; i++)
         set[i].mode = MOVE;          .
       set[1].pos = ( angle1 - offset1 ) / RAD_PER_POT1 ;
       set[2].pos = ( angle2 - offset2 ) / RAD_PER_POT2 ;
       set[3].pos = ( angle3 - offset3 ) / RAD_PER_POT3 ;
      )
    else
      (
       printf(" *** Path requires arm to leave allowed workspace\n") ;
       printf(" *** Please use pendant to move arm. Thank you. \n");
       onpath = 0 ;
      )
  )
)
/*******************
   PROCESS.C                          860123
 ******************/


/*
     This subroutine makes it look like the Arthrobot is being
     controlled by distributed processing.  On each iteration of the main
loop,
     this procedure performs in serial the tasks which the joint controllers
     would normally perform in parallel.
*/



#include   "stdio.h"

#include   "defines.h"
#include   "structs.h"
#include   "extrnvar.h"

#define TIMEOUT 0

process ()
  (
```

```
int i, pitchmode;
int moving;
static int dntimer = 0;



 if (onpath)
   path() ;

.

pitchmode = set[5].mode;     /* record the existing pitch mode */
moving = 0;                  /* flag to show if arm is is motion */
for (i=0; i<=6; ++i)         /* forward order so that shoulder acts first */
  {
  if (set[i].mode == MOVE)
    {
    moving = 1;
    if ( set[5].mode != FREE )
      set[5].mode = TRACK;    /*if arm moving, do pitch tracking  */
    }
  switch (parms[i].type)     /* decide on actuator type */
    {
      case HYDRO:
        hydro(i);
        break;
      case BRIDGES:
        bridges(i);
        break;
    }
  }

outbits |= WDON;
dwrport(8,outbits);
set[5].mode = pitchmode;       /* set pitch back to normal */
if (moving)
  ctime = 200;
if ((!moving)&& (cycler))
  {
  if (ctime)
     --ctime;
  else
```

```
    {
    ++cycle_position;
    if (cycle_position > 4)
       cycle_position = 0;          .
    recall (cycle_position+49);  /*recall uses ascii codes */
    }
  }
for (i=0; i<TIMERS; ++i)     /* now increment the duty timers */
  {
  ++timer[i];
  if (timer[i] > PERIOD)    /* make it cyclic */
    timer[i] = 0;
  }
}
/*******************
   RECALL.C                              851213
 *****************/


/*
    This routine recalls the scaled positions from memory and sets the
desired
    positions to these positions. The routine path is called to give points
    along a path towards the new desired position. The routine setspeed is
    also called.
*/



#include  "stdio.h"
#include  "defines.h"
#include  "structs.h"
#include  "extrnvar.h"

recall (code)
int code ;


{
  int pose, i ;

  pose = code - 49 ;
```

```
    if (memory[pose][0] != 9999)    /* check if arm is scaled and programmed */
      {
        for (i=0; i<7; ++i)
          {
            set[i].pos = memory[pose][i] ;
            if ( (!pathon) && (i<4) )
              set[i].mode = MOVE ;
          }
        set[5].pos = state[5].pot;              /*pitch is always naturalized */
        prepropos = pose;                       /*set global position indicator */
        if (pathon)
          path() ;
        setspeed() ;
      }
    else
      {
      clearscreen(23,24) ;
      movecursor(24,0) ;
      printf ("\tPOSITION NOT TRAINED") ;
      }
  }
/********************
    REVERSE.C                                   851203, 851031, 850828
  *****************/


/*
    This routine takes a world coordinate (x,y) in millimeters and computes
    the necessary joint angles (a1,a2,a3) in radians.
*/



#include   "stdio.h"

#include   "defines.h"
#include   "structs.h"
#include   "extrnvar.h"
```

```
world2arm(a1,a2,a3,x,y)
double *a1,*a2,*a3,x,y ;


{
extern double acos(),sin(),sqrt();
int okay,region ;
double len0,len1,len2,len3,pi,temp_a1,temp_a2,temp_a3;
double len12,len22,len32,ang_hys,hys ;
double tempx,r1,r12,r2,r22,cor_ang,xnest,ynest,a1nest,a2nest,a3nest ;
double  org_x,org_x2,y2,hyp_len,side3,side32,ang_b1,ang_b2 ;
double  org_x1,org_y1,hyp_len2 ;


len0    = 370.00 ;
len1    = 152.40 ;
len2    = 304.80 ;
len3    = 374.00 ;
pi      = 3.141592654 ;
len12   = len1 *len1 ;
len22   = len2 *len2 ;
len32   = len3* len3 ;
okay    = 1 ;
region = 0 ;
ang_hys = 2.0 ;
tempx = 55.2 ;            /* xnest - len0 */
r1 = 220.9077862 ;             /* sqrt(tempx*tempx+ynest*ynest) */
r12 = r1 * r1 ;
r2 = 369.1324618 ;            /*len1 + sqrt(len33-len22) */
r22 = r2 * r2 ;
cor_ang = 0.252554276 ;         /* acos(ynest/r1) */
xnest =   425.2 ;
ynest = -213.9 ;
a1nest = -pi/2.0 ;
a2nest = -pi/2.0 ;
a3nest = 2.972392682 ;


org_x = x - len0 ;
org_x2 = org_x * org_x ;
y2 = y * y ;
if ( ((org_x2 + y2) <= r12) && (y<=0)  )
```

```
     {
       /*  REGION A */
       region = 1 ;
       hyp_len =  sqrt( org_x2 + y2 ). ;
       temp_a1 = ((-1) * acos( org_x / hyp_len )) - cor_ang ;
       temp_a2 = a2nest ;
       temp_a3 = a3nest ;
     . }
 else if ( ((org_x2 +y2) <= r22) && (y<=0) )
   {
       /* REGION B */
       region = 2 ;
       hyp_len =  sqrt( org_x2 + y2 ) ;
       hyp_len2 = hyp_len * hyp_len ;
       temp_a1 = ((-1) * acos( org_x / hyp_len )) - cor_ang ;
       org_x1 = org_x - ( len1 * cos(temp_a1) ) ;
       org_y1 = y - ( len1 * sin(temp_a1) ) ;
       side32 =  (org_x1 * org_x1) + (org_y1 * org_y1) ;
       side3 = sqrt(side32) ;
       ang_b1 = acos( (len12+side32-hyp_len2)/(2*len1*side3) ) ;
       ang_b2 = pi - ang_b1 ;
       temp_a2 = (-1)*(acos((side32+len22-len32)/(2*len2*side3)) - ang_b2);
       temp_a3 = pi - acos( (len22 + len32 - side32) / (2 * len2 *len3) ) ;
   }
 else if ( ((org_x2 + y2) > r22) && (y<=0) )
   {
       /* REGION C */
       region = 3 ;
       hyp_len =  sqrt( org_x2 + y2 ) ;
       temp_a1 =  (-1) * acos( org_x / hyp_len ) ;
       side3 =  hyp_len - len1 ;
       side32 =  side3 * side3 ;
       temp_a2 =  (-1) * acos( (len22 + side32 - len32) / (2 * len2 * side3) ) ;
       temp_a3 =  pi - acos( (len22 + len32 - side32) / (2 * len2 *len3) ) ;
   }
 else if ( ((org_x2 + y2) > r22) && (y>0) )
   {
       /* REGION D */
       region = 4 ;
```

```
    temp_a1 = 0.0 ;
    org_x =  x - len0 - len1 ;
    side3 =  sqrt( (org_x * org_x) + y2 ) ;
    side32 =  side3 * side3 ;         .
    ang_b1  = acos( org_x / side3 ) ;
    temp_a2 = ang_b1  - acos( (len22+side32-len32)/(2*len2*side3) ) ;
    temp_a3 = pi -      acos( (len22+len32-side32)/(2*len2*len3) ) ;
  . }
else okay = 0 ;


if ( okay == 1 )
  {
   hys =  ( ang_hys / 180.0) * pi ;


   if (temp_a1 > 0.0 )
     if ( (temp_a1 - hys) <= 0 )   temp_a1 = 0.0 ;
     else okay = 0 ;
   if (temp_a1 < (-1*pi/2) )
     if ( (temp_a1 + hys) >= -pi/2 ) temp_a1 = -pi/2 ;
     else okay = 0;
   if (temp_a2 > 0.0 )                      /* joint safety check */
     if ( (temp_a2 - hys) <= 0 )   temp_a2 = 0.0 ;
     else okay = 0 ;
   if (temp_a2 < (-1*pi/2) )
     if ( (temp_a2 + hys) >= -pi/2 ) temp_a2 = -pi/2 ;
     else okay = 0;
   if (temp_a3 > a3nest )                   /* joint safety check */
     if ( (temp_a3 - hys) <= a3nest)   temp_a3 = a3nest ;
     else okay = 0 ;
   if (temp_a3 < (a3nest - (pi/2)) )
     if ( (temp_a3 + hys) >= (a3nest-(pi/2)) )  temp_a3 = a3nest-(pi/2) ;
     else okay = 0;
  }
if ( okay == 1 )
  {
    *a1 = temp_a1 ;
    *a2 = temp_a2 ;
    *a3 = temp_a3 ;
    return(0);
```

```
    }
else return(-1);
}
/*******************
   REV_LEG.C                                    860129, 851114
 *****************/


/*
     This routine finds the arm joint angles a0,a1,...a6 given
     the desired hip elevation and knee flex angles.
*/



#include   "stdio.h"

#include   "defines.h"
#include   "structs.h"
#include   "extrnvar.h"



rev_leg(a0,a1,a2,a3,a4,a5,a6,elevt,flexx)
double *a0,*a1,*a2,*a3,*a4,*a5,*a6,elevt,flexx ;


{
extern double sin(),cos(),acos() ;
double elv,flx,u,v,x,y,ul,ll,pi,d ;
int bad ;
double ang0,ang1,ang2,ang3,ang4,ang5,ang6 ;
                            /* a0        shoulder swing
                               a1        joint #1
                               a2        joint #2
                               a3        joint #3
                               a4        yaw
                               a5  ·     pitch
                               a6        roll

                               WIDTH     PAP WIDTH
                               DEPTH     PAP DEPTH
```

179

```
                        ul         upper leg length
                        ll         lower leg length
                        leg        total leg length
                        shiftx     horizontal shoulder to arm
                        shifty     vertical shoulder to arm
                        x,y        arm coordinates of pitch axis
                        u,v        leg coordinates of pitch axis
                    */

                    /* calculate upper and lower leg lengths */
pi = 3.141592654 ;
ul = leg / 2.0 ;
ll = ul ;
u = v = x = y = 0.0 ;
ang0 = 0.00 ;              /* shoulder swing stays at present value */
ang4 = 0.0 ;              /* planar routine so yaw = 0.0 rads */
ang6 = 0.0 ;              /* planar routine so roll = 0.0 rads */


                    /* convert elevate and flex into rads */
elv = elevt ;
flx = flexx ;
                    /* calculate u,v  */
u  = ul * cos(elv) ;
u += ll * cos(elv) * cos(flx)  -  ll * sin(elv) * sin(flx) ;
u += (-1) * ( DEPTH * cos((elv+flx))  -  WIDTH * sin((elv+flx)) ) ;

v  = ul * sin(elv) ;
v += ll * sin(elv) * cos(flx)  +  ll * cos(elv) * sin(flx) ;
v += (-1) * ( DEPTH * sin((elv+flx))  +  WIDTH * cos((elv+flx)) ) ;


                    /* calculate x,y */
x = u + shiftx ;
y = v + shifty ;



                    /* call reverse transform routine for arm joints */
bad = world2arm(&ang1,&ang2,&ang3,x,y) ;


if ( bad != -1 )
```

```
    {
                            /* calculate pitch angle */

       ang5 = elv + flx + (pi/2) - ang1 - ang2 - ang3 ;
                            /* return arm angles */

       *a0 = ang0 ;

       *a1 = ang1 ;

       *a2 = ang2 ;

       *a3 = ang3 ;

       *a4 = ang4 ;

       *a5 = ang5 ;

       *a6 = ang6 ;

       }

   else   arout( 33  );  /* Speak "LIMIT" */

}
```

```
/******************

  SAFE.C                                    851203

 ******************/
```

```
/*
    Safe tests the given x and y positions to see if they are within
    the safe workspace of the patient's leg.  This is determied by the
    patient leg data calculated in scale.c, and indicated by a return of
    TRUE for safe positions, and FALSE for unsafe ones.
*/



#include   "stdio.h"

#include   "defines.h"
#include   "structs.h"
#include   "extrnvar.h"



safe(x,y)
double x,y;


{
double tempa, tempb;
```

```
if (safety == FALSE)
  return (TRUE);   /*if no scaling done, skip */


if (y >= 0)
  {
  tempa = (x-shiftx)*(x-shiftx) + (y-shifty)*(y-shifty);
  tempb = (leg-GRIP_DEPTH)*(leg-GRIP_DEPTH);
  if (tempa > tempb)
    return (FALSE);
  else
    return (TRUE);
  }
else
  {
  tempa = (x-shiftx-leg/2.0)*(x-shiftx-leg/2.0)
              + (y)*(y);
  tempb = (leg/2.0-GRIP_DEPTH)*(leg/2.0-GRIP_DEPTH);
  if (tempa > tempb)
    return (FALSE);
  else
    return (TRUE);
  }
}
/******************
   SCALE.C                              860123, 850918
 *****************/

/*
    This routine scales the five preprogrammed positions according
    to the patient's leg length, which leg (left or right), and
    positioning of the arthrobot relative to the patient.
    globals used :
        side       1 = left  -1 = right
        offset1, offset2, offset3
        RAD_PER_POT1, RAD_PER_POT2, RAD_PER_POT3
        memory [pose][pot]    pose 0 to 4    pot 0 to 6
        leg, shiftx, shifty;
*/
```

```c
#include  "stdio.h"

#include  "defines.h"
#include  "structs.h"
#include  "extrnvar.h"


scale()
 {
extern double cos(),sin();

#define PI    3.141592654
#define YPOSITION  100.00      /* arm to leg height */
#define LEN0   370.0          /* length of first arm segment */
#define GRIP_WIDTH    125.0   /* width of patient applied part */
#define YAW_SWING 789.0
#define ROLL_SWING 200.0

double u,v,x,y,angle1,angle2,angle3;
double tempx,tempy ;
int shldr_pos,yaw,roll,pitch,position,bad ;

char dummy[15] ;

  lockup(TRUE) ;
  clearscreen(0,24);
  movecursor(0,0);
  flex_ang = 90 ;
  elevate = 45 ;
  printf(" Arthoscopic Leg Positioning Scaling Routine\n\n") ;
  printf("Is leg positioned in FULL EXTENSION position? (y/n) :") ;
  dummy[0] = bdos(1);
  printf("\n");
  if (dummy[0] != 'y')
      {
       clearscreen(0,24);
```

```
            movecursor(0,0);
            mainmenu();
            lockup(FALSE);
            }
    else
        {
        printf(" \n\n Please enter heel to knee length in mm :");
        scanf("%F\n",&leg) ;
        printf("\n");
        trained = TRUE;
        safety = TRUE ;
        leg = leg * 2.0 ;
        angle1 = pots[1] * RAD_PER_POT1 + offset1 ;
        angle2 = pots[2] * RAD_PER_POT2 + offset2 ;
        angle3 = pots[3] * RAD_PER_POT3 + offset3 ;
        arm2world( &x, &y, angle1, angle2, angle3 ) ;
        tempx = x ;
        tempy = y ;

        printf("leg length is : %4.2f\n",leg);
        shiftx = ( x + GRIP_DEPTH ) - (leg);
        shifty = YPOSITION ;

        hx = shiftx;      /* these values used by anthro routine */
        hy = shifty;
        z0 = sqrt((leg/2-GRIP_DEPTH)*(leg/2-
GRIP_DEPTH)+(GRIP_WIDTH*GRIP_WIDTH));
        minq = leg*(GRIP_WIDTH) / (2*(leg-GRIP_DEPTH));

        printf(" shiftx = %f    shifty = %f   z0 = %f\n",shiftx,shifty, z0);
        if ( side == -1 )            /* right side */
          shldr_pos = 3289 ;
        else                       /* left side */
          shldr_pos = 740 ;
        for ( position = 1 ;  position < 6 ;  ++position )
            {
            yaw = yaw_center ;       /*  0.0 degrees, centered */
            pitch = pitch_center ;    /*  0.0 degrees, centered */
            roll= roll_center ;       /*  0.0 degrees, centered */
```

```
       switch ( position )
         {
      case 1 :                    /* elevate position */
              do              .
               {
              u = (leg-GRIP_DEPTH) * cos( elevate * PI / 180.0 ) ;
              u = u + ( GRIP_WIDTH * sin(elevate * PI /180.0) ) ;
              v = (leg-GRIP_DEPTH) * sin( elevate * PI / 180.0 ) ;
              v = v - ( GRIP_WIDTH * cos(elevate * PI /180.0) ) ;
              x = u + shiftx ;
              y = v + shifty ;
              bad = world2arm( &angle1, &angle2, &angle3, x, y ) ;
              if ( bad < 0 )
                  elevate = elevate - 1 ;
               }
              while ( (bad < 0) && (elevate > 0) );
              printf("elevate = %f\n",elevate);
              roll =  pots[6]  + ( side * wrparms[12] ) ;
              yaw = yaw_center + ( side * wrparms[10] ) ;
              break ;
      case  2  :                  /* full extension */
              u = tempx - shiftx ;
              v = tempy - shifty ;
              roll =  pots[6] ;
              yaw = yaw_center;
              break ;
      case  3  :                  /* full flex */
              do
               {
          u = (leg/2.0) + ((leg/2.0)-GRIP_DEPTH) *
cos(flex_ang*PI/180.0) ;
              u = u - ((GRIP_WIDTH) * sin(flex_ang*PI/180.0)) ;
              v = (-1) *       ((leg/2.0)-GRIP_DEPTH) *
sin(flex_ang*PI/180.0) ;
              v = v - ((GRIP_WIDTH) * cos(flex_ang*PI/180.0)) ;
                x = u + shiftx ;
                y = v + shifty ;
                bad = world2arm( &angle1, &angle2, &angle3, x, y ) ;
                if ( bad < 0 )
```

```
                flex_ang - flex_ang - 1 ;
              )
            while ( (bad < 0) && (flex_ang > 0) );
            printf("flex_ang - %f\n",flex_ang);
            roll - roll_center ;
            yaw - yaw_center + ( side * YAW_SWING / 4 ) ;
            break ;
      case  4  :                  /* half flex */
            u - ( 0.80 * leg ) - GRIP_DEPTH ;
            v - GRIP_WIDTH - leg / 2.3 ;
            roll - roll_center + ( side * abs(pots[6] - roll_center));
            yaw - yaw_center + 0.5 * ( side * abs(pots[6] -
roll_center) );
            break ;
      case  5  :
            u - (0.800 * leg ) - GRIP_DEPTH ;
            v - -GRIP_WIDTH  ;
            if ( side -- -1 )
              (       /* right side */
               yaw - yaw_center - YAW_SWING ;
               roll - roll_center + ROLL_SWING ;
               )
              else   /* left side */
                (
                 yaw - yaw_center + YAW_SWING ;
                 roll - roll_center - ROLL_SWING ;
                 )
            break ;
      )
    /* translate (u,v) to (x,y) */
    x - u + shiftx ;
    y - v + shifty ;
    /* calculate angles for joints #1 #2 #3 */
    bad - world2arm( &angle1, &angle2, &angle3, x, y ) ;
    /* convert angles to pot values */
    if ( bad !- -1 )
      (
        printf("Position %d programmed \n",position) ;
        printf("     x- %f          y- %f \n",x,y);
```

```c
            printf("\n");
            memory [position - 1] [0] = shldr_pos ;
            memory [position - 1] [1] = (angle1 - offset1) / RAD_PER_POT1 ;
            memory [position - 1] [2] = (angle2 - offset2) / RAD_PER_POT2 ;
            memory [position - 1] [3] = (angle3 - offset3) / RAD_PER_POT3 ;
            memory [position - 1] [4] = yaw ;
            memory [position - 1] [5] = pitch ;
            memory [position - 1] [6] = roll ;
            }
        else
            {
            printf("  ** Position  %d not programmed \n",position) ;
            printf(" ** Please do position %d manually **  \n",position) ;
            printf("      x= %f           y= %f \n",x,y);
            printf("\n");
            }
        }
    lockup(FALSE);
    prepropos = 1;
    mainmenu();
    }
}
/*******************
    SELACT.C                                    860205, 851109
*****************/


/*
    This file contains the routines to do specific actions
    that were commanded via the new hand control units
*/




#include  "stdio.h"

#include  "defines.h"
#include  "structs.h"
#include  "extrnvar.h"
```

```
iabs( value )
  int  value;
  {
      if( value  <  0 )
                return( -value );
        else    return( value );
  }




speak(   sar10_word_number )
  int  sar10_word_number;
  {

    if( are_we_in_continuous_motion  ==  NO  )
            return(   arout(  sar10_word_number  )  );
     else   return( 1 );
  }




set_the_reject_buzzer_flag( setting )
  int  setting;
  {
    unsigned char  received_junk[ 128 ];


    send_command_to_the_SAR10( " ",  received_junk );   /* Stop Recog */


    if( setting == TRUE )
        {
          send_command_to_the_SAR10( "B,1\r",  received_junk );
```

```
          send_command_to_the_SAR10( "M1\r", received_junk ); /* Restart
recog */
            )

      else
         (
         send_command_to_the_SAR10( "B,0\r", received_junk );
         send_command_to_the_SAR10( "M0\r", received_junk ); /* Restart
recog */
            )


    )



stuff_selection_or_action_into_the_keyboard_buffer( pendant_selection,
pendant_action )
  int  pendant_selection, pendant_action;
  (

     if( pendant_action == 0 )
       switch( pendant_selection )
          (

            case 0 :
                    stuff_kbuf( ELEVATE_COMMAND );
                    break;
            case 1 :
                    stuff_kbuf( FULL_EXTENSION_COMMAND );
                    break;
            case 2 :
                    stuff_kbuf( HALF_FLEX_COMMAND );
                    break;
            case 3 :
                    stuff_kbuf( FULL_FLEX_COMMAND );
                    break;
            case 4 :
                    stuff_kbuf( FIGURE_4_COMMAND );
                    break;
```

```
    case 5 :

            stuff_kbuf(  WRIST_COMMAND  );
            break;
    case 6 :

            stuff_kbuf(  FOOT_COMMAND  );
            break;
    case 7 :

            stuff_kbuf(  KNEE_COMMAND  );
            break;
    case 8 :

            stuff_kbuf(  HIP_COMMAND  );
            break;
    case 9 :

            stuff_kbuf(  ARM_COMMAND  );
            break;
    default:

            break;


    }


else

  switch( pendant_action  )

    {

      case 1 :

              stuff_kbuf(  MINUS_COMMAND  );
              break;


      case 2 :

              stuff_kbuf(  PLUS_COMMAND  );
              break;


      case 3 :

              stuff_kbuf(  STOP_COMMAND  );
              break;


      }
```

```
)

stuff_kbuf(  a_character  )
 unsigned int  a_character;
 {

    static  unsigned int  ASCII_to_scan_code_and_key[ 128 ] =
      {
        0x0300, 0x1E01, 0x3002, 0x2E03, 0x2004, 0x1205, 0x2106, 0x2207,
        0x2308, 0x1709, 0x240A, 0x250B, 0x260C, 0x320D, 0x310E, 0x180F,
        0x1910, 0x1011, 0x1312, 0x1F13, 0x1414, 0x1615, 0x2F16, 0x1117,
        0x2D18, 0x1519, 0x2C1A, 0x1A1B, 0x2B1C, 0x1B1D, 0x071E, 0x0C1F,
        0x3920, 0x0221, 0x2822, 0x0423, 0x0524, 0x0625, 0x0826, 0x2827,
        0x0A28, 0x0B29, 0x092A, 0x0D2B, 0x332C, 0x0C2D, 0x342E, 0x352F,
        0x0B30, 0x0231, 0x0332, 0x0433, 0x0534, 0x0635, 0x0736, 0x0837,
        0x0938, 0x0A39, 0x273A, 0x273B, 0x333C, 0x0D3D, 0x343E, 0x353F,
        0x0340, 0x1E41, 0x3042, 0x2E43, 0x2044, 0x1245, 0x2146, 0x2247,
        0x2348, 0x1749, 0x244A, 0x254B, 0x264C, 0x324D, 0x314E, 0x184F,
        0x1950, 0x1051, 0x1352, 0x1F53, 0x1454, 0x1655, 0x2F56, 0x1157,
        0x2D58, 0x1559, 0x2C5A, 0x1A5B, 0x2B5C, 0x1B5D, 0x075E, 0x0C5F,
        0x2960, 0x1E61, 0x3062, 0x2E63, 0x2064, 0x1265, 0x2166, 0x2267,
        0x2368, 0x1769, 0x246A, 0x256B, 0x266C, 0x326D, 0x316E, 0x186F,
        0x1970, 0x1071, 0x1372, 0x1F73, 0x1474, 0x1675, 0x2F76, 0x1177,
        0x2D78, 0x1579, 0x2C7A, 0x1A7B, 0x2B7C, 0x1B7D, 0x297E, 0x0E7F
      };

    unsigned int  head,  tail,  place;



    disable_interrupts();

    head = peek( 0x001a, 0x0040 );
    tail = peek( 0x001c, 0x0040 );
    place = tail;
```

```
     tail  += 2;
     if( tail  ==  0x003e  )
        tail -  0x001e;


     if( head  == tail  )
        return( 0 );


     if( ( a_character & 0x00ff )  == 0x0000  )
            pokew( place,  0x0040,  a_character  );
        else  pokew( place,  0x0040,  ASCII_to_scan_code_and_key( a_character
]   );



     pokew( 0x001c, 0x0040,  tail                  );
     return( 1 );


     enable_interrupts();



  }




flush_the_keyboard_buffer()
  {
     unsigned int  head;

/*
     The keyboard buffer has a "head" where characters are read from
     and a "tail" where characters are placed.  This routine forces the
     "tail" back to the "head" effectively flushing the keyboard buffer.
     Note:  0x0040:0x001a  contains the head position ( in segment 0x0040 )
            0x0040:0x001c  contains the tail position ( in segment 0x0040 )
*/

     disable_interrupts();
```

```
head = peek( 0x001a, 0x0040 );      /*  Get the head        */
pokew( 0x001c, 0x0040, head );      /*  Put it into the tail  */

enable_interrupts();

}




is_the_arm_moving()
  {
    int  jointt,  moving;



    moving = FALSE;

    for(  jointt = 0;   jointt < 4;   jointt++   )
      if(   abs( set[ jointt ].pos - state[ jointt ].pot )   >   parms[
jointt ].f_hys  )
         {
           moving = TRUE;
           break;
         }

    for(  jointt = 4;   jointt < 7;   jointt++   )
      if(   abs( set[ jointt ].pos - state[ jointt ].pot )   >   30   )
       {
         moving = TRUE;
         break;
       }

    return(  moving  );



  }
```

```
put_the_arm_into_emergency_stop()
   {

      dwrport (8, outbits & 0xE000);          /* Shut it all off!!!!!! */
      snapshot();                             /* Get up to date values */
      for (joint=0; joint<7; ++joint)
        {
          set[joint].pos = state[joint].pot;
          if(   joint < 4   )
            set[joint].mode = LOCK ;
          else
            set[joint].mode = FREE;
        }


   }




stop_and_maintain_the_present_position_of_the_arm()
   {

      dwrport(  8,   outbits & 0xe000  );
      snapshot();
      for(  joint = 0;   joint < 7;   joint++  )
        {
          set[ joint ].mode =  HOLD;
          set[ joint ].pos  =  state[ joint ].pot;
        }


   }
```

```
elevate_the_arm()
   {
        recall( '1' );
        wrparms[ 18 ] =  TRUE;
   }



full_extension_the_arm()
   {
        recall( '2' );
   }



half_flex_the_arm()
   {
        recall( '4' );
   }



full_flex_the_arm()
   {
        recall( '3' );
   }



figure_4_the_arm()
   {
        recall( '5' );
   }



setup_the_arm()
   {
        recall( '6' );
```

```
)

move_arm_in()
   {
        set[ 0 ].pos  -=  ( shoulder * parms[ 0 ].inc * 2 );
        set[ 0 ].mode  =  MOVE;
   }



move_arm_out()
   {
        set[ 0 ].pos  +=  ( shoulder * parms[ 0 ].inc * 2 );
        set[ 0 ].mode  =  MOVE;
   }




flex_the_hip()
   {
   if (trained)
        anatom (ANG_STEP*2.0, 0.0);
   else
        inc_xy (0 , 50);
   }



extend_the_hip()
   {
   if (trained)
        anatom (-ANG_STEP*2.0, 0.0);
   else
        inc_xy (0 ,-50);
   }



flex_the_knee()
```

```
     {
     if (trained)
          anatom (0.0, -ANG_STEP*2.0);
     else
          inc_xy (-50, 0);
     }



extend_the_knee()
     {
     if (trained)
          anatom (0.0,  ANG_STEP*2.0);
     else
          inc_xy (50, 0);
     }




rotate_foot_in()
     {
     set[4].pos -= (side*parms[4].inc);
     }




rotate_foot_out()
     {
     set[4].pos += (side*parms[4].inc);
     if (external_rotation == 0)
        {
        external_rotation = 1;   /* flag reset by wrist free or snapshot */
        set[6].pos += (side*parms[6].inc);
        }
     }




release_the_wrist()
     {
          for( joint = 4;   joint < 7;   joint++   )
            set[ joint ].mode  =  FREE;
```

0201883

```
        external_rotation - 0;
   }



hold_the_wrist()
   {
        for(  joint - 0;    joint < 7;    joint++    )
          {
            set[ joint ].mode   -   HOLD;
            set[ joint ].pos    -   state[ joint ].pot;
          }
   }
/*******************
   SETSPEED.C                         851203
 *****************/


/*
     This routine sets the speed for the hydraulic joints
*/



#include   "stdio.h"

#include   "defines.h"
#include   "structs.h"
#include   "extrnvar.h"



setspeed()

{
  double delta1, delta2, delta3, temp;
  int speed1, speed2, speed3;

  delta1 - abs(set[1].pos - pots[1]);
  delta2 - abs(set[2].pos - pots[2]);
  delta3 - abs(set[3].pos - pots[3]);
  if (delta1>delta2)
    {
```

```
      if (delta1>delta3)
        temp = delta1;
      else
        temp = delta3;
      }
    else
      {
    . if (delta2>delta3)
        temp = delta2;
      else
        temp = delta3;
      }
    speed1 = PERIOD * delta1/temp;
    speed2 = PERIOD * delta2/temp;
    speed3 = PERIOD * delta3/temp;
    if (speed1 < 1)                              /* make sure speed > 0 */
      speed1 = 1;
    if (speed2 < 1)
      speed2 = 1;
    if (speed3 < 1)
      speed3 = 1;
    set[1].duty = speed1;
    set[2].duty = speed2;
    set[3].duty = speed3;
}
/********************

    SETUP.C                              860207

 *******************/


/*

    This routine initializes the joint settings and I/O ports.  It also

    sets up the parameter array for the simulated distributed processing

    routines.

*/




#include   "stdio.h"


#include   "defines.h"
```

```
#include  "structs.h"
#include  "extrnvar.h"



setup ()
  (

  extern  int  dac_refresh();

  int i;
  char reply[10] ;

  debug = 0;     /*turn off debugger */
  /* set up joint parameters */
  parms[0].type = HYDRO;              /* horizontal shoulder joint */
    parms[0].f_hys = 15;
    parms[0].r_hys = -15;
    parms[0].clock = 0;
    parms[0].outadr = 0;
    parms[0].vmask = 0x0004;
    parms[0].xgain = 1;
    parms[0].vgain = 1;
    parms[0].dead = 2;
    parms[0].inc = 15;
  parms[1].type = HYDRO;              /* vertical shoulder joint */
    parms[1].f_hys = 30;
    parms[1].r_hys = -30;
    parms[1].clock = 1;
    parms[1].outadr = 1;
    parms[1].vmask = 0x0020;
    parms[1].xgain = 1;
    parms[1].vgain = 1;
    parms[1].dead = 2;
    parms[1].inc = 10;
  parms[2].type = HYDRO;              /* first elbow joint */
    parms[2].f_hys = 30;
    parms[2].r_hys = -30;
    parms[2].clock = 2;
    parms[2].outadr = 2;
```

```
    parms[2].vmask = 0x0100;
    parms[2].xgain = 1;
    parms[2].vgain = 1;
    parms[2].dead = 2;
    parms[2].inc = 10;
  parms[3].type = HYDRO;                /* second elbow joint */
    parms[3].f_hys = 35;
    parms[3].r_hys = -35;
    parms[3].clock = 3;
    parms[3].outadr = 3;
    parms[3].vmask = 0x0800;
    parms[3].xgain = 1;
    parms[3].vgain = 1;
    parms[3].dead = 2;
    parms[3].inc = 10;


/* note: wrist parms have been redefined as noted below  */


  /* wrist parameters needed for ctrl */
    wrparms[0] = 1;    /* wrist mode 1=normal 2=lockyaw&pitch 3=pitchtrack */


    wrparms[1] = 0;    /* v offset for valves */
    wrparms[2] = 6;     /* Pitch window    */
    wrparms[3] = 4;     /* overshoot constant */
    wrparms[4] = 1;     /* pitch velocity scaling factor */
    wrparms[5] = 50;    /* count down timer initialization (# of cycles) */


    wrparms[10] = -150;  /* yaw offset const, used by scale.c for elevate */
    wrparms[11] = 120;   /* pitch increment when elevate is reached */
    wrparms[12] = 100;   /* roll elevate offset used by scale.c   */


    wrparms[13] =  8;  /* scaling factor motors to DACs;  8 for 512 levels */
    wrparms[14] = 4095 / wrparms[13];   /* complementary constant */


    wrparms[15] = 2050;   /* pitch tracking offset constant */
    wrparms[16] = 2050;   /* roll tracking offset constant  */


    wrparms[18] = FALSE;  /* flag that is set when elevate is recalled */
    wrparms[19] = 1;   /* variable for pitch velocity */
```

```
parms[4].type = BRIDGES;                /* wrist yaw (pitch)  */
  parms[4].f_hys = 0;            /* hysteris window for wrist ctrl      */
  parms[4].r_hys = 200;     /* actually the velocity limiting term */
  parms[4].outadr = 0;
  parms[4].vmask = 0x2000;
  parms[4].xgain = 25;
, parms[4].vgain = 25;          /* velocity feedback gain  */
  parms[4].dead = 2048;
  parms[4].inc = 100;
  parms[4].msh =  2048;         /* mush mode constant (650)  */
parms[5].type = BRIDGES;                /* wrist pitch (yaw)  */
  parms[5].f_hys = 0;
  parms[5].r_hys = 200;
  parms[5].outadr = 1;
  parms[5].vmask = 0x4000;
  parms[5].xgain = 25;
  parms[5].vgain = 25;
  parms[5].dead = 2048;
  parms[5].inc = 10;
  parms[5].msh =  2048;      /* 650 */
parms[6].type = BRIDGES;                /* wrist roll */
  parms[6].f_hys = 0;
  parms[6].r_hys = 200;
  parms[6].outadr = 2;
  parms[6].vmask = 0x8000;
  parms[6].xgain = 25;
  parms[6].vgain = 15;
  parms[6].dead = 0;
  parms[6].inc = 400;
  parms[6].msh = 2048;    /* 1000  */


  offset1 = -2952.0 * RAD_PER_POT1;
  offset2 = -2768.0 * RAD_PER_POT2;       /* determined experimentally */
  offset3 = 1.379748483 - 1362 * RAD_PER_POT3;
  offset5 =  - pitch_center * RAD_PER_POT5 ;


  memory [5][0] = 740 ;
  memory [5][1] = 2810 ;
```

```
        memory [5][2] = 1822 ;

        memory [5][3] = 1864 ;

        memory [5][4] = yaw_center ;

        memory [5][5] = 1395 ;              .

        memory [5][6] = roll_center ;




        .




        dtreset();                          /* Reset digital and analog I/O ports */

        setclock(120);

        setport(8);

        dwrport(8,0);


        for (i=0; i<7; ++i)                 /* Set initial conditions for joints */

          {

          if (i <4)

            set[i].mode = LOCK;

          else

            set[i].mode = FREE;

          set[i].duty = PERIOD;

          set[i].pos = adi (i,1,0);

          parms[i].reset = (pump[parms[i].outadr].mask | parms[i].vmask) ^ 0xFFFF;

          state[i].wait = 0;

          }


        movecursor (0,29);

        printf( "ARTHROBOT II  SETUP\n");

        movecursor (1,22);

        printf( "Copyright 1986 Andronic Devices Ltd \n\n");

        printf ("Operating on left or right leg?  ( Enter l or r ): "  );

        scanf ("%s\n", &reply[0]);

        if (reply[0] == 'r')

          {

          parms[0].xgain = -1;

          side = -1;

          memory [5][0] = 3289 ;
```

```
  }
  link_to_interrupt( dac_refresh,  256,  USER_SUPPLIED_CLOCK_ROUTINE_VECTOR
);
  snapshot() ;
  adjust_the_wrist_compliance( 6 );
  }
/*******************
  .SNAPSHOT.C                      860122
 *****************/


/*
    This routine samples the position feedback sensors, calculates
    position changes, and checks for pot errors.
*/



#include   "stdio.h"

#include   "defines.h"
#include   "structs.h"
#include   "extrnvar.h"



snapshot ()
{

  static  int  is_the_heel_being_pulled_out = NO,
               previous_state_of_joint_3;
  int temp[10];
  int i, j;



  for (i=0; i<10; ++i)
    temp[i]=0;
  disintr();                 /* disable interrupt processing */
  setadc (0,9,1,0,10);       /* set adc for 10 readings, chn 0-9, gain of 1 */
  for (i=0; i<10; ++i)       /* read pots on all joints */
    temp[i] = adc();
```

```
    enabintr();              /* enable interrupts again */


    for (i=0; i<7; ++i)        /* calc.pot displacement on all joints */
      {
      if (temp[i] < 10  ||  temp[i] > 4085)
        fault[i] - POTERR;
      state[i].deltax = temp[i] - state[i].pot;
      state[i].pot = temp[i];
      }


    pendsample = temp[7];     /* store last sample as pendant input */



/*
    Check the heel sensor to see if the foot is being pulled out of the
    patient applied part
*/



    if( temp[ 8 ]  <  2048  )
        {
          is_the_heel_being_pulled_out =  YES;
          if(  set[ 3 ].mode != LOCK   )
            {
              previous_state_of_joint_3  =  set[ 3 ].mode;  /* Save for later
restoration */
              set[ 3 ].mode  =  HOLD;
            }
        }
      else
        {
            if(  is_the_heel_being_pulled_out  ==  YES )
              is_the_heel_being_pulled_out  =  NO;
              set[ 3 ].mode              =  MOVE;              /*
Restore previous mode */
        }
```

**0201883**

```
/*
      Check the environment sensor to see if the arm or wrist and hitting any
      obstructions.
*/



   if( temp[ 9 ]  <  2048  )
     stop_and_maintain_the_present_position_of_the_arm();




   if (       state[0].pot  >   (SHOULDER - 50)
        &&  state[0].pot  <   (SHOULDER + 50)
        &&  set[0].mode   ==  MOVE                    )
      set[0].mode = LOCK;


   if (state[0].pot > SHOULDER)
      shoulder = -1;
   else                    /*determine actual polarity of shoulder position */
      shoulder = 1;


   if (abs(state[6].pot - yaw_center) < 100.0)   /* ie, if near yaw center */
      if (external_rotation > 0)
        {
        set[6].pos -= (side * parms[6].inc);
        external_rotation = 0;
        }




   }
/*******************
   STORE.C                          851203
  *****************/


/*
      This subroutine allows positions to be trained.  To use it, the
      operator presses "s", followed by the number of the position to be
      stored (1-5).
*/
```

```
#include   "stdio.h"


#include   "defines.h"

#include   "structs.h"

#include   "extrnvar.h"



store (code, mode)
  int code, *mode;


{

  int pose, i;


  if (*mode == 0)

    {

    lockup(TRUE);

    clearscreen (2,24);

    movecursor (2,0);

    printf ("S T O R E    P O S I T I O N\n\n\n");

    printf ("standard positions:\n\t1 - Elevate\n\t2 - Full Extension\n");

    printf ("\t3 - Full Flex\n\t4 - Half Flex\n\t5 - Figure Four\n\n");

    printf ("Press 1-5 to store position\n");

    *mode = 2;

    lockup(FALSE);

    }

  else

    {

    if (code > 48  &&  code < 54)

      {

      pose = code - 49;

      for (i=0; i<7; ++i)

        memory[pose][i] = pots[i];

      movecursor (24,0);

      printf ("\tPosition %d trained", pose+1);

      }

    *mode = 0;

    mainmenu();
```

```
    }
}
/*******************
   TIMEDAY.C                          .   860122
 ******************/


/*
    . This routine returns the numbers of clock ticks since midnite.
      ( 18.2 clock ticks/second )
*/



  unsigned long get_the_time_of_day()
  {
  unsigned long clock_ticks;
  unsigned short peek();

  disintr();
  clock_ticks = peek (0x006e, 0x0040);
  clock_ticks = clock_ticks * 0x10000L + peek (0x006c, 0x0040);
  enabintr();
  return (clock_ticks);
  }
/*******************
   WTCHDOG.C                              851213, 850830
 ******************/


/*
     This routine toggles the watch dog timer when called.
     ie. it resets the monostable controlling the digital voltage regulator.
*/



#include  "stdio.h"

#include  "defines.h"
#include  "structs.h"
#include  "extrnvar.h"
```

```
wtchdog()

{
outbits |= WDON ;
dwrport ( 8 , outbits ) ;
outbits &= WDOFF ;
dwrport ( 8 , outbits ) ;
}
#include  "stdio.h"



FILE  *fpi,  *fpo,  *fopen();

unsigned char  storage[ 256 ];
int            stop, i, length, character, ret;



srv_arv_crstrip( c, v )
  int   c;
  char *v[];
  {

  if( c < 3 )
   printf( "Count error \n" );

  fpi = fopen( v[ 1 ], "r" );
  fpo = fopen( v[ 2 ], "w" );

  do
    {
      ret = fgets( storage, 257, fpi );
      character = fgetc( fpi );

          length = strlen( storage );
          for( stop = 0, i = length - 1; stop == 0 && i > 0; i-- )
            switch(  storage[ i ] )

               {
```

```
            case ' ' :
            case '\t' :
            case '\r' :
            case '\n' :    .

                        storage[ i ] = '\0';
                        break;
            default :

                        stop = 1;
                        break;
        }


    if(     character == ' '
        ||  character == '\t'
        ||  character == '\n'
        ||  isupper( character )
        ||  isdigit( character )    )
        {
          length = strlen( storage );
          storage[ length     ] = '\n';
          storage[ length + 1 ] = '\0';


          fputs( storage,    fpo );
          fputc( character, fpo );
        }
      else
        {
          length = strlen( storage );
          storage[ length     ] = ' ';
          storage[ length + 1 ] = '\0';


          fputs( storage,    fpo );
          fputc( character, fpo );
        }


    }
  while( character != EOF && ret != EOF );



fclose( fpi );
```

```
     fclose( fpo );


   }
/*****************

   DTHEAD.H                                    851023

 ****************/



/*

     This file contains the constants and macros used by the C software

     for the DT2801 Analog I/O board

*/




/* Data Translation board, factory standard address */


#define DTCSR     0x02ed
#define DTDAT     0x02ec


/* Status bit masks */


#define ERROR     0x80
#define OBFE      0x40
#define CMND      0x08
#define READY     0x0004
#define OBF       0x01
#define IBF       0x02




#define commandwait  {while (!(inportb(DTCSR) & READY));}
#define readwait  {while (!(inportb(DTCSR) & OBF));}
#define writewait  {while (inportb(DTCSR) & IBF);}
;
;   EPILOGUE.H
;
;     end of any assembly code
```

```
@CODE ENDS
;
;   MODEL.H
;
;      define memory model for library assembly code


FALSE equ   0      ;for small model
TRUE  equ   1      ;for big model


; small model:=   @bigmodel and @medmodel are false
; medium model:=  @bigmodel is false, @medmodel is true
; big model:=             @bigmodel is true, @medmodel is false


@BIGMODEL EQU FALSE      ; select this for model desired
@MEDMODEL EQU FALSE      ; medium model switch


ifl
if      @bigmodel
%out  BIG MODEL ASSEMBLY
else
if      @medmodel
%out  MEDIUM MODEL ASSEMBLY
else
%out  SMALL MODEL ASSEMBLY
endif
endif
endif


;      @BIGCODE means far procedures
;      @BIGDATA means 4 byte data pointers


@bigcode     equ   (@bigmodel or @medmodel)
@bigdata     equ   @bigmodel
;
;      PROLOGUE.H
;
;      standard prologue for c86 assembly code


;      if you set @COMFILE, the COM memory model
```

```
;       will be selected
@COMFILE        equ     0


;       @BIGCODE means far procedures.
;       @BIGDATA means 4 byte data pointers


@bigcode        equ     (@bigmodel or @medmodel)
@bigdata        equ     @bigmodel


;       DEFINE ARGUMENT BASE RELATIVE FROM BP


IF      @BIGCODE
@AB     EQU     6
ELSE
@AB     EQU     4
ENDIF


@CODE SEGMENT BYTE PUBLIC 'CODE'
@CODE ENDS
@DATAB          SEGMENT PARA PUBLIC 'DATAB'
@DATAB          ENDS
@DATAC          SEGMENT BYTE PUBLIC 'DATAC'
@sb     label byte
@sw     label word
@DATAC          ENDS
@DATAI          SEGMENT BYTE PUBLIC 'DATAI'
@ib     label byte
@iw     label word
@DATAI          ENDS
@DATAT          SEGMENT BYTE PUBLIC 'DATAT'
@DATAT          ENDS
@DATAU          SEGMENT BYTE PUBLIC 'DATAU'
@ub     label byte
@uw     label word
@DATAU          ENDS
@DATAV          SEGMENT BYTE PUBLIC 'DATAV'
@DATAV          ENDS
```

```
if      @COMFILE
DGROUP      GROUP @CODE,@DATAB,@DATAC,@DATAI,@DATAT,@DATAU,@DATAV
@CODE SEGMENT BYTE PUBLIC 'CODE'
        ASSUME      CS:DGROUP,DS:DGRQUP
else
DGROUP      GROUP @DATAB,@DATAC,@DATAI,@DATAT,@DATAU,@DATAV
@CODE SEGMENT BYTE PUBLIC 'CODE'
        ASSUME      CS:@CODE,DS:DGROUP
endif


;       END OF PROLOGUE.h
;
;
;   DISINTR.ASM                   851023
;
; The routines DISINTR and ENABINTR are used to enable and disable
; interrupt processing during time critical applications such as
; reading an AD converter.
;
;


            INCLUDE     MODEL.H
            INCLUDE     PROLOGUE.H
PUBLIC      DISINTR
DISINTR     PROC  NEAR
            CLI
            RET
DISINTR     ENDP


PUBLIC      ENABINTR
ENABINTR    PROC  NEAR
            STI
            RET
ENABINTR    ENDP
            INCLUDE     EPILOGUE.H
            END
/******************
    ADC.C                              851023
```

```
****************/

/*
    adc(data) must be preceded by a startadc() command with
    all parameters set properly.  adc() can be called indefinatly
*/




#include "dthead.h"

adc()
  {
  int datal, datah;

  readwait;                        /* wait till data ready */
  datal = inportb( DTDAT );        /* read low byte */
  readwait;                        /* wait till upper byte ready */
  datah = inportb( DTDAT );        /* and read it */
  return (datal + (datah <<8));    /* pack into integer and return */
  }
/*****************

  ADI.C                                          851023
 ****************/

/*
    adi( channel, gain, mode) reads in analog data from the designated
    channel when triggered, using the designated gain and returns
    an integer containing 12 bit data.
*/




#include "dthead.h"


adi( channel, gain, mode)
char channel, gain, mode;
```

```
{
int data;

    commandwait;      /*wait for ready flag */
    if (mode > 0)
        outportb(DTCSR, 0x8C );   /*triggered a to d immediate command */
    else
      , outportb(DTCSR, 0x0c );   /* non triggered a to d immediate command */
    writewait;    /*wait until command processed */
    outportb( DTDAT, gain );   /* set gain parameters */
    writewait;    /*wait until command processed */
    outportb( DTDAT, channel );   /* set channel */
    readwait;
    data = inportb(DTDAT);
    readwait;
    data = data +  (inportb(DTDAT) << 8);
    commandwait;                     /* wait till ready */
    if (inportb(DTCSR) & ERROR )
        {
            printf("Adi error --");
            dterror();
            return( -1);
        }
    else
        return (data);
    }
/******************
    CLEARSC.C                                      851023
 ****************/


/*
    This procedure clears the screen starting at row "first"
    through to and including row "last".
*/



clearscreen( first, last)
int first, last;
```

```
    {
    struct regval ( int ax, bx, cx, dx, si, di, ds, es; ) ;
    struct regval sreg;


    sreg.ax - 0x600;        /*clear screen using sysint */
    sreg.cx - 0x100 * first;
    sreg.dx - (0x100 * last) + 0x50;
  . sreg.bx - 0x0700;
    sysint (0x10, &sreg, &sreg);
  }
/******************
   DTCLEAR.C                                            851023
 *****************/


/*
    Clears all processes and resets DT2801
*/



#include "dthead.h"

dtclear()
  {
  dtstop();                 /* stop instruction*/
  commandwait;              /* wait for ready flag */
  outportb ( DTCSR, 0x01); /* clear instruction */
  }
/*****************
   DTERROR.C                                            851023
 ****************/


/*
    dterror() will decode the error register of the data translation
    board and print out appropriate diagnostics.  This should be called
    each time an error occurs.  dterror() also calls dtclear() to clear the
    error bit after the diagnostic is generated.
*/
```

```
#include "dthead.h"

dterror()


(
short i, error1, error2;


   if (inportb(DTCSR) & ERROR )
      (
         printf ( "DT2801 Composite error flag detected\n");
         dtstop();                /*stop execution command */
         commandwait;             /* wait for ready flag */
         outportb( DTCSR, 0x02); /*read error command */
         readwait;                /* wait for ready flag */
         error1 = inportb( DTDAT );
         readwait;                /* wait for ready flag */
         error2 = inportb( DTDAT );
         if (error1 & 0x01 )
            printf ( "--Error 0: Reserved \n");
         if (error1 & 0x02 )
            printf ( "--Error 1: Command overwrite error \n");
         if (error1 & 0x04 )
            printf ( "--Error 2: Clock set error \n");
         if (error1 & 0x08 )
            printf ( "--Error 3: Digital port select error\n");
         if (error1 & 0x10 )
            printf ( "--Error 4: Digital port set error\n");
         if (error1 & 0x20 )
            printf ( "--Error 5: DAC select error\n");
         if (error1 & 0x40 )
            printf ( "--Error 6: DAC clock error\n");
         if (error1 & 0x80 )
            printf ( "--Error 7: DAC # conversions error \n");
         if (error2 & 0x01 )
            printf ( "--Error 8: ADC channel error \n");
         if (error2 & 0x02 )
            printf ( "--Error 9: ADC gain error \n");
         if (error2 & 0x04 )
```

```
        printf ( "--Error 10: ADC clock error \n");
    if (error2 & 0x08 )
        printf ( "--Error 11: ADC multiplexer error \n");
    if (error2 & 0x10 )           .
        printf ( "--Error 12: ADC # conversions error \n");
    if (error2 & 0x20 )
        printf ( "--Error 13: Data where command expected! \n");
    if (error2 & 0x40 )
        printf ( "--Error 14: Reserved\n");
    if (error2 & 0x80 )
        printf ( "--Error 15: Reserved\n");
    dtclear();  /*clear error flag */
    }
}
/*******************
   DTRESET.C                                        851023
 ****************/


/*
    Program to reset DT 2801 board.  Proper setup commands must be
    executed before any input or output commands are used.
*/



#include "dthead.h"

dtreset()
  {
  short i;

  outportb( DTCSR, 0x0f); /* stop instruction*/
  i = inportb (DTDAT);    /* read and throw away data in register */


  while (!((inportb(DTCSR)) & READY))    /* wait for ready flag */
    ;
  outportb ( DTCSR, 0x00); /* reset instruction */
  while (!((inportb(DTCSR)) & OBF ))    /* wait for OBF flag */
    ;
  i = inportb (DTDAT);     /* read and throw away data in register */
```

```
i = inportb(DTCSR);

while (!((inportb(DTCSR)) & READY ))        /* wait for ready flag */
    ;

}
/******************

   DTSTOP.C                                              351023

 ****************/

.

/*

    dtstop() stops all processing.  This is used to exit continous block
    modes.
*/



#include "dthead.h"


dtstop()

  {
  outportb(DTCSR, 0x0F); /*stop command--no wait needed */
  inportb(DTDAT);          /*empty data register and throw away data */
  }
/******************

   DWRPORT.C                                              851023

 ****************/


/*

    dwrport() writes 16-bit data out to both of the two digital
    i-o ports.  Note that the io mode of the port must be set using
    setport before this routine is executed.
*/



#include "dthead.h"


dwrport (port, data)
  unsigned int data;
  int port;
```

```c
    {
    unsigned char low, high;

low = data & 0x00FF;
high = (data & 0xFF00) >> 8;
    switch (port)
     {
    case 8:
        commandwait;    /* wait for ready flag */
        outportb (DTCSR, 0x07 );      /* write command */
        writewait;    /* while input buffer full, wait*/
        outportb( DTDAT, 0x02 );      /* port select data */
        writewait;    /* while input buffer full, wait*/
        outportb( DTDAT, low );        /* output the data */
        writewait;
        outportb( DTDAT, high );
       break;


    case 11:
        commandwait;    /* wait for ready flag */
        outportb (DTCSR, 0x87 );
        writewait;
        outportb( DTDAT, 0x02 );
        writewait;    /*while input buffer full*/
        outportb( DTDAT, low );
        writewait;
        outportb( DTDAT, high );
       break;


    default:
       printf ("Illegal dwrport() port number\n");
       return (-1);
       break;
       }


    commandwait;
    if (inportb(DTCSR) & ERROR)
       {
```

```
        printf("Wrport error -- ");
        dterror();
        return (-1);
        }
    else
        return (0);
    }
/******************
    MOVECU.C                                        851023
 ****************/



/*
    This procedure moves the cursor to "row" and "column"
*/



movecursor (row, column)
int row, column;
    {
        struct regval ( int ax, bx, cx, dx, si, di, ds, es; ) ;
        struct regval sreg;
        sreg.ax = 0x200;
        sreg.dx = (0x100 * row) + column;
        sreg.bx =0;
        sysint (0x10, &sreg, &sreg);
    }
/******************
    SETADC.C                                        851023
 ****************/



/*
    setadc(schannel, echannel, gain, mode) starts a to d conversion.
    schannel and echannel are the start and end channels for conversions,
    gain is the adc gain, legal values are 0 to 3, corresponding to gains
    of 1 2 4 and 8 respectivly.
    Mode is a short containing 2 bit flags such that bit 0 turns
    the external clock on and off and bit 1 turns the external trigger on
```

and off.
*/


#include "dthead.h"


setadc( schannel, echannel, gain, mode, reads)
short schannel, echannel, gain, mode;
int reads;

```
    {
    commandwait;                        /* wait for ready flag */
    outportb(DTCSR, 0x0D);              /* set adc parameters command */
    writewait;                          /* wait till ready for data */
    outportb(DTDAT, gain);              /* set the gain */
    writewait;                          /* wait till ready for data */
    outportb(DTDAT, schannel);          /* set start channel */
    writewait;                          /* wait till ready for data */
    outportb(DTDAT, echannel);          /* set end channel */
    writewait;                          /* you guess */
    outportb(DTDAT, reads & 0x00FF);    /* set number of conversions */
    writewait;                          /* monotonous isn't it? */
    outportb(DTDAT, reads & 0xFF00);    /* upper byte of dummy number */
    commandwait;                        /* wait for ready flag */
    if (inportb(DTCSR) & ERROR)         /* check for errors */
        {
            printf("startadc() error --");
            dterror();
            return(-1);
        }
    else
        {
            commandwait;                        /* wait for ready flag this time */
            outportb( DTCSR, ((mode << 6) | 0x0E)); /* move mode bits */
            return(0);
        }
```

```
)
/*****************
   SETCLOCK.C                              851023
 *****************/



/*
     setclock (period) sets the internal clock period to (5us * period).
     A period of 68 us is minimum for single channel multiple d to a ,
     a period of 121 us is minimum for two channel multiple d to a,
     and a period of 75 us is minimum for multiple a to d.
*/



#include "dthead.h"



setclock(period)
int period;


   {
   short low, high;

   low = 0x00FF & period;
   high = (0xFF00 & period) >>8;

   commandwait;                    /* wait for ready flag */
   outportb(DTCSR, 0x03);          /* set clock command */
   writewait;                      /* wait till input ready */
   outportb(DTDAT, low);           /* low byte out */
   writewait;                      /* wait again */
   outportb(DTDAT, high);          /* high byte out */
   commandwait;                    /* wait for ready flag */
   if (inportb(DTCSR) & ERROR)
      {
         printf("Setclock error --");
         dterror();
         return(-1);
```

```
        )
    else
        return(0);
    )
/******************
    SETPORT.C                                    851023
 ****************/
```

```
/*
        setport(mode) sets up the digital io ports as described below
                0       non triggered input port 0
                1       non triggered input port 1
                2       non triggered input ports 0 and 1
                3       triggered input port 0
                4       triggered input port 1
                5       triggered input ports 0 and 1
                6       non triggered output port 0
                7       non triggered output port 1
                8       non triggered output ports 0 and 1
                9       triggered output port 0
               10       triggered output port 1
               11       triggered output ports 0 and 1


        setport returns 0 if successful, else returns -1
*/



#include "dthead.h"



setport (mode)
int mode;


    (
    switch (mode)
        (
    case 0:
```

```
        while (!(inportb(DTCSR) & READY))
            ;

        outportb( DTCSR, 0x04);
        while (inportb(DTCSR) & IBF)
            ;

         outportb( DTDAT, 0x00);
        break;
case 1:
        while (!(inportb(DTCSR) & READY))
            ;

        outportb( DTCSR, 0x04);
        while (inportb(DTCSR) & IBF )
            ;

        outportb( DTDAT, 0x01);
        break;

    case 2:
        while (!(inportb(DTCSR) & READY))
            ;

        outportb( DTCSR, 0x04);
        while (inportb(DTCSR) & IBF )
            ;

        outportb( DTDAT, 0x02);
        break;

    case 3:
        while (!(inportb(DTCSR) & READY))
            ;

        outportb( DTCSR, 0x84);
        while (inportb(DTCSR) & IBF)
            ;

        outportb( DTDAT, 0x00);
        break;

    case 4:
        while (!(inportb(DTCSR) & READY))
            ;

        outportb( DTCSR, 0x84);
        while (inportb(DTCSR) & IBF)
```

```c
            ;
        outportb( DTDAT, 0x01);
        break;

    case 5:
        while (!(inportb(DTCSR) & READY))
            ;
        outportb( DTCSR, 0x84);
        while (inportb(DTCSR) & IBF)
            ;
        outportb( DTDAT, 0x02);
        break;

    case 6:
        while (!(inportb(DTCSR) & READY))
            ;
        outportb( DTCSR, 0x05);
        while ((inportb(DTCSR)) & IBF)
            ;
        outportb( DTDAT, 0x00);
        break;

    case 7:
        while (!(inportb(DTCSR) & READY))
            ;
        outportb( DTCSR, 0x05);
        while (inportb(DTCSR) & IBF )
            ;
        outportb( DTDAT, 0x01);
        break;

    case 8:
        while (!(inportb(DTCSR) & READY))
            ;
        outportb( DTCSR, 0x05);
        while (inportb(DTCSR) & IBF)
            ;
        outportb( DTDAT, 0x02);
        break;
```

```
case 9:
   while (!(inportb(DTCSR) & READY))
      ;
   outportb( DTCSR, 0x85);
   while (inportb(DTCSR) & IBF)
      ;
   outportb( DTDAT, 0x00);
   break;


case 10:
   while (!(inportb(DTCSR) & READY))
      ;
   outportb( DTCSR, 0x85);
   while (inportb(DTCSR) & IBF)
      ;
   outportb( DTDAT, 0x01);
   break;


case 11:
   while (!(inportb(DTCSR) & READY))
      ;
   outportb( DTCSR, 0x85);
   while (inportb(DTCSR) & IBF )
      ;
   outportb( DTDAT, 0x02);
   break;

default:
   printf ("Illegal DIO mode detected in setport.\n");
   return (-1);
   break;
   }


while (!(inportb(DTCSR) & READY))
   ;
if (inportb (DTCSR) & ERROR)
   {
      printf ("In Setport:  ");
```

```
            dterror();
            return (-1);
        }
    else
        return(0);
    }
/******************
    LTDAC.C                                 850809
 ****************/


/*
    This routine operates the multichannel DAC on the Tecmar Lab Tender
    Board.
*/



#define BASE 0x330


ltdac (channel, value)
int channel, value;


{
static int muxsel=0;

muxsel &= 0xE7;                 /* Disable both muxes */
outportb (BASE+4, muxsel);
muxsel &= 0xF8;                 /* Change channel */
muxsel |= channel & 0x07;
outportb (BASE+4, muxsel);
outportb (BASE+5, value);       /* Output data */
if ((channel & 0x08) == 0)      /* Enable appropriate mux */
    muxsel |= 0x08;
else
    muxsel |= 0x10;
outportb (BASE+4, muxsel);
}
;
```

; INTCONTL.ASM

;

```
        title           'Interrupt Support Package'


include         model.h
include         prologue.h

                public          enable_interrupts
                public          disable_interrupts




enable_interrupts       proc    near

                sti
                ret

enable_interrupts       endp




disable_interrupts      proc    near

                cli
                ret

disable_interrupts      endp




include         epilogue.h


                end
```

```
;

;   INTRSERV.ASM                                    851118

;

;   This routine handles the code setup prior to entering a C routine
;   that is attached to an interrupt.  It also handles the exit code as well.
;


        include     model.h


;       title 'interrupt support package'


        include     prologue.h


;       initialisation for this routine provided by intrinit.c



;       when we reach the following entry point
;       we have executed the code created by intrinit
;       and the following conditions are true (we hope)


;       cs is set to our cs value
;       the return address points to a region containing
;               0, place to save sp and ss
;               4, the stack size required
;               6, the data segment value
;               8, the address of the function to execute
;


@code ends
@data       segment
        extrn   _heaptop:word
savesize dw 0
@data       ends
@code segment


;       entry point for interrupt service routines


sp      equ     .
```

```
          public      intrserv

intrserv      proc  far
       push   ds
       push   es
       push   di
       push   bp
       mov    bp,sp          ;so we can see stack
       les    di,dword ptr ap[bp]
       mov    es:[di],sp
       mov    es:2[di],ss
       mov    ds,es:6[di]        ;set up data segment
if     @bigmodel
       mov    ss,_heaptop+2      ;a segment for the stack
else
       mov    ss,es:6[di]        ;a segment for the stack
endif
       mov    sp,es:4[di]
       add    sp,savesize
       mov    savesize,sp
       add    sp,_heaptop
       push   ax
       push   bx
       push   cx
       push   dx
       push   si
       push   es
       push   di
       mov    bp,sp
if     @bigmodel
       call   dword ptr es:8[di]
else
       call   word ptr 8[di]
endif
       pop    di
       pop    es
       pop    si
       pop    dx
       pop    cx
```

```
        pop     bx
        pop     ax
        mov     sp,es:4[di]         ; adjust for subsequent calls
        sub     savesize,sp
        mov     ss,es:2[di]
        mov     sp,es:[di]
        pop     bp
        pop     di
        pop     es
        pop     ds
        add     sp,4
        iret                    ;all done
intrserv    endp
        include     epilogue.h
        end
;
;   IRQSERV.ASM                              851118
;
;   Same as INTRSERV.ASM except specific to hardware interrupts
;


        include     model.h


;       title       'IRQ interrupt support package'


        include     prologue.h


;       initialisation for this routine provided by irqinit.c



;       when we reach the following entry point
;       we have executed the code created by irqinit
;       and the following conditions are true (we hope)


;       cs is set to our cs value
;       the return address points to a region containing
;           0, place to save sp and ss
;           4, the stack size required
;           6, the data segment value
```

;         8, the address of the function to execute      **0201883**

;

```
@code        ends
@datab       segment
      extrn      _heaptop:word
savesize dw       0
@datab       ends
@code        segment


;      entry point for IRQ interrupt service routines


ap      equ      8


        public     irqserv


irqserv        proc        far


        cli                             ;****disable external interrupts


        push     ds
        push     es
        push     di
        push     bp
        mov      bp,sp                  ;so we can see stack
        les      di,dword ptr ap[bp]
        mov      es:[di],sp
        mov      es:2[di],ss
        mov      ds,es:6[di]            ;set up data segment
if      @bigmodel
        mov      ss,_heaptop+2          ;a segment for the stack
else
        mov      ss,es:6[di]            ;a segment for the stack
endif
        mov      sp,es:4[di]
        add      sp,savesize
        mov      savesize,sp
        add      sp,_heaptop
        push     ax
```

```
        push    bx
        push    cx
        push    dx
        push    si
        push    es
        push    di
        mov     bp,sp

        sti                             ;*** enable external interrupts


if      @bigmodel
        call    dword ptr es:8[di]
else
        call    word ptr 8[di]
endif

        cli                             ;****disable external interrupts
        mov     al, 20h                 ; send end of interrupt to 8259
        out     20h, al

        pop     di
        pop     es
        pop     si
        pop     dx
        pop     cx
        pop     bx
        pop     ax
        mov     sp,es:4[di]             ; adjust for subsequent calls
        sub     savesize,sp
        mov     ss,es:2[di]
        mov     sp,es:[di]
        pop     bp
        pop     di
        pop     es
        pop     ds
        add     sp,4

        sti                             ;*** enable external interrupts
```

```
        iret                            ;all done
irqserv         endp
        include         epilogue.h
        end
/******************
    INTRINIT.C                          351118
 *****************/


/*
        initialise for interrupt processing
*/


#include     "stdio.h"


unsigned char *alloc();


/*
        needs function intrserv.asm
*/


struct intrcode{
    unsigned char farcall;      /* contains far call opcode */
    int (*farip)();         /* ip value for far call */
#ifndef _C86_BIG
    unsigned farcs;         /* cs value for far call */
#endif
    unsigned savesp;            /* where we save the sp value */
    unsigned savess;            /* where to save the ss value */
    unsigned stacsize;          /* number of bytes of stack needed */
    unsigned fards;         /* the required ds value */
    int (*farfunc)();           /* function to be executed */
};


#define SIZE_IC (sizeof(struct intrcode))

intrinit(func,stack,vecno)
int (*func)();                  /* function which will process interrupt */
unsigned stack;                 /* # of bytes of stack needed by function */
unsigned vecno;                 /* # of vector for interrupt trap */
```

```c
(
    unsigned char *ustack;
    struct intrcode *icp;
    extern intrserv();                  /* our service function */
    struct {int cs,ss,ds,es;} segregs;      /* for getting seg registers */


    segread(&segregs);                  /* get cs and ds values */
    icp=alloc(SIZE_IC);                 /* this is where interrupt begins */
    icp->farcall=0x9a;                  /* direct intersegment call */
    icp->farip=intrserv;                /* addr of interrupt serv routine */
#ifndef _C86_BIG
    icp->farcs=segregs.cs;              /* get cs value */
#endif
    icp->fards=segregs.ds;
    if(stack<0x80)stack=0x80;           /* mdos requires this */
    icp->stacsize=stack;
    icp->farfunc=func;                  /* the function we want to execute */
    pokew(vecno*4,0,icp);               /* set up the interrupt vector */
#ifdef _C86_BIG
    pokew(vecno*4+2,0,((unsigned long)icp)>>16);
#else
    pokew(vecno*4+2,0,segregs.ds);      /* that does it */
#endif
}
/***********************************************************************************
*/
/*                   *
*/
/*   INTRLINK.C     *                                         840815
*/
/*                   *                      Revised  851118
*/
/******************
*/
/*
*/
/*      This procedure provides two procedures which link a C function to
*/
```

```
/*  an interrupt and unlink the C function from that interrupt.  Keep the
*/
/*  C routines short and to the point.
*/
/*
*/
/*
*/
/*    This procedure calls:    intrinit
*/
/*                             irqinit
*/
/*                             enable_interrupts
*/
/*                             disable_interrupts
*/
/*
*/
/*    Arguments:
*/
/*
*/
/*    function          - FN - C function to be linked to the interrupt
*/
/*    int_stack_space   - INT - the stack space needed for the function
*/
/*                             ( at least 512 bytes recommended )
*/
/*    int_number        - INT - the interrupt number you want to attach
*/
/*                             the C function to
*/
/*
*/
/*
*/
```

```
/*    Global Variables:       none
*/
/*
*/
/*
*/
/*
*/
/*
*/
/*    Local Variables:
*/
/*
*/
/*    interrupt[ 256 ]  = STR =      array of structures containing the
*/
/*                                   original interrupt vectors
*/
/*
*/
/*    enable_mask[ 16 ] = US  =      masks to enable the IRQ interrupts
*/
/*                                   ( interrupts 8 to 15  )
*/
/*    disable_mask[ 16 ]= US  =      masks to disable the IRQ interrupts
*/
/*                                   ( interrupts 8 to 15  )
*/
/*
*/
/*
*/
/*
*/
/*    Constants:
*/
/*
*/
/*    IMR                     =      8259's Interrupt Mask Register
*/
```

```
/*
*/
/*
*/
/*
*/
/**************************
*/
/*
*/
/*
*/
/*
*/
```

```c
#define   IMR    0x0021                    /*  Interrupt Mask Register
*/


struct   int_off_seg  { unsigned short  original_offset, original_segment; };

static   struct   int_off_seg   interrupt_[ 256 ];

static  unsigned short  enable_mask[ 16 ] =
  { 0,0,0,0,0,0,0,0, 0x00fe,0x00fd,0x00fb,0x00f7,0x00ef,0x00df;0x00bf,0x007f
};

static  unsigned short  disable_mask[ 16 ] =
  { 0,0,0,0,0,0,0,0, 0x0001,0x0002,0x0004,0x0008,0x0010,0x0020,0x0040,0x0080
};
```

**0201883**

```
link_to_interrupt( function,  int_stack_space,  int_number  )
  int  (*function)() ,  int_stack_space,  int_number;
  {


    disable_interrupts();

    interrupt_[ int_number ].original_offset  =  peek( int_number * 4,     0
);
    interrupt_[ int_number ].original_segment =  peek( int_number * 4 + 2 , 0
);

    if(    int_number > 7     &&     int_number < 16       )
      {
            irqinit(  function,  int_stack_space,  int_number );
            outportb(  IMR,  inportb( IMR )  &  enable_mask[ int_number ]
);

      }
      else    intrinit(  function,  int_stack_space,  int_number );

    enable_interrupts();



  }




unlink_from_interrupt(  int_number  )
  unsigned short  int_number;
  {


    disable_interrupts();

    if(    int_number > 7     &&     int_number < 16       )
          outportb(  IMR,  inportb( IMR )  |  disable_mask[ int_number ]  );

    pokew( int_number * 4,      0,  interrupt_[ int_number ].original_offset
);
```

```
      pokew( int_number * 4 + 2,   0,  interrupt_[ int_number
].original_segment);
```

```
    enable_interrupts();


  }
/*******************

  IRQINIT.C                              851118

 ****************/



/*

       irqinit.c   initialise for IRQ interrupt processing
*/



#include      "stdio.h"


unsigned char *alloc();


/*

     needs function irqserv.asm
*/


struct intrcode{
  unsigned char farcall;      /* contains far call opcode */
  int (*farip)();             /* ip value for far call */
#ifndef _C86_BIG
  unsigned farcs;             /* cs value for far call */
#endif
  unsigned savesp;            /* where we save the sp value */
  unsigned savess;            /* where to save the ss value */
  unsigned stacsize;            /* number of bytes of stack needed */
  unsigned fards;             /* the required ds value */
  int (*farfunc)();            /* function to be executed */
};


#define SIZE_IC (sizeof(struct intrcode))
```

```
irqinit(func,stack,vecno)
int (*func)();                        /* function which will process interrupt */
unsigned stack;                       /* # of bytes of stack needed by function */
unsigned vecno;                       /* # of vector for interrupt trap */
{
  unsigned char *ustack;
  struct intrcode *icp;
  extern irqserv();                   /* our IRQ service function */
  struct {int cs,ss,ds,es;} segregs;    /* for getting seg registers */

  segread(&segregs);                  /* get cs and ds values */
  icp=alloc(SIZE_IC);                  /* this is where interrupt begins */
  icp->farcall=0x9a;                  /* direct intersegment call */
  icp->farip=irqserv;                  /* addr of IRQ interrupt serv routine */
#ifndef _C86_BIG
  icp->farcs=segregs.cs;              /* get cs value */
#endif
  icp->fards=segregs.ds;
  if(stack<0x80)stack=0x80;              /* mdos requires this */
  icp->stacsize=stack;
  icp->farfunc=func;                  /* the function we want to execute */
  pokew(vecno*4,0,icp);                 /* set up the interrupt vector */
#ifdef _C86_BIG
  pokew(vecno*4+2,0,((unsigned long)icp)>>16);
#else
  pokew(vecno*4+2,0,segregs.ds);      /* that does it */
#endif
}
```

· CLAIMS

1.      Apparatus for manipulating an effector relative to a patient in response to changes in a physiological or morphological condition of said patient, said apparatus comprising:

(a)  medical condition sensing means for sensing said physiological or morphological condition and for producing a medical condition output signal representative thereof;

(b)  position sensing means for sensing the position of said effector relative to said patient and for producing a position output signal representative thereof;

(c)  signal processing means for:

(i)     receiving said medical condition and position output signals;

(ii)    comparing said medical condition signal with a signal representative of a desired physiological or morphological condition of said patient;

(iii)   determining an alternate position of said effector capable of producing said desired physiological or morphological condition in said patient; and,

(iv)    producing a position control signal representative of said alternate position; and,

(d) positioning means responsive to said position control signal for positioning said effector in said alternate position.

2. Apparatus for sensing and altering a physiological or morphological condition of a patient, said apparatus comprising:

(a) medical condition sensing means for sensing said physiological or morphological condition and for producing a medical condition output signal representative thereof;

(b) effector means responsive to an effector control signal for producing a desired physiological or morphological condition in said patient;

(c) position sensing means for sensing the position of said effector means relative to said patient and for producing a position output signal representative thereof;

(d) signal processing means for:

(i) receiving said medical condition and position output signals;

(ii) comparing said medical condition signal with a signal representative of said desired physiological or morphological condition;

(iii)    determining an alternate position of
         said effector means capable of
         producing said desired physiological or
         morphological condition in said
         patient; and,

(iv)    producing a position control signal
        representative of said alternate
        position; and,

(e)    positioning means responsive to said position
       control signal for positioning said effector
       means in said alternate position.


3.       Apparatus as defined in claim 2, wherein said
signal processing means is further for:

(a)    determining said desired physiological or
       morphological condition; and,

(b)    producing said signal representative of said
       desired physiological or morphological
       condition.


4.       Apparatus as defined in claim 2, wherein said
signal processing means is further for:

(a)    determining an operating condition of said
       effector means capable of producing said
       desired physiological or morphological
       condition in said patient; and,

(b) producing said effector control signal to
cause said effector means to assume said
operating condition.

5. Apparatus as defined in claim 2, wherein said
signal processing means is further for:

(a) comparing said position control signal with a
range of signals representative of safe
alternate positions of said effector means;
and,

(b) inhibiting transmission of said position
control signal to said positioning means if
said position control signal is outside said
range of safe signals.

6. Apparatus as defined in claim 4, wherein said
signal processing means is further for:

(a) comparing said effector control signal with a
range of signals representative of safe
operating conditions of said effector means;
and,

(b) inhibiting transmission of said effector
control signal to said effector means if said
effector control signal is outside said range
of safe signals.

7. Apparatus as defined in claim 5 or 6, further comprising memory means accessible by said signal processing means and for storing said medical condition output signal, said effector control signal, said position output signal, said signal representative of said desired physiological or morphological condition, said position control signal, said range of signals representative of safe alternate positions of said effector means, and said range of signals representative of safe operating conditions of said effector means.

8.      Apparatus as defined in claim 4, wherein said signal processing means is further for:

    (a)  determining a range of possible alternate positions of said effector means;

    (b)  determining a range of possible operating conditions of said effector means;

    (c)  evaluating the physiological or morphological effect on said patient of said possible alternate positions and operating conditions and of combinations of said possible alternate positions and operating conditions;

    (d)  selecting from said ranges an alternate position and operating condition of said effector means most likely to produce said desired physiological or morphological condition in said patient; and,

    (e)  producing said position and effector control signals corresponding to said selected alternate position and operating condition.


9.     A patient limb manipulator, comprising:

(a)   selectably positionable grasping means for grasping a patient's limb and for moving said limb into a selected position in response to a control signal;

(b)   limb position sensing means for:

    (i)     sensing the position of said limb while said limb is grasped by said grasping means; and,

    (ii)    producing a limb position signal representative of said sensed limb position; and,

(c)   signal processing means for:

    (i)     comparing said limb position signal with a signal representative of said selected limb position;

    (ii)    determining a path for moving said limb from said sensed position to said selected position; and,

    (iii)   producing said control signal to cause said grasping means to move said limb from said sensed position along said path to said selected position.

10.      A patient limb manipulator as defined in claim 9, wherein said signal processing means is further for:

(a)    comparing said control signal with a range of signals representative of anatomically safe paths and positions of said limb, and,

(b)    inhibiting transmission of said control signal to said grasping means if said control signal is outside said range of safe signals.

11.      A method of manipulating a portion of a living body for diagnostic or therapeutic purposes, said method comprising:

(a)    positioning an effector relative to said portion;

(b)    monitoring the position of said effector relative to said portion;

(c)    monitoring a physiological or morphological condition of said patient;

(d)    determining an alternate position of said portion capable of producing a desired change in said physiological or morphological condition; and,

(e)    actuating said effector to position such portion in said alternate position.

12.      A method as defined in claim 11, further comprising, before said actuating step:

    (a)   comparing said alternate position with a range of safe alternate positions of said limb; and,

    (b)   cancelling said actuating step if said alternate position is outside said range of safe alternate positions.

13.        Apparatus as defined in claim 1 or 2 further comprising voice recognition means for recognizing spoken command words representative of a desired position of said effector and for producing voice command signals representative thereof; and wherein said signal processing means is further for receiving said voice command signals and for producing position control signals corresponding thereto.

14.        Apparatus as defined in claim 4, further comprising voice recognition means for recognizing spoken command words representative of a desired operating condition of said effector and for producing voice command signals representative thereof; and wherein said signal processing means is further for receiving said voice command signals and producing effector control signals corresponding thereto.

15.        Apparatus as defined in claim 9, further comprising a patient applied part for attaching said manipulator to said limb, said part comprising a sensor for sensing dislodgement of said limb from said manipulator and for producing an alarm signal upon sensation of said dislodgement, and wherein said signal processing means is further for varying said path upon detection of said alarm signal to maintain attachment of said manipulator to said limb throughout said movement.

16.     Apparatus as defined in claim 15, wherein said patient applied part is removably attachable to said manipulator.

17.     Apparatus as defined in claim 16, wherein said patient applied part is detached from said manipulator when said manipulator applies a force greater than a selected force to said limb.

18.     Apparatus as defined in claim 9, further comprising force limit means for limiting the maximum force exerted upon said limb by said manipulator.

19.     Apparatus as defined in claim 18, wherein said maximum force is variable.

20.     Apparatus as defined in claim 19, wherein said signal processing means is further for determining said maximum force by operating said manipulator to detect the weight of said limb and thus the minimum force which must be applied by said manipulator to maintain said limb in a desired position, whereupon said minimum force is defined to be said maximum force.

21.          Apparatus as defined in claim 9, wherein said signal processing means is further for determining the coordinates of a path for moving a portion of said limb, with respect to a joint of said limb, from a sensed position of said limb portion to a selected position of said limb portion, and for producing a control signal to cause said grasping means to move said limb portion through said coordinates.

22.          Apparatus as defined in claim 21, wherein said signal processing means is further for comparing said path coordinates to stored coordinates representative of a range of anatomically natural paths for movement of said limb portion with respect to said joint, and for varying said determined path coordinates to maintain anatomically natural movement of said limb portion during movement thereof by said grasping means.

23.          Apparatus as defined in claim 9, further comprising memory means accessible by said signal processing means, said memory means for storing signals representative of paths for moving said limb from said sensed position into any one of a plurality of pre-selected positions.

24.      Apparatus as defined in claim 23, wherein said signal processing means is further for varying said stored path signals in proportion to the length of said limb, to facilitate movement of said limb into said pre-selected positions along anatomically natural paths.

25.      Apparatus as defined in claim 24, further comprising limb length sensing means for sensing the length of said limb and for producing an output signal representative thereof for detection by said signal processing means.

26.      Apparatus as defined in claim 9, further comprising operator control means for operator control of said manipulator, said operator control means coupled to said signal processing means and comprising a multi-function switch for producing said signal representative of said selected limb position, said switch being removably attachable for movement with said manipulator at a location which may be continuously accessed by the operator without requiring diversion of the operator's eyes to search for said switch.

27.      Apparatus as defined in claim 26, wherein said operator control means is sterilizable for use in a hospital operating room.

28.        Apparatus as defined in claim 9, further comprising voice recognition means for recognizing spoken command words representative of said selected limb position and for producing said signal representative of said selected limb position.

29.        A particulate or bacterial filtration face mask having a speech transducer for converting speech into electrical signals representative thereof without impairing the filtration capability of said mask.

30.        A face mask as defined in claim 29, further comprising voice collection means for maximizing the acoustic level of speech incident upon said speech transducer.

31.        A face mask as defined in claim 29, wherein said speech transducer is positioned within said mask to maximize the acoustic level of speech incident upon said speech transducer.

32.        Apparatus as defined in claim 30 or 31, further comprising acoustic filer means for filtration of background noise sounds from said speech prior to impingement thereof upon said transducer.

FIG 1

FIG 2

0201883

FIG 3

FIG 4

FIG 5

0201883

FIG 6

FIG 7

FIG 8

FIG 9A

FIG 9B

FIG 9C

FIG 10

FIG II

252

240

236

238

FIG 12

FIG 13

FIG 14